(19) Europäisches Patentamt
European Patent Office
Office européen des brevets

(11) EP 4 019 958 B1

(12) **EUROPEAN PATENT SPECIFICATION**

(45) Date of publication and mention of the grant of the patent:
**24.09.2025 Bulletin 2025/39**

(21) Application number: **20857212.3**

(22) Date of filing: **21.08.2020**

(51) International Patent Classification (IPC):
***G01N 27/62*** *(2021.01)* ***G01N 33/66*** *(2006.01)*
***G01N 30/04*** *(2006.01)* ***G01N 30/72*** *(2006.01)*
***G01N 30/88*** *(2006.01)* ***B01D 15/30*** *(2006.01)*
***B01D 15/32*** *(2006.01)* ***B01D 15/36*** *(2006.01)*
***G01N 30/96*** *(2006.01)* ***G01N 33/50*** *(2006.01)*

(52) Cooperative Patent Classification (CPC):
**G01N 30/88; B01D 15/305; B01D 15/322; B01D 15/325; B01D 15/363; G01N 30/96; G01N 33/50;** G01N 2030/8813; G01N 2560/00

(86) International application number:
**PCT/JP2020/031670**

(87) International publication number:
**WO 2021/039644 (04.03.2021 Gazette 2021/09)**

(54) **QUANTIFICATION METHOD OF HEX4, LYSO-GM1, FUC-GLCNAC-ASN, AND LYSO-SULFATAIDE INCLUDED IN CEREBROSPINAL FLUID**

QUANTIFIZIERUNGSVERFAHREN VON HEX4, LYSO-GM1, FUC-GLCNAC-ASN UND LYSO-SULFATAID, EINGESCHLOSSEN IN CEREBROSPINALFLÜSSIGKEIT

PROCÉDÉ DE QUANTIFICATION D’HEX4, LYSO-GM1, FUC-GLCNAC-ASN, ET LYSO-SULFATAIDE INCLUS DANS LE LIQUIDE CÉPHALORACHIDIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.08.2019 JP 2019153280**

(43) Date of publication of application:
**29.06.2022 Bulletin 2022/26**

(73) Proprietor: **JCR Pharmaceuticals Co., Ltd.**
**Ashiya-shi, Hyogo 659-0021 (JP)**

(72) Inventors:
- **HASHIMOTO, Hidehiko**
  **Kobe-shi, Hyogo 651-2241 (JP)**
- **TANAKA, Satowa**
  **Kobe-shi, Hyogo 651-2241 (JP)**
- **FUKATSU, Tomoki**
  **Kobe-shi, Hyogo 651-2241 (JP)**
- **TANAKA, Noboru**
  **Kobe-shi, Hyogo 651-2241 (JP)**

(74) Representative: **Balder IP Law, S.L.**
**Paseo de la Castellana 93**
**5ª planta**
**28046 Madrid (ES)**

(56) References cited:
**JP-A- 2004 501 365** **JP-A- 2016 506 501**
**JP-A- 2019 060 837** **US-A1- 2002 102 737**
**US-A1- 2017 350 900**

- **PIRAUD, M. ET AL.: "Contribution of tandem mass spectrometry to the diagnosis of lysosomal storage disorders", JOURNAL OF INHERITED METABOLIC DISEASE, vol. 41, 19 March 2018 (2018-03-19), pages 457 - 477, XP036507662, DOI: 10.1007/s10545-017-0126-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HEREBIAN DIRAN, ALHADDAD BADER, SEIBT ANNETTE, SCHWARZMAYR THOMAS, DANHAUSER KATHARINA, KLEE DIRK, HARMSEN STEFANI, MEITINGER THOM: "Coexisting variants in OSTMI and MANEAL cause a complex neurodegenerative disorder with NBIA-like brain abnormalities", EUROPEAN JOURNAL OF HUMAN GENETICS, vol. 25, no. 9, 14 June 2017 (2017-06-14), pages 1092 - 1095, XP055796432, DOI: 10.1038/ejhg.2017.96
- ANONYMOUS: "Matreya's 2017-2018 Catalog", MATREYA NEWSLETTER FOR GLYCO/ SPHINGOLIPID RESEARCH, MATREYA LLC. LIPIDS AND BIOCHEMICALS, US, 30 November 2015 (2015-11-30), US, pages 1 - 4, XP009534659
- WOLF HEIKE, DAMME MARKUS, STROOBANTS STIJN, D'HOOGE RUDI, BECK HANS CHRISTIAN, HERMANS-BORGMEYER IRM, LÜLLMANN-RAUCH RENATE, DIERK: "A mouse model for fucosidosis recapitulates storage pathology and neurological features of the milder form of the human disease", DISEASE MODELS & MECHANISMS, vol. 9, no. 9, 7 September 2016 (2016-09-07), pages 1015 - 1028, XP055796441, DOI: 10.1242/dmm.025122
- KRUGER, R. ET AL.: "Quantification of the Fabry marker lysoGb3 in human plasma by tandem mass spectrometry", JOURNAL OF CHROMATOGRAPHY B, vol. 883 - 88, 18 November 2011 (2011-11-18), pages 128 - 135, XP028889179, DOI: 10.1016/j.jchromb. 2011.11.020
- LOBATO, J. B. ET AL.: "Biomarkers in Lysosomal Storage Diseases", DISEASES, vol. 4, no. 40, 17 December 2016 (2016-12-17), pages 1 - 17, XP055796438, DOI: 10.3390/diseases4040040
- PETTAZZONI, M.: "LC-MS/MS multiplex analysis of lysosphingolipids in plasma and amniotic fluid: A novel tool for the screening of sphingolipidoses and Niemann-Pick type C disease", PLOS ONE, vol. 12, no. 0181700, 27 July 2017 (2017-07-27), pages 1 - 19, XP055587763, DOI: 10.1371/journal.pone.0181700
- MICHALSKI, J. C. ET AL.: "Glycoprotein lysosomal storage disorders: a-and b-mannosidosis, fucosidosis and a-N-acetylgalactosaminidase deficiency", BIOCHIMICA ET BIOPHYSICA ACTA, vol. 1455, 1999, pages 69 - 84, XP004276899, DOI: 10.1016/S0925-4439(99)00077-0

## Description

[TECHNICAK FIELD]

The present invention relates to a quantitative determination method for Hex4, *lyso-GM1,* Fuc-GlcNAc-Asn, or lyso-sulfatide included in the cerebrospinal fluid, and more particularly, the invention relates to a quantitative determination method including a step of submitting a solution including the cerebrospinal fluid to liquid chromatography to obtain an eluate, and a step of subjecting the eluate to mass analysis.

[BACKGROUND ART]

Pompe disease is a type of glycogen storage disease (type II glycogen storage disease) caused by deficiency of acidic α-glucosidase. Acidic α-glucosidase is a kind of lysosomal enzyme and has an activity of breaking down glycogen into glucose in lysosomes. When this enzyme is deficient, glycogen accumulates in lysosomes. Although this enzyme is pan-cellular, in Pompe disease, symptoms caused by invasion into the myocardium, skeletal muscles, and diaphragm constitute the main symptoms. Clinically, Pompe disease is classified into three types, namely, classical infant type, infant type, and adult type; however, in all cases, muscle weakness and respiratory disorders due to glycogen accumulation in the myocardium, skeletal muscles, and diaphragm are recognized. Regarding respiratory disorders, it has been reported that not only the disorder of the muscular contraction ability of the diaphragm is causative, but the disorder of the phrenic nerve that controls the respiratory organs is also one of the causes (Non-Patent Document 1).

As a method of treatment for Pompe disease, enzyme replacement therapy of replenishing acidic α-glucosidase in a patient by performing intravenous drip infusion has been conducted. Regarding a method for evaluating the effect of enzyme replacement therapy, a method of quantitatively determining the glycogen concentration in muscle tissue is generally carried out.

As a biomarker for Pompe disease other than glycogen, hexose tetrasaccharide (Hex4) has been reported (Patent Document 1 and Non-Patent Documents 2 to 7), and it has been reported that the concentration of hexose tetrasaccharide increases in the urine and blood of Pompe disease patients (Non-Patent Document 5). Hex4 is considered to be a metabolite of glycogen, and important constituent components thereof include Glc4 (Glc α1-6 Glc α1-4 Glc α1-4 Glc) and M4 (Glc α1-4 Glc α1-4 Glc α1-4 Glc) (Non-Patent Document 3).

GM1 gangliosidosis is a type of genetic disease caused by deficiency of β-galactosidase. β-galactosidase is a kind of lysosomal enzyme and has an activity of breaking down monosialoganglioside GM1, which is a kind of sphingolipid, into monosaccharides in lysosomes. When this enzyme is deficient, monosialoganglioside GM1 accumulates in the brain and internal organs. Clinically, GM1 gangliosidosis is classified into four types, namely, infantile type (type 1), which develops from early infancy, and in which a wide range of central nervous system disorders including spastic paraparesis are observed, and cherry red spots in the ocular fundus, hepatosplenomegaly, bone abnormalities, and the like progress; juvenile type (type 2), which develops from around the age of 1, and in which central nervous system disorders progress; adult type (type 3), in which symptoms such as dysarthria appear from later childhood, and extrapyramidal symptoms such as gait disturbance and dystonia constitute the main symptoms; and Morquio's disease type B, which shows strong bone deformities without any central nervous system manifestation.

The treatment method for GM1 gangliosidosis is limited to symptomatic treatment, and currently, there is no clinically applied enzyme replacement therapy.

In lysosomal diseases in which sphingolipids accumulate, there have been reported cases in which not only a substrate substance itself but also a lyso-form (deacylated form) of the accumulated substance are evaluated as biomarkers (Non-Patent Document 8), and it has been reported that the level of *lyso*-monosialoganglioside GM1 (*lyso*-GM1), which is a lyso-form of monosialoganglioside GM1, increases in the blood of GM1 gangliosidosis patients (Non-Patent Document 9).

Fucosidosis is a type of genetic disease caused by deficiency of α-L-fucosidase (FUCA1). FUCA1 is a kind of lysosomal enzyme and has an activity of hydrolyzing α-L-fucoside into L-fucose and alcohol in lysosomes. When this enzyme is deficient, glycolipids and glycoproteins including fucose (α-L-fucoside and the like) accumulate in the brain and internal organs. Clinically, fucosidosis is classified into two types, namely, fast-developing severe type (type 1), which develops in infancy, is accompanied by facial abnormalities and psychomotor retardation, and is characterized by an increase in the concentration of NaCl included in sweat; and slow-developing mild type (type 2), which develops from the age of 1 to 2, and in which mainly angiokeratoma is observed, and the concentration of NaCl included in sweat is normal.

The method of treatment for fucosidosis is limited to supportive therapy for neurological symptoms, and currently there is no clinically applied enzyme replacement therapy.

With regard to fucosidosis in which α-L-fucoside and the like accumulate, a method of evaluating Fuc1-α-6GlcNAc1-β-Asn (hereinafter, referred to as Fuc-GlcNAc-Asn) as a biomarker has been reported. It has been reported that Fuc1-α-6GlcNAc1-β-Asn accumulates in the urine of fucosidosis patients and the brain of fucosidosis model dogs

(Non-Patent Documents 10 to 14).

Metachromatic leukodystrophy is a type of genetic disease caused by deficiency of arylsulfatase A (ARSA). ARSA is a kind of lysosomal enzyme and has an activity of breaking down sulfatide, which is a kind of sphingolipid, into galactosyl ceramide in lysosomes. When this enzyme is deficient, sulfatides and the like accumulate in the white matter of the brain, peripheral nerves, kidneys, and the like. Clinically, metachromatic leukodystrophy is classified into three types, namely, infant type, which develops by the age of 2 and exhibit muscle hypotonia, absent deep tendon reflex, and gait disturbance; juvenile type, which develops around the age of 4 to 6 and exhibits optic nerve atrophy, intellectual disturbance, spastic paralysis, and the like; and adult type, which develops after the age of late teens with emotional disturbance, speech impediment, dementia, neurologic manifestation, and the like and progresses over the course of 5 to 10 years.

The method for treatment of metachromatic leukodystrophy is limited to symptomatic treatment, and currently, there is no clinically applied enzyme replacement therapy.

It has been reported that regarding the accumulating substances for metachromatic leukodystrophy, the levels of not only sulfatide but also lyso-sulfatide, which is a lyso-form of sulfatide, increase in the brain, kidneys, and liver of the patients having metachromatic leukodystrophy (Patent Document 2 and Non-Patent Documents 15 to 19).

[PRIOR ART DOCUMENTS]

[Patent Documents]

[Patent Document 1] JP 2004-501365 A
[Patent Document 2] JP 2016-506501 A
Patent Document US 2002/102737 A1


[Non-Patent Documents]

[Non-Patent Document 1]: DeRuisseau LR. et al., Proc Natl Acad Sci U S A. 106(23), 9419-24 (2009)
[Non-Patent Document 2]: Chien YH. et al., JIMD Rep. 19, 67-73 (2015)
[Non-Patent Document 3]: Sluiter W. et al., Clin Chem. 58(7), 1139-47 (2012)
[Non-Patent Document 4]: Young SP. et al., Genet Med. 11(7), 536-41 (2009)
[Non-Patent Document 5]: An Y. et al., Mol Genet Metab. 85(4), 247-54 (2005)
[Non-Patent Document 6]: Young SP. et al., Anal Biochem. 316(2), 175-80 (2003)
[Non-Patent Document 7]: Rozaklis T. et al., Clin Chem. 48(1), 131-9 (2002)
[Non-Patent Document 8]: JB Lobato et al., Diseases. 4(4): 40 (2016)
[Non-Patent Document 9]: Pettazzoni et al., PLoS ONE. 12(7): e0181700 (2017)
[Non-Patent Document 10]: Strecker G et al. Biochimie. 60(8): 725-34 (1978)
[Non-Patent Document 11]: Abraham D et al. Biochem J. 222(1): 25-33 (1984)
[Non-Patent Document 12]: Michalski JC et al. Eur J Biochem. 201(2): 439-58 (1991)
[Non-Patent Document 13]: Michalski JC et al. Biochim Biophys Acta. 1455(2-3): 69-84 (1999)
[Non-Patent Document 14]: Wolf H et al. Dis Model Mech. 9(9): 1015-28 (2016)
[Non-Patent Document 15]: Toda K et al. Biochem Biophys Res Commun. 159(2): 605-11 (1989)
[Non-Patent Document 16]: Blomqvist M et al. Lipids Health Dis. 10 28 (2011)
[Non-Patent Document 17]: Dali C et al. Ann Clin Transl Neurol. 2(5): 518-33 (2015)
[Non-Patent Document 18]: Mirzaian M et al. J Lipid Res. 56(4): 936-43 (2015)
[Non-Patent Document 19]: Blomqvist M et al. J Lipid Res. 58(7): 1482-1489 (2017)


[SUMMARY OF THE INVENTION]

[TECHNICAL PROBLEM]

An object of the present invention is to provide a method for quantifying Hex4, *lyso-GM1,* Fuc-GlcNAc-Asn, or *lyso*-sulfatide, all of which are included in the cerebrospinal fluid.

The present invention is defined in the appended claims.

[MEANS TO SOLVE THE PROBLEM]

In the study directed to the above-described object, the inventors of the present invention found that Hex4, *lyso-GM1,* Fuc-GlcNAc-Asn, or *lyso*-sulfatide included in the cerebrospinal fluid collected from an experimental animal can be quantified with high sensitivity by mass spectrometry, thus completing the present invention. In general the present invention relates to a

method for quantifying a substance included in cerebrospinal fluid, the method including:

a step of adding an internal standard substance to a solution including the cerebrospinal fluid, to which the internal standard substance has been added;
a step of submitting the solution including the cerebrospinal fluid to liquid chromatography to obtain an eluate; and
a step of subjecting the eluate to mass analysis.

In a first aspect of the invention
the substance included in the cerebrospinal fluid is Hex4.

When analysing Hex4,
liquid chromatography typically is hydrophilic interaction liquid chromatography.

According to the present invention Hex4 is measured in comparison with the internal standard substance represented by the following Formula (VII):

[Chemical Formula 7]

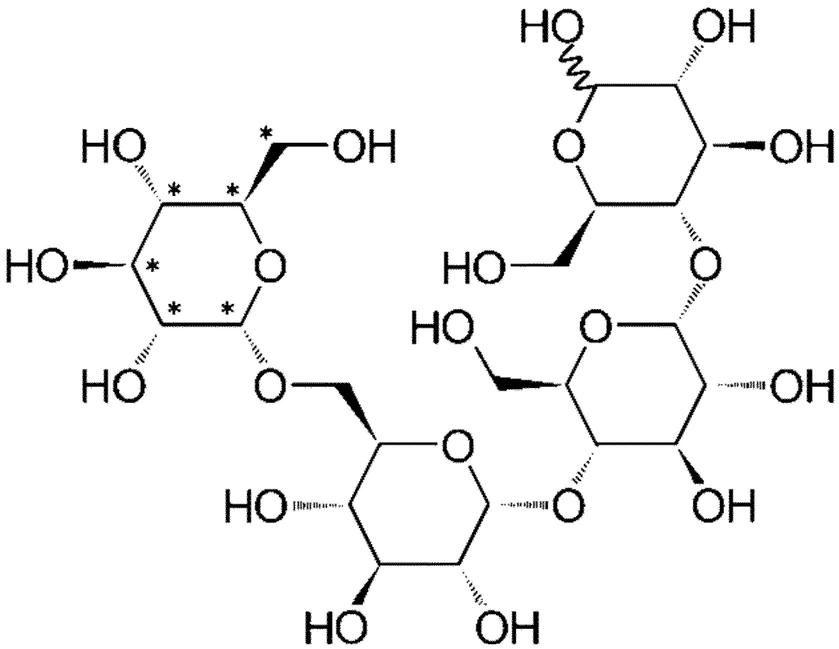

(VII)

wherein at least one of the carbon atoms marked by asterisks is carbon-13.

In general,
the cerebrospinal fluid is obtained from a patient with a disease in which Hex4 and/or glycogen accumulates in the body,
wherein the disease is Pompe disease. In some cases
the patient has received treatment to reduce Hex4 and/or glycogen present in the body.

In another aspect of the present invention
the substance included in the cerebrospinal fluid is *lyso*-monosialoganglioside GM1.

For the purification of monosialoganglioside GM1, the liquid chromatography typically is reverse phase chromatography.

According to the present invention, *lyso*-monosialoganglioside GM1 is measured in comparison with the internal standard substance represented by the following Formula (VIII):

[Chemical Formula 8]

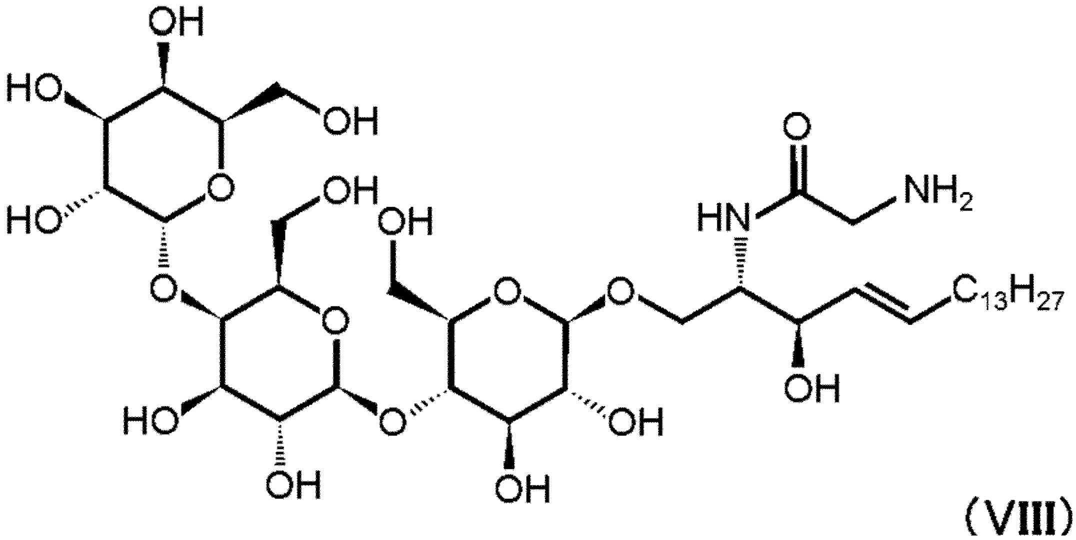

(VIII)

In this case, the cerebrospinal fluid is obtained from a patient with GM1 gangliosidosis.

In some embodiments, the patient has received treatment to reduce *lyso*-monosialoganglioside GM1 and/or monosialoganglioside GM1 present in the body.

In a further aspect of the present invention, the substance included in the cerebrospinal fluid is Fuc-GlcNAc-Asn.

For the purification of Fuc-GlcNAc-Asn the liquid chromatography is normal phase chromatography or anion exchange chromatography.

According to the present invention Fuc-GlcNAc-Asn is measured in comparison with the internal standard substance represented by the following Formula (IX):

[Chemical Formula 9]

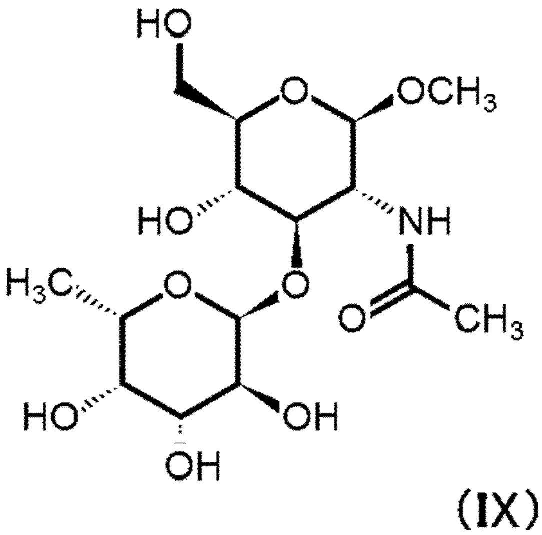

(IX)

The cerebrospinal fluid is obtained from a patient with fucosidosis.

In some embodiments, the patient has received treatment to reduce Fuc-GlcNAc-Asn and/or α-L-fucoside present in the body.

According to a further aspect of the present invention the substance included in the cerebrospinal fluid is *lyso*-sulfatide.

For the purification of lyso-sulfatide the liquid chromatography is reverse phase chromatography.

According to the present invention lyso-sulfatide is measured in comparison with the internal standard substance represented by the following Formula (X):

[Chemical Formula 10]

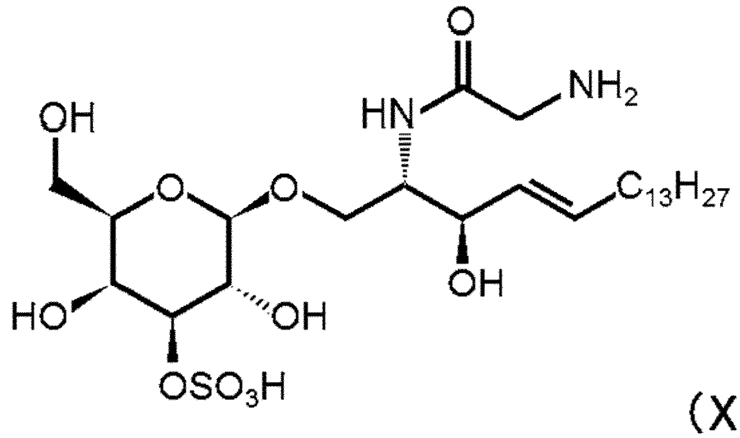

(X)

The cerebrospinal fluid is obtained from a patient with metachromatic leukodystrophy.

Typically, the patient has received treatment to reduce *lyso*-sulfatide and/or sulfatide present in the body.

[EFFECT OF INVENTION]

According to the invention, diagnosis of a disease that develops as Hex4 and/or glycogen accumulates in the central nervous system can be conducted, and the effect of treatment carried out to reduce Hex4 and/or glycogen accumulated in the central nervous system, the treatment being carried out for a disease such as described above, can be investigated.

Furthermore, according to the invention, diagnosis of a disease that develops as lyso-monosialoganglioside GM1 and/or monosialoganglioside GM1 accumulates in the central nervous system can be conducted, and the effect of treatment carried out to reduce lyso-monosialoganglioside GM1 and/or monosialoganglioside GM1 accumulated in the central nervous system, the treatment being carried out for a disease such as described above, can be investigated.

Furthermore, according to the invention, diagnosis of a disease that develops as Fuc-GlcNAc-Asn and/or $\alpha$-L-fucoside accumulates in the central nervous system can be conducted, and the effect of treatment carried out to reduce Fuc-GlcNAc-Asn and/or $\alpha$-L-fucoside accumulated in the central nervous system, the treatment being carried out for a disease such as described above, can be investigated.

Furthermore, according to the invention, diagnosis of a disease that develops as lyso-sulfatide and/or sulfatide accumulates in the central nervous system can be conducted, and the effect of treatment carried out to reduce lyso-sulfatide and/or sulfatide accumulated in the central nervous system, the treatment being carried out for a disease such as described above, can be investigated.

[BRIEF DESCRIPTION OF THE DRAWINGS]

Fig. 1 is a diagram showing a calibration curve for Hex4. The ordinate axis represents the area ratio (Hex4 detection peak area/H-IS detection peak area), and the abscissa axis represents the concentration (ng/mL) of Hex4, respectively.

Fig. 2 is a diagram showing the results of measuring the Hex4 concentration in the brain in wild-type mice and Pompe disease model mice. The ordinate axis represents the concentration ($\mu$g/wet tissue weight (g)) of Hex4 in the brain. The white bar represents the measured values of the concentration of Hex4 in the brain of wild-type mice (WT), and the black bar represents the measured values of the concentration of Hex4 in the brains of Pompe disease model mice (KO), respectively. Error bars indicate standard deviations (WT; n = 5: KO; n = 4).

Fig. 3 is a diagram showing the results of measuring the Hex4 concentration in the cerebrospinal fluid (CSF) in wild-type mice and Pompe disease model mice. The ordinate axis represents the concentration (ng/mL) of Hex4 in the CSF. The white bar represents the measured values of the concentration of Hex4 in the CSF of wild-type mice (WT), and the black bar represents the measured values of the concentration of Hex4 in the CSF of Pompe disease model mice (KO), respectively. Error bars indicate standard deviations (WT; n = 5: KO; n = 3).

Fig. 4 is a diagram showing the results of measuring the glycogen concentration in the brain in wild-type mice and

Pompe disease model mice. The ordinate axis represents the concentration (mg/wet tissue weight (g)) of glycogen. The white bar represents the measured values of the concentration of glycogen in the brains of wild-type mice (WT), and the black bar represents the measured values of the concentration of glycogen in the brains of Pompe disease model mice (KO), respectively. Error bars indicate standard deviations (WT; n = 5: KO; n = 4).

Fig. 5 is a diagram showing the relationship between the glycogen concentration in the brain and the Hex4 concentration in the brain in the same mouse individuals. The ordinate axis represents the glycogen concentration (mg/wet tissue weight (g)) in the brain, and the abscissa axis represents the Hex4 concentration (μg/wet tissue weight (g)) in the brain, respectively. White diamonds indicate wild-type mice (WT), and black diamonds indicate Pompe disease model mice (KO), respectively.

Fig. 6 is a diagram showing the relationship between the glycogen concentration in the brain and the Hex4 concentration in the CSF in the same mouse individuals. The ordinate axis represents the glycogen concentration (mg/wet tissue weight (g)) in the brain, and the abscissa axis represents the Hex4 concentration (ng/mL) in the CSF. White diamonds indicate wild-type mice (WT), and black diamonds indicate Pompe disease model mice (KO), respectively.

Fig. 7 is a diagram showing the relationship between the Hex4 concentration in the brain and the Hex4 concentration in the CSF in the same mouse individuals. The axis of ordinate represents the Hex4 concentration (μg/wet tissue weight (g)) in the brain, and the abscissa axis represents the Hex4 concentration (ng/mL) in the CSF. White diamonds indicate wild-type mice (WT), and black diamonds indicate Pompe disease model mice (KO), respectively.

Fig. 8 is a diagram showing a calibration curve for *lyso-GM1.* The ordinate axis represents the area ratio (*lyso*-GM1 detection peak area/G-IS detection peak area), and the abscissa axis represents the concentration (ng/mL) of *lyso-GM1,* respectively.

Fig. 9 is a diagram showing the results of measuring the *lyso*-GM1 concentration in the brain in wild-type mice (WT), β-Gal KO homozygous mice (KO), and β-Gal KO heterozygous mice (Hetero). The ordinate axis represents the concentration (μg/wet tissue weight (g)) of *lyso-GM1* in the brain. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 10 is a diagram showing the results of measuring the *lyso*-GM1 concentration in the spinal cord in wild-type mice (WT), β-Gal KO homozygous mice (KO), and β-Gal KO heterozygous mice (Hetero). The ordinate axis represents the concentration (μg/wet tissue weight (g)) of *lyso-GM1* in the spinal cord. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 11 is a diagram showing the results of measuring the *lyso*-GM1 concentration in the lung in wild-type mice (WT), β-Gal KO homozygous mice (KO), and β-Gal KO heterozygous mice (Hetero). The ordinate axis represents the concentration (ng/wet tissue weight (g)) of *lyso-GM1* in the lung. 12wks indicates 12 weeks of age, and 38wks indicates 38 weeks of age, respectively. Error bars indicate standard deviations (WT12wks, Hetero12wks; n = 1, WT38wks: KO12wks, KO38wks; n = 2).

Fig. 12 is a diagram showing the results of measuring the *lyso*-GM1 concentration in the liver in wild-type mice (WT), β-Gal KO homozygous mice (KO), and β-Gal KO heterozygous mice (Hetero). The ordinate axis represents the concentration (ng/wet tissue weight (g)) of *lyso*-GM1 in the liver. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 13 is a diagram showing the results of measuring the *lyso*-GM1 concentration in the kidney in wild-type mice (WT), β-Gal KO homozygous mice (KO), and β-Gal KO heterozygous mice (Hetero). The ordinate axis represents the concentration (ng/wet tissue weight (g)) of *lyso-GM1* in the kidney. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 14 is a diagram showing the results of measuring the *lyso*-GM1 concentration in the quadriceps femoris muscle in wild-type mice (WT), β-Gal KO homozygous mice (KO), and β-Gal KO heterozygous mice (Hetero). The ordinate axis represents the concentration (ng/wet tissue weight (g)) of *lyso-GM1* in the quadriceps femoris muscle. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 15 is a diagram showing the results of measuring the *lyso*-GM1 concentration in the heart in wild-type mice (WT), β-Gal KO homozygous mice (KO), and β-Gal KO heterozygous mice (Hetero). The ordinate axis represents the concentration (ng/wet tissue weight (g)) of *lyso-GM1* in the heart. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 16 is a diagram showing the results of measuring the *lyso*-GM1 concentration in the spleen in wild-type mice (WT), β-Gal KO homozygous mice (KO), and β-Gal KO heterozygous mice (Hetero). The ordinate axis represents the

concentration (ng/wet tissue weight (g)) of *lyso-GM1* in the spleen. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 17 is a diagram showing the results of measuring the *lyso*-GM1 concentration in the blood plasma in wild-type mice (WT), β-Gal KO homozygous mice (KO), and β-Gal KO heterozygous mice (Hetero). The ordinate axis represents the concentration (ng/mL) of *lyso-GM1* in the blood plasma. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 18 is a diagram showing the results of measuring the *lyso*-GM1 concentration in the CSF in wild-type mice (WT), β-Gal KO homozygous mice (KO), and β-Gal KO heterozygous mice (Hetero). The ordinate axis represents the concentration (ng/mL) of *lyso*-GM1 in the CSF. 12wks indicates 12 weeks of age, and 38wks indicates 38 weeks of age, respectively. Error bars indicate standard deviations (WT12wks, Hetero12wks, KO12wks; n = 1: WT38wks, KO38wks; n = 2).

Fig. 19 is a diagram showing the results of measuring the GM1 concentration in the brain in wild type mice (WT), β-Gal KO homozygous mice (KO), and β-Gal KO heterozygous mice (Hetero). The ordinate axis represents the concentration (mg/wet tissue weight (g)) of GM1 in the brain. 12wks indicates 12 weeks of age, and 38wks indicates 38 weeks of age, respectively. Error bars indicate standard deviations (WT12wks, Hetero12wks; n = 1: WT38wks, KO12wks, KO38wks; n = 2).

Fig. 20 is a diagram showing the relationship between the *lyso*-GM1 concentration in the brain and the *lyso-GM1* concentration in the CSF in the same mouse individuals. The ordinate axis represents the concentration (μg/wet tissue weight (g)) of *lyso*-GM1 in the brain, and the abscissa axis represents the concentration (ng/mL) of *lyso-GM1* in the CSF, respectively. White diamonds indicate wild-type mice (WT), white circles indicate β-Gal KO heterozygous mice (Hetero), and black diamonds indicate β-Gal KO homozygous mice (KO), respectively.

Fig. 21 is a diagram showing the relationship between the GM1 concentration in the brain and the *lyso-GM1* concentration in the CSF in the same mouse individuals. The ordinate axis represents the GM1 concentration (mg/wet tissue weight (g)) in the brain, and the abscissa axis represents the *lyso-GM1* concentration (ng/mL) in the CSF, respectively. White diamonds indicate wild-type mice (WT), white circles indicate β-Gal KO heterozygous mice (Hetero), and black diamonds indicate β-Gal KO homozygous mice (KO), respectively.

Fig. 22 is a diagram showing the relationship between the *lyso*-GM1 concentration in the brain and the GM1 concentration in the brain in the same mouse individuals. The ordinate axis represents the GM1 concentration (mg/wet tissue weight (g)) in the brain, and the abscissa axis represents the *lyso-GM1* concentration (μg/wet tissue weight (g)) in the brain, respectively. White diamonds indicate wild-type mice (WT), white circles indicate β-Gal KO heterozygous mice (Hetero), and black diamonds indicate β-Gal KO homozygous mice (KO), respectively.

Fig. 23 is a diagram showing a calibration curve of Fuc-GlcNAc-Asn. The ordinate axis represents the area ratio (Fuc-GlcNAc-Asn detection peak area/F-IS detection peak area), and the abscissa axis represents the concentration (ng/mL) of Fuc-GlcNAc-Asn, respectively.

Fig. 24 is a diagram showing the results of measuring the Fuc-GlcNAc-Asn concentration in the brain in wild-type mice and fucosidosis model mice. The ordinate axis represents the concentration (mg/dry tissue weight (g)) of Fuc-GlcNAc-Asn in the brain. The white bar represents the measured values of the concentration of Fuc-GlcNAc-Asn in the brain of wild-type mice (WT), and the black bar represents the measured values of the concentration of Fuc-GlcNAc-Asn in the brain of fucosidosis model mice (KO), respectively. Error bars indicate standard deviations (WT; n = 3: KO; n = 3).

Fig. 25 is a diagram showing the results of measuring the Fuc-GlcNAc-Asn in the liver in wild-type mice and fucosidosis model mice. The ordinate axis represents the concentration (mg/dry tissue weight (g)) of Fuc-GlcNAc-Asn in the liver. The white bar represents the measured values of the concentration of Fuc-GlcNAc-Asn in the liver of wild-type mice (WT), and the black bar represents the measured values of the concentration of Fuc-GlcNAc-Asn in the liver of fucosidosis model mice (KO), respectively. Error bars indicate standard deviations (WT; n = 3: KO; n = 3).

Fig. 26 is a diagram showing the results of measuring the Fuc-GlcNAc-Asn concentration in the kidney in wild-type mice and fucosidosis model mice. The ordinate axis represents the concentration (mg/dry tissue weight (g)) of Fuc-GlcNAc-Asn in the kidney. The white bar represents the measured values of the concentration of Fuc-GlcNAc-Asn in the kidney of wild-type mice (WT), and the black bar represents the measured values of the concentration of Fuc-GlcNAc-Asn in the kidney of fucosidosis model mice (KO), respectively. Error bars indicate standard deviations (WT; n = 3: KO; n = 3).

Fig. 27 is a diagram showing the results of measuring the Fuc-GlcNAc-Asn concentration in the spleen in wild-type mice and fucosidosis model mice. The ordinate axis represents the concentration (mg/dry tissue weight (g)) of Fuc-GlcNAc-Asn in the spleen. The white bar represents the measured values of the concentration of Fuc-GlcNAc-Asn in the spleen of wild-type mice (WT), and the black bar represents the measured values of the concentration of Fuc-GlcNAc-Asn in the spleen of fucosidosis model mice (KO), respectively. Error bars indicate standard deviations (WT; n = 3: KO; n = 3).

Fig. 28 is a diagram showing the results of measuring the Fuc-GlcNAc-Asn concentration in the blood plasma in wild-type mice and fucosidosis model mice. The ordinate axis represents the concentration (μg/mL) of Fuc-GlcNAc-Asn in the blood plasma. The white bar represents the measured values of the concentration of Fuc-GlcNAc-Asn in the blood plasma of wild-type mice (WT), and the black bar represents the measured values of the concentration of Fuc-GlcNAc-Asn in the blood plasma of fucosidosis model mice (KO), respectively. Error bars indicate standard deviations (WT; n = 3: KO; n = 3).

Fig. 29 is a diagram showing the results of measuring the Fuc-GlcNAc-Asn concentration in urine in wild-type mice and fucosidosis model mice. The ordinate axis represents the concentration (μg/mg) of Fuc-GlcNAc-Asn in urine. The white bar represents the measured values of the concentration of Fuc-GlcNAc-Asn in the urine of wild-type mice (WT), and the black bar represents the measured values of the concentration of Fuc-GlcNAc-Asn in the urine of fucosidosis model mice (KO), respectively. Error bars indicate standard deviations (WT; n = 3: KO; n = 2: no error bar for KO).

Fig. 30 is a diagram showing the results of measuring the Fuc-GlcNAc-Asn concentration in the CSF in wild-type mice and fucosidosis model mice. The ordinate axis represents the concentration (μg/mL) of Fuc-GlcNAc-Asn in the CSF. The white bar represents the measured values of the concentration of Fuc-GlcNAc-Asn in the CSF of wild-type mice (WT), and the black bar represents the measured values of the concentration of Fuc-GlcNAc-Asn in the CSF of fucosidosis model mice (KO), respectively. Error bars indicate standard deviations (WT; n = 3: KO; n = 3).

Fig. 31 is a diagram showing the relationship between the Fuc-GlcNAc-Asn concentration in the brain and the Fuc-GlcNAc-Asn concentration in the CSF in the same mouse individuals. The ordinate axis represents the Fuc-GlcNAc-Asn concentration (mg/dry tissue weight (g)) in the brain, and the abscissa axis represents the Fuc-GlcNAc-Asn concentration (μg/mL) in the CSF, respectively. White diamonds indicate wild-type mice (WT), and black diamonds indicate fucosidosis model mice (KO), respectively.

Fig. 32 is a diagram showing a calibration curve for *lyso*-sulfatide. The ordinate axis represents the area ratio (*lyso*-sulfatide detection peak area/S-IS detection peak area), and the abscissa axis represents the concentration (ng/mL) of *lyso*-sulfatide, respectively.

Fig. 33 is a diagram showing the results of measuring the *lyso*-sulfatide concentration in the brain in wild-type mice, ARSA KO homozygous mice, and ARSA KO heterozygous mice. The ordinate axis represents the concentration (μg/dry tissue weight (g)) of *lyso*-sulfatide in the brain. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young); n = 2: KO, KO(young); n = 3: Hetero; n = 4).

Fig. 34 is a diagram showing the results of measuring the *lyso*-sulfatide concentration in the brain in wild-type mice, ARSA KO homozygous mice, and ARSA KO heterozygous mice. The ordinate axis represents the concentration (μg/dry tissue weight (g)) of *lyso*-sulfatide in the spinal cord. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young); n = 2: KO, KO(young); n = 3: Hetero; n = 4).

Fig. 35 is a diagram showing the results of measuring the *lyso*-sulfatide concentration in the brain in wild-type mice, ARSA KO homozygous mice, and ARSA KO heterozygous mice. The ordinate axis represents the concentration (μg/dry tissue weight (g)) of *lyso*-sulfatide in the sciatic nerve. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young): n = 2, KO, KO(young): n = 3, Hetero: n = 4).

Fig. 36 is a diagram showing the results of measuring the *lyso*-sulfatide concentration in the brain in wild-type mice, ARSA KO homozygous mice, and ARSA KO heterozygous mice. The ordinate axis represents the concentration (μg/dry tissue weight (g)) of *lyso*-sulfatide in the kidney. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young); n = 2: KO, KO(young); n = 3: Hetero; n = 4). BLOQ indicates that the measured value is the lower limit of quantitative determination.

Fig. 37 is a diagram showing the results of measuring the *lyso*-sulfatide concentration in the brain in wild-type mice, ARSA KO homozygous mice, and ARSA KO heterozygous mice. The ordinate axis represents the concentration (ng/mL) of lyso-sulfatide in the blood plasma. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young); n = 2: KO, KO(young); n =

3: Hetero: n = 4). BLOQ indicates that the measured value is the lower limit of quantitative determination.

Fig. 38 is a diagram showing the results of measuring the *lyso*-sulfatide concentration in the brain in wild-type mice, ARSA KO homozygous mice, and ARSA KO heterozygous mice. The ordinate axis represents the concentration (ng/mL) of lyso-sulfatide in the CSF. WT(young) indicates 20- to 23-week-old wild-type mice, WT indicates 93- to 100-week-old wild-type mice, KO(young) indicates 20- to 23-week-old ARSA KO homozygous mice, KO indicates 93- to 100-week-old ARSA KO homozygous mice, and Hetero indicates 93- to 100-week-old ARSA KO heterozygous mice, respectively. Error bars indicate standard deviations (WT, WT(young); n = 2: KO, KO(young); n = 3: Hetero; n = 4). BLOQ indicates that the measured value is the lower limit of quantitative determination.

Fig. 39 is a diagram showing the relationship between the *lyso*-sulfatide concentration in the brain and the lyso-sulfatide concentration in the CSF in the same mouse individuals. The ordinate axis represents the lyso-sulfatide concentration (μg/dry tissue weight (g)) in the brain, and the abscissa axis represents the *lyso*-sulfatide concentration (ng/mL) in the CSF. White diamonds indicate wild-type mice (WT), white circles indicate ARSA KO heterozygous mice (Hetero), and black diamonds indicate ARSA KO homozygous mice (KO), respectively.

[DETAILED DESCRIPTION OF THE INVENTION]

The term "glucose tetrasaccharide" according to the invention implies that four molecules of D-glucose are bonded through an α1-6 bond, an α1-4 bond, and an α1-4 bond, respectively in this order, and refers to a compound represented by the following Formula (I). A salt of the compound represented by Formula (I) and an equivalent thereof are also included in the glucose tetrasaccharide. Glucose tetrasaccharide can be written as Glc4 for short. The term 6-α-D-Glucopyranosyl Maltotriose used in Example 2 has the same meaning as glucose tetrasaccharide.

[Chemical Formula 1]

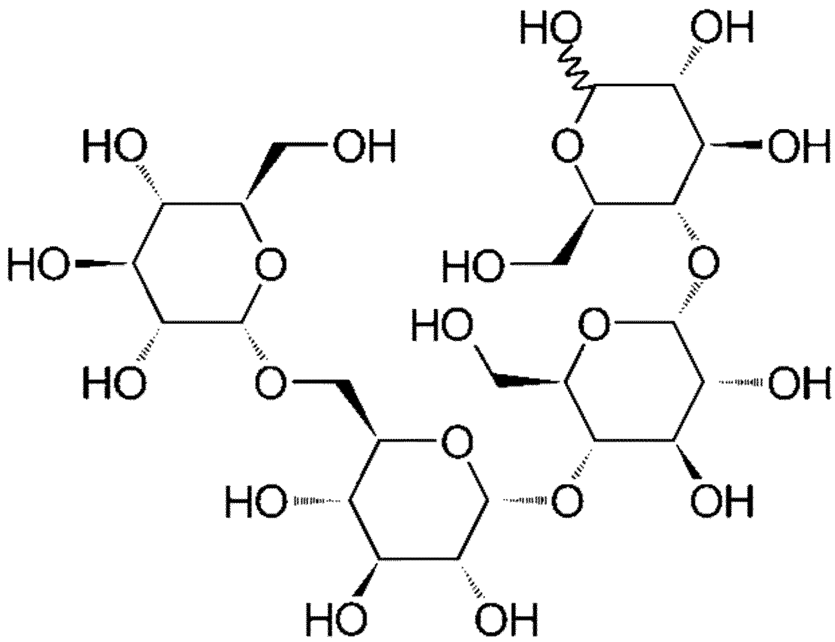

(I)

The term "lyso-monosialoganglioside GM1" according to the invention implies that monosialoganglioside GM1, which is a sphingolipid represented by the following Formula (II), is a compound represented by the following Formula (III), which is a deacylated lyso-form, and a salt of the compound represented by the following Formula (III) and an equivalent thereof are also included in lyso-monosialoganglioside GM1. Monosialoganglioside GM1 can be written as GM1 for short, and lyso-monosialoganglioside GM1 can be written as *lyso-GM1* for short.

[Chemical Formula 2]

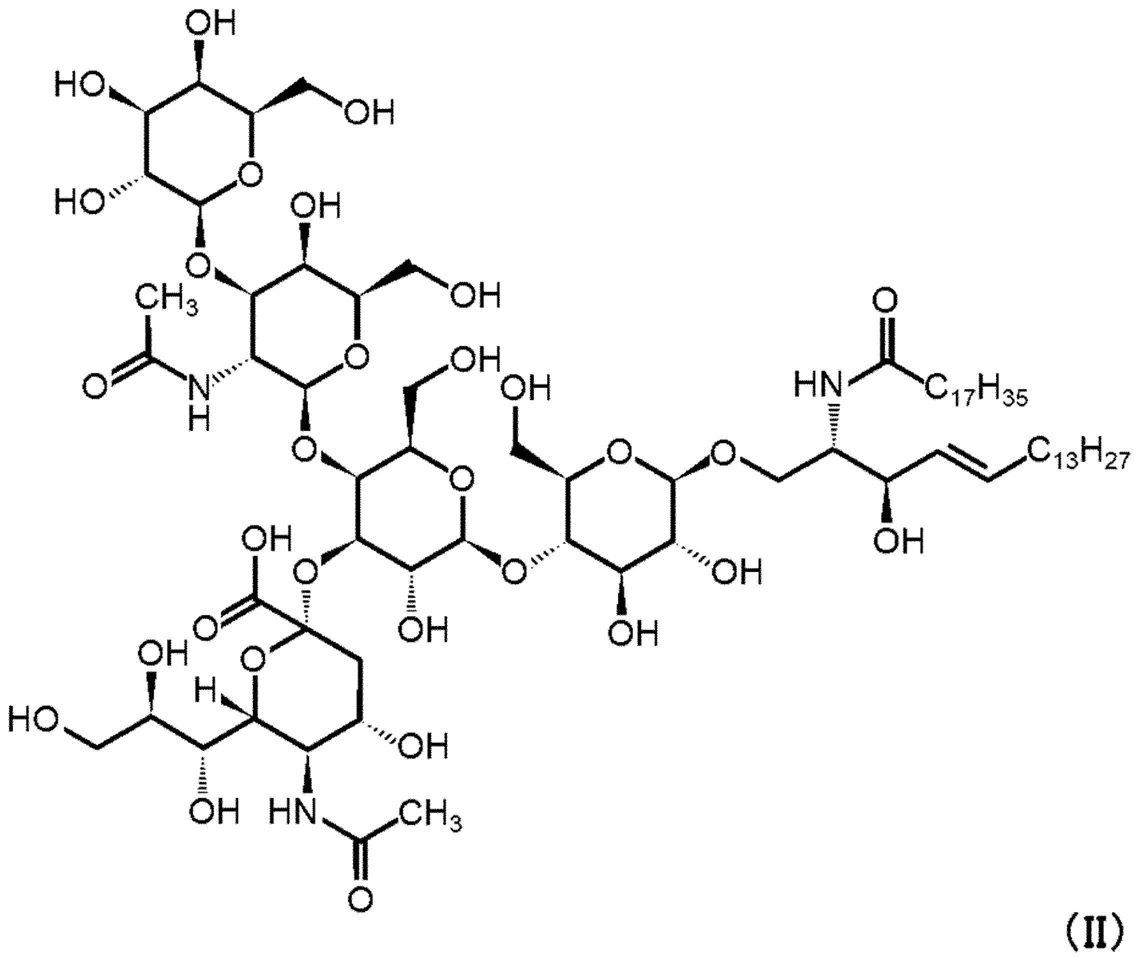

(II)

[Chemical Formula 3]

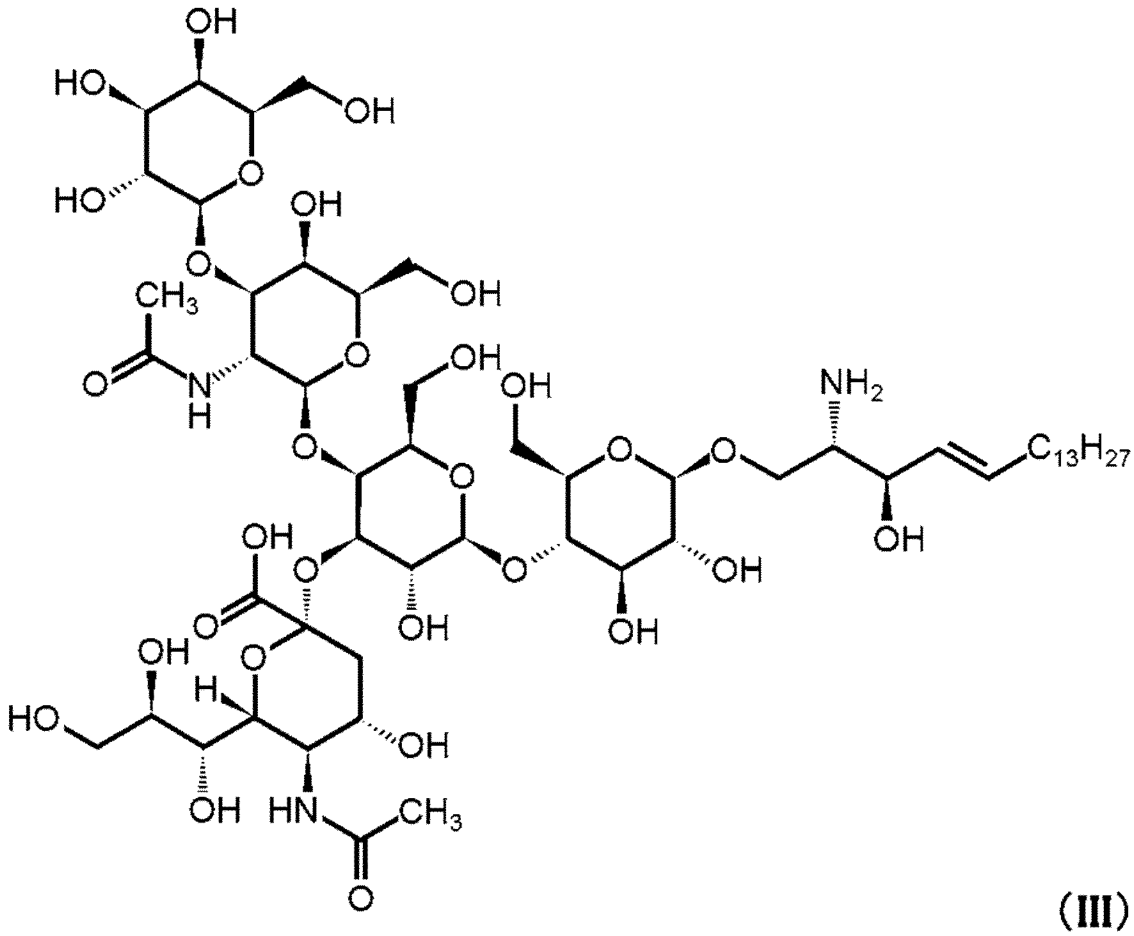

(III)

The term "Fuc1-α-6GlcNAc1-β-Asn" according to the invention refers to a compound represented by the following Formula (IV), and a salt of the compound represented by the following Formula (IV) and an equivalent thereof are also included in Fuc1-α-6GlcNAc1-β-Asn. Fuc1-α-6GlcNAc1-β-Asn can be written as Fuc-GlcNAc-Asn for short.

[Chemical Formula 4]

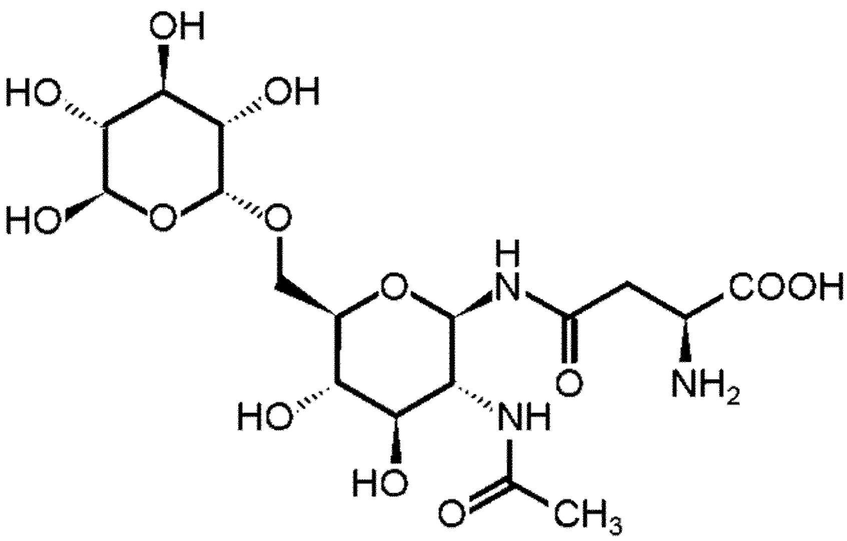

(IV)

The term "lyso-sulfatide" according to the invention implies that sulfatide, which is a glycolipid represented by the

following Formula (V), is a compound represented by the following Formula (VI), which is a deacylated lyso-form, and a salt of the compound represented by the following Formula (VI) and an equivalent thereof are also included in lyso-sulfatide. Lyso-sulfatide can be written as lyso-sulfatide.

[Chemical Formula 5]

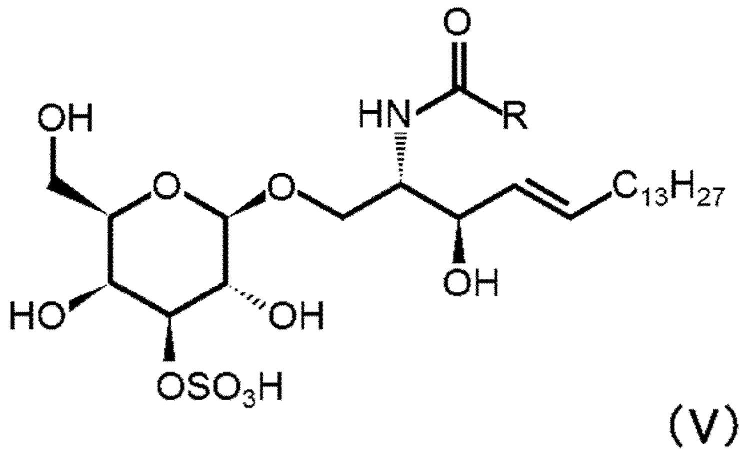

(V)

[Chemical Formula 6]

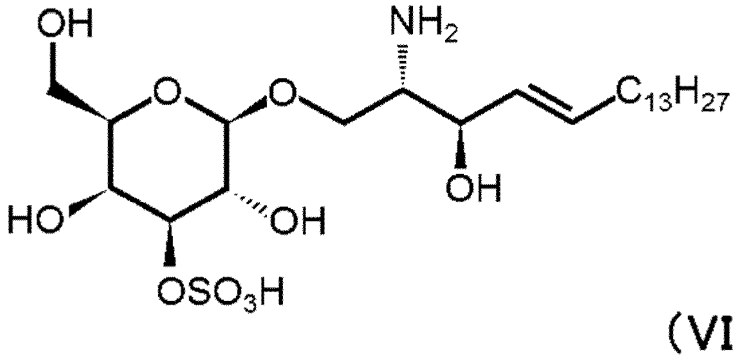

(VI)

The method of the invention for quantifying Hex4 included in cerebrospinal fluid preferably includes a step of adding an internal standard substance to a solution including the cerebrospinal fluid; a step of submitting the solution including the cerebrospinal fluid to liquid chromatography to obtain an eluate; and a step of subjecting the eluate to mass analysis.

The method of the invention for quantifying *lyso-GM1* included in cerebrospinal fluid preferably includes a step of adding an internal standard substance to a solution including the cerebrospinal fluid; a step of submitting the solution including the cerebrospinal fluid to liquid chromatography to obtain an eluate; and a step of subjecting the eluate to mass analysis.

The method of the invention for quantifying Fuc-GlcNAc-Asn included in cerebrospinal fluid preferably includes a step of adding an internal standard substance to a solution including the cerebrospinal fluid; a step of submitting the solution including the cerebrospinal fluid to liquid chromatography to obtain an eluate; and a step of subjecting the eluate to mass analysis.

The method of the invention for quantifying lyso-sulfatide included in cerebrospinal fluid preferably includes a step of adding an internal standard substance to a solution including the cerebrospinal fluid; a step of submitting the solution including the cerebrospinal fluid to liquid chromatography to obtain an eluate; and a step of subjecting the eluate to mass analysis.

According to the invention, the "internal standard substance" refers to a substance that is added in a certain amount to the standard samples for creating a calibration curve and a measurement sample, the substance being added for a method of relatively calculating the amount of a component to be analyzed, by determining the value of the ratio of a measured value originating from the measured internal standard substance and a measured value originating from the component to be analyzed.

There are no particular limitations in the biological species from which the cerebrospinal fluid is derived; however, examples include experimental animals including mice, rats, and monkeys, or human beings.

The quantitative value of Hex4 included in the cerebrospinal fluid has a positive correlation with the quantitative value of glycogen included in the brain. Therefore, glycogen included in the brain can be indirectly quantified by quantifying Hex4 included in the cerebrospinal fluid. Furthermore, the change in the concentration of glycogen included in the brain can be indirectly quantified by measuring the change in the concentration of Hex4 included in the cerebrospinal fluid. Therefore, for example, by quantifying Hex4 included in the cerebrospinal fluid of an experimental animal, a drug having drug efficacy of reducing Hex4 and/or glycogen accumulated in the cerebrospinal fluid can be screened. Furthermore, diagnosis of a patient who contracted a disease in which Hex4 and/or glycogen accumulates in the central nervous system can be conducted by quantifying Hex4 included in the human cerebrospinal fluid. Furthermore, the effect of treatment carried out in order to reduce Hex4 and/or glycogen accumulated in the central nervous system, the treatment being carried out for such a disease, can be investigated. Examples of the disease in which glycogen accumulates in the central nervous system include Pompe disease, which develops when acidic α-glucosidase (GAA) is genetically deficient. Examples of the treatment carried out in order to reduce glycogen accumulated in the body of a patient with Pompe disease, include enzyme replacement therapy of replenishing GAA. However, this enzyme replacement therapy is not to be carried out in order to reduce glycogen accumulated in the central nervous system.

The quantitative value of *lyso-GM1* included in the cerebrospinal fluid has a positive correlation with the quantitative value of *lyso-GM1* included in the brain. Therefore, *lyso-GM1* included in the brain can be indirectly quantified by quantifying *lyso-GM1* included in the cerebrospinal fluid. Furthermore, by measuring the change in the concentration of *lyso-GM1* included in the cerebrospinal fluid, the change in the concentration of *lyso-GM1* included in the brain can be indirectly quantified. Therefore, for example, by quantifying *lyso-GM1* included in the cerebrospinal fluid of an experimental animal, a drug having drug efficacy of reducing *lyso-GM1* and/or monosialoganglioside GM1 accumulated in the cerebrospinal fluid can be screened. Furthermore, diagnosis of a patient who contracted a disease in which *lyso-GM1* and/or monosialoganglioside GM1 accumulates in the central nervous system can be conducted by quantifying *lyso-GM1* included in the human cerebrospinal fluid. Furthermore, the effect of treatment carried out in order to reduce *lyso-GM1* and/or monosialoganglioside GM1 accumulated in the central nervous system, the treatment being carried out for such a disease, can be investigated. Examples of the disease in which monosialoganglioside GM1 accumulates in the central nervous system include GM1 gangliosidosis, which develops when β-galactosidase (β-Gal) is genetically deficient. Regarding the treatment carried out in order to reduce monosialoganglioside GM1 accumulated in the body of a patient with GM1 gangliosidosis, for example, enzyme replacement therapy of replenishing β-Gal may be assumed; however, this enzyme replacement therapy is not to be carried out in order to reduce monosialoganglioside GM1 accumulated in the central nervous system.

The quantitative value of Fuc-GlcNAc-Asn included in the cerebrospinal fluid has a positive correlation with the quantitative value of Fuc-GlcNAc-Asn included in the brain. Therefore, Fuc-GlcNAc-Asn included in the brain can be indirectly quantified by quantifying Fuc-GlcNAc-Asn included in the cerebrospinal fluid. Furthermore, by measuring the change in the concentration of Fuc-GlcNAc-Asn included in the cerebrospinal fluid, the change in the concentration of Fuc-GlcNAc-Asn included in the brain can be indirectly quantified. Therefore, for example, by quantifying Fuc-GlcNAc-Asn included in the cerebrospinal fluid of an experimental animal, a drug having drug efficacy of reducing Fuc-GlcNAc-Asn and/or α-L-fucoside accumulated in the cerebrospinal fluid can be screened. Furthermore, diagnosis of a patient who contracted a disease in which Fuc-GlcNAc-Asn and/or α-L-fucoside accumulates in the central nervous system can be conducted by quantifying Fuc-GlcNAc-Asn included in the human cerebrospinal fluid. Furthermore, the effect of treatment carried out in order to reduce Fuc-GlcNAc-Asn and/or α-L-fucoside accumulated in the central nervous system, the treatment being carried out for such a disease, can be investigated. Examples of the disease in which α-L-fucoside accumulates in the central nervous system include fucosidosis, which develops when α-L-fucosidase (FUCA1) is genetically deficient. Regarding the treatment carried out in order to reduce α-L-fucoside accumulated in the body of a patient with fucosidosis, for example, enzyme replacement therapy of replenishing FUCA1 may be assumed; however, this enzyme replacement therapy is not to be carried out in order to reduce α-L-fucoside accumulated in the central nervous system.

The quantitative value of lyso-sulfatide included in the cerebrospinal fluid has a positive correlation with the quantitative value of lyso-sulfatide included in the brain. Therefore, lyso-sulfatide included in the brain can be indirectly quantified by quantifying lyso-sulfatide included in the cerebrospinal fluid. Furthermore, by measuring the change in the concentration of lyso-sulfatide included in the cerebrospinal fluid, the change in the concentration of *lyso*-sulfatide included in the brain can be indirectly quantified. Therefore, for example, by quantifying *lyso*-sulfatide included in the cerebrospinal fluid of an experimental animal, a drug having drug efficacy of reducing *lyso*-sulfatide and/or sulfatide accumulated in the cerebrospinal fluid can be screened. Furthermore, diagnosis of a patient who contracted a disease in which *lyso*-sulfatide and/or sulfatide accumulates in the central nervous system can be conducted by quantifying *lyso*-sulfatide included in the human cerebrospinal fluid. Furthermore, the effect of treatment carried out in order to reduce *lyso*-sulfatide and/or sulfatide accumulated in the central nervous system, the treatment being carried out for such a disease,

can be investigated. Examples of the disease in which sulfatide accumulates in the central nervous system include metachromatic leukodystrophy, which develops when arylsulfatase A (ARSA) is genetically deficient. Regarding the treatment carried out in order to reduce sulfatide accumulated in the body of a patient with metachromatic leukodystrophy, for example, enzyme replacement therapy of replenishing ARSA may be assumed; however, this enzyme replacement therapy is not to be carried out in order to reduce sulfatide accumulated in the central nervous system.

With regard to the cerebrospinal fluid according to the invention, a cerebrospinal fluid collected from a test subject such as an experimental animal may be immediately analyzed, or a cerebrospinal fluid that has been stored in a frozen state may be thawed and analyzed. When a large number of cerebrospinal fluids are analyzed, the operation efficiency can be increased by freezing and storing the cerebrospinal fluids and analyzing these all at once.

According to an embodiment of the present invention, cerebrospinal fluid is submitted to liquid chromatography, and an eluate including Hex4 is fractionated. The material for the stationary phase used for this liquid chromatography is not particularly limited as long as the material can be used to fractionate an eluate including Hex4 from the cerebrospinal fluid; however, a material that can first adsorb Hex4 and then elute the same is preferred. The material for the stationary phase is preferably a material capable of adsorbing Hex4 by ionic interaction, hydrophobic interaction, hydrophilic interaction, or the like. Hex4 is highly polar. Therefore, regarding the material that can first adsorb this Hex4 and then elute the same, a material capable of retaining Hex4 by hydrophilic interaction can be suitably used. A preferred example of the material for the stationary phase of such high-performance liquid chromatography may be a material containing a primary amide group. The ACQUITY UPLC BEH Amide Column (Nihon Waters K.K.) used in the following Example 6 is a suitable example of the stationary phase material including a first amide group.

According to an embodiment of the invention, cerebrospinal fluid is submitted to liquid chromatography, and an eluate including *lyso-GM1* is fractionated. The material for the stationary phase used for this liquid chromatography is not particularly limited as long as the material can be used to fractionate an eluate including *lyso-GM1* from the cerebrospinal fluid; however, a material that can first adsorb *lyso-GM1* and then elute the same is preferred. The material for the stationary phase is preferably a material capable of adsorbing *lyso-GM1* by ionic interaction, hydrophobic interaction, hydrophilic interaction, or the like. *Lyso-GM1* has a long carbon chain. Therefore, as a material that can first adsorb this *lyso-GM1* and then elute the same, a material capable of retaining *lyso-GM1* by hydrophobic interaction can be suitably used. A preferred example of the material for the stationary phase of such high-performance liquid chromatography may be a porous silica gel having the surface modified with an octadecylsilyl group (ODS). Cadenza CW-C18 (Imtakt Corp.) used in the following Example 16 is a suitable example of an ODS column.

According to an embodiment of the invention, cerebrospinal fluid is submitted to liquid chromatography, and an eluate including Fuc-GlcNAc-Asn is fractionated. The material for the stationary phase used for this liquid chromatography is not particularly limited as long as the material can be used to fractionate an eluate including Fuc-GlcNAc-Asn from the cerebrospinal fluid; however, a material that can first adsorb Fuc-GlcNAc-Asn and then elute the same is preferred. The material for the stationary phase is preferably a material capable of adsorbing Fuc-GlcNAc-Asn by ionic interaction, hydrophobic interaction, hydrophilic interaction, or the like. Fuc-GlcNAc-Asn is highly polar. Therefore, regarding the material that can first adsorb this Fuc-GlcNAc-Asn and then elute the same, a material capable of retaining Fuc-GlcNAc-Asn by hydrophilic interaction can be suitably used. A preferred example of the material for the stationary phase of such high-performance liquid chromatography may be a porous silica gel having the surface modified with an aminopropyl group. The Unison UK-Amino (Imtakt Corp.) used in the following Example 26 is a suitable example of an aminopropyl type column.

According to an embodiment of the invention, cerebrospinal fluid is submitted to liquid chromatography, and an eluate including lyso-sulfatide is fractionated. The material for the stationary phase used for this liquid chromatography is not particularly limited as long as the material can be used to fractionate an eluate including lyso-sulfatide from the cerebrospinal fluid; however, a material that can first adsorb lyso-sulfatide and then elute the same is preferred. The material for the stationary phase is preferably a material capable of adsorbing lyso-sulfatide by ionic interaction, hydrophobic interaction, hydrophilic interaction, or the like. Lyso-sulfatide has a long carbon. Therefore, as a material that can first adsorb this lyso-sulfatide and then elute the same, a material capable of retaining *lyso*-sulfatide by hydrophobic interaction can be suitably used. A preferred example of the material for the stationary phase of such high-performance liquid chromatography may be a porous silica gel having the surface modified with an octadecylsilyl group (ODS). Cadenza CW-C18 (Imtakt Corp.) used in the following Example 35 is a suitable example of an ODS column.

In the cerebrospinal fluid, a certain amount of an internal standard substance is added before the cerebrospinal fluid is submitted to liquid chromatography. The internal standard substance according to an embodiment of the invention is, in particular, a Glc4 in which any one or a plurality of the six carbon atoms marked with asterisks in the Glc4 molecule represented by the following Formula (VII) is $^{13}C$, and preferably a Glc4 in which all of these six carbon atoms are $^{13}C$ (6-a-D-Glucopyranosyl maltotriose-13C6). This internal standard substance is added particularly in the case of quantifying Hex4 included in cerebrospinal fluid.

[Chemical Formula 7]

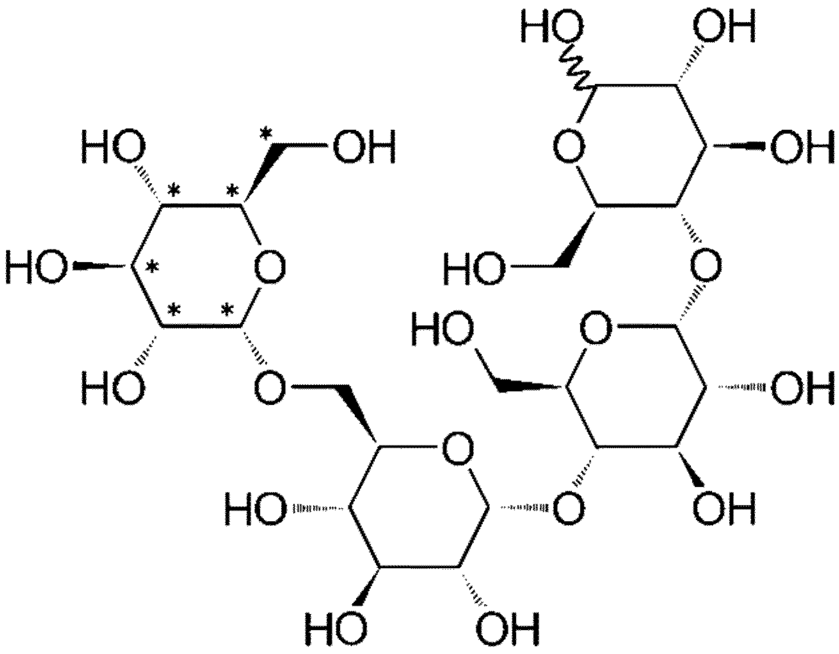

(VII)

As the internal standard substance to be added in the case of quantifying Hex4 included in cerebrospinal fluid, a compound in which any one or a plurality of the six carbon atoms marked with asterisks in the M4 molecule represented by the following Formula (XXV) is $^{13}C$ can also be used. M4 in which all of these six carbon atoms are $^{13}C$ is suitable as the internal standard substance.

[Chemical Formula 25]

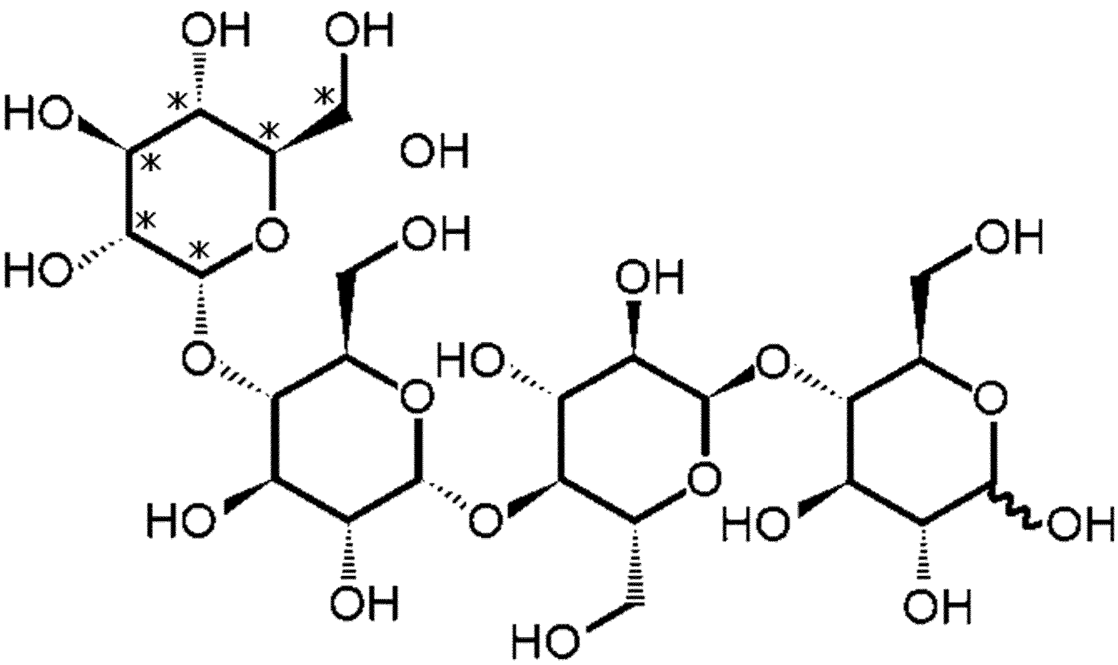

(XXV)

In the cerebrospinal fluid, a certain amount of an internal standard substance is added before the cerebrospinal fluid is submitted to liquid chromatography. The internal standard substance according to an embodiment of the invention is, in particular, a compound represented by the following Formula (VIII), N-Glycinated lyso-ceramide trihexoside. This internal standard substance is added particularly in the case of quantifying *lyso*-monosialoganglioside GM1 included in cerebrospinal fluid.

[Chemical Formula 8]

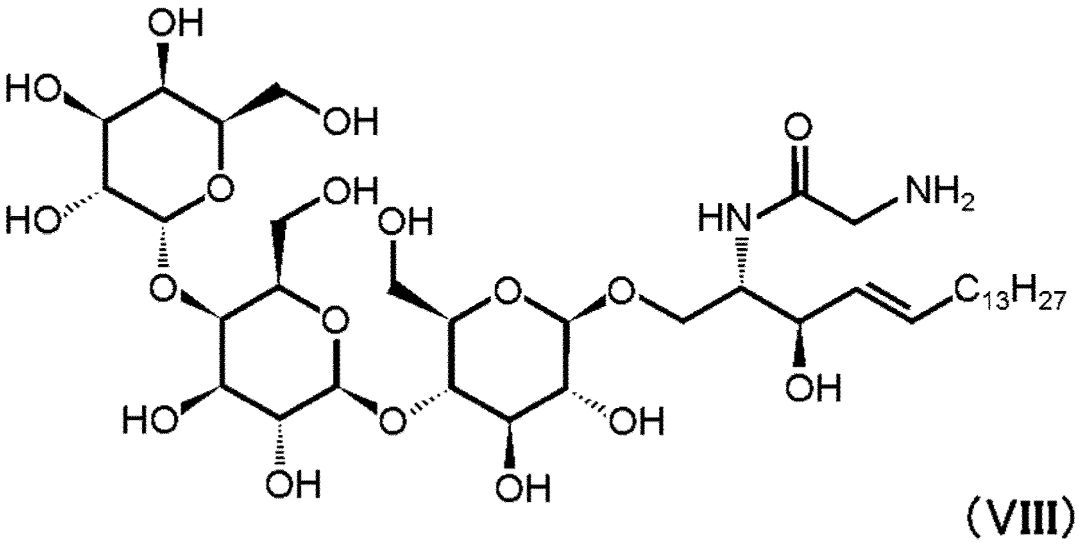

(VIII)

In the cerebrospinal fluid, a certain amount of an internal standard substance is added before the cerebrospinal fluid is submitted to liquid chromatography. The internal standard substance according to an embodiment of the invention is, in particular, a compound represented by the following Formula (IX), Fuc1-α-3GlcNAc1-β-OMe (Fuc-GlcNAc-OMe). This internal standard substance is added particularly in the case of quantifying Fuc-GlcNAc-Asn included in cerebrospinal fluid.

[Chemical Formula 9]

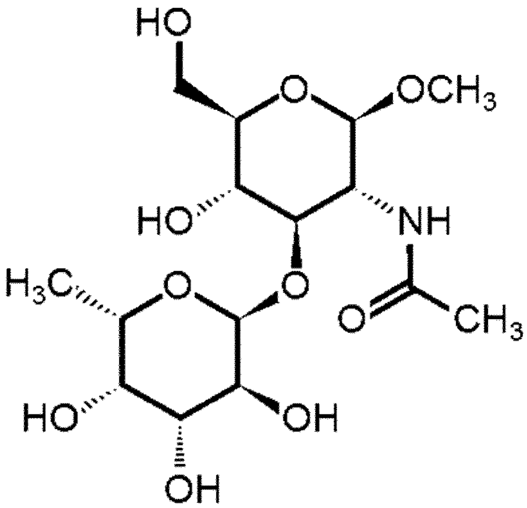

(IX)

In the cerebrospinal fluid, a certain amount of an internal standard substance is added before the cerebrospinal fluid is submitted to liquid chromatography. The internal standard substance according to an embodiment of the invention is, in particular, a compound represented by the following Formula (X), N-Glycinated lyso-sulfatide. This internal standard substance is added particularly in the case of quantifying lyso-sulfatide included in cerebrospinal fluid.

[Chemical Formula 10]

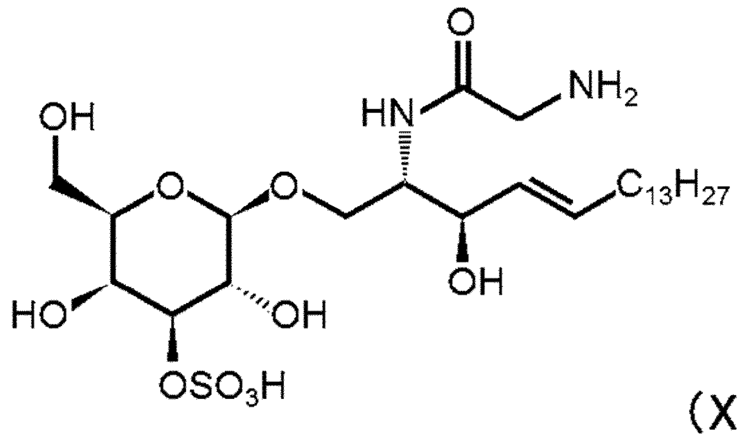

(X)

A flow channel from the outflow port of liquid chromatography is connected to a mass analyzer, and the eluate from liquid chromatography is sequentially transported to mass analysis.

The mass analyzer that can be used at this time is not particularly limited. For example, the mass analyzer may be an apparatus employing any ionization method, including a photoionization method (APPI), an electron ionization method (EI), a chemical ionization method, an electric desorption method, a high-speed atomic impact method (FAB), a matrix-assisted laser desorption ionization method (MALDI), and an electrospray ionization method (ESI), as an ion source for ionizing the molecules to be analyzed. Furthermore, the mass analyzer may be such that the analysis unit for separating ionized molecules is of any type, including magnetic field deflection type, quadrupole type, ion trap type, and tandem quadrupole type.

A mass analyzer having an ion source operated by an electrospray ionization method (ESI) operated in a cation mode; and an analysis unit of tandem quadrupole type, can be suitably used for the method of the invention. A tandem quadrupole type mass analyzer is a mass analyzer in which a quadrupole (Q1) that functions as a mass filter, a quadrupole (Q2) that functions as a collision cell, and a quadrupole (Q3) that functions as a mass filter are disposed in series. In the quadrupole (Q1), a target precursor ion is separated from a plurality of ions generated by ionization, based on the mass-to-charge ratio (m/z) of the ion. Next, the precursor ion is caused to collide with an inert gas or the like (for example, argon) in the collision cell (Q2) to generate a product ion (fragment ion). Next, in the quadrupole (Q3), the obtained product ion can be selectively detected based on the mass-to-charge ratio (m/z), and the substance causative of the product ion can be quantified.

A specific example of a method for generating a precursor ion and a product ion from Glc4 by means of a tandem quadrupole type mass analyzer will be described below, by taking Glc4, which is one of Hex4, as an example. First, a precursor ion having a mass-to-charge ratio (m/z) of 665.1 is obtained by ionizing Glc4. One of the theoretically conceivable chemical formulas of the precursor ion is shown as the following Formula (XI):

[Chemical Formula 11]

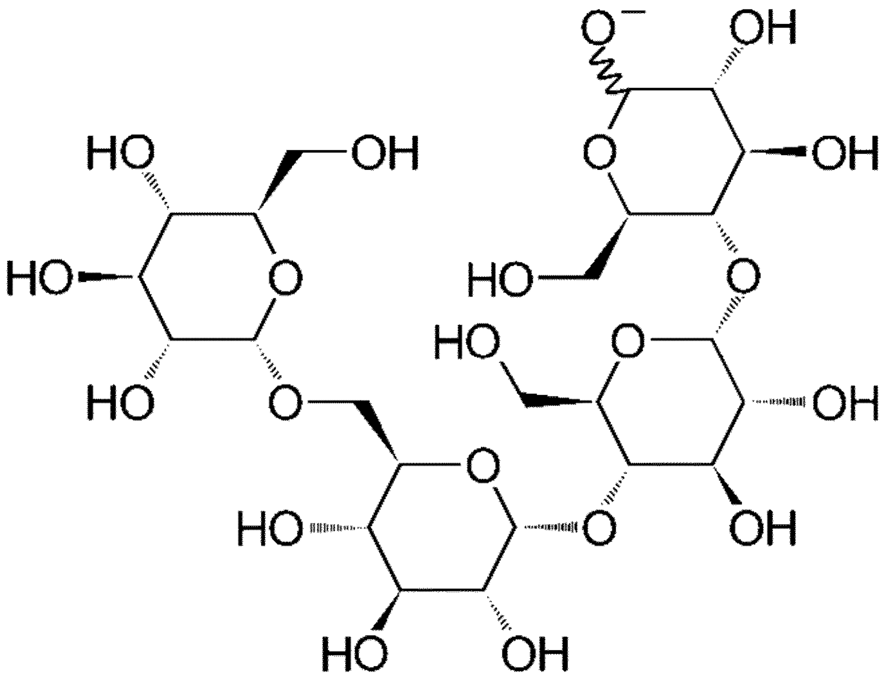

(XI)

When the above-described precursor ion is separated and cleaved in the collision cell (Q2), a product ion ([M-H]$^-$) having a mass-to-charge ratio (m/z) of 179.0 is obtained. The theoretically conceivable chemical formula of the product ion ([M-H]$^-$) is represented by the following Formula (XII):

[Chemical Formula 12]

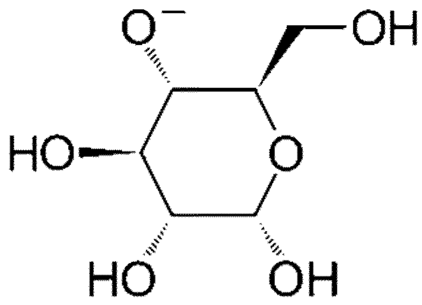

(XII)

The amount of Glc4 included in a sample can be measured by measuring the product ion generated from Glc4 by the above-described method of using liquid chromatography and a tandem quadrupole type mass analyzer. Incidentally, Glc4 and Hex4 compounds other than Glc4 are present in the cerebrospinal fluid, and among the Hex4 compounds other than Glc4, there are also some Hex4 compounds that are not completely separated from Glc4 by liquid chromatography and generate the same product ion according to the measured values of the tandem quadrupole type mass analyzer. Therefore, the measured values of the cerebrospinal fluid include values originating from the Hex4 compounds other than Glc4; however, since a significant number of Hex4 compounds present in the cerebrospinal fluid are assumed to produce the product ion represented by Formula (XII), the measured value of Glc4 can be regarded as the measured value of Hex4. For example, M4 represented by Formula (XXV) is also considered to produce the product ion represented by Formula (XII) by the above-described treatment. An eluate obtained by submitting cerebrospinal fluid to liquid chromatography is analyzed using a tandem quadrupole type mass analyzer, and thereby the area of a peak (detection peak) detected on the chromatographic chart corresponding to the product ion (XII) having a mass-to-charge ratio (m/z) of 179.0 is calculated. The value of dividing this area by the area of the peak (internal standard peak) corresponding to a product ion originating

from the internal standard substance included in the same eluate is determined. Meanwhile, when the internal standard substance is such that all of the six carbon atoms marked with asterisks in the Glc4 molecule represented by the above-described Formula (VII) are $^{13}C$, the mass-to-charge ratio (m/z) of the product ion originating from the internal standard substance is 185.0.

Standard samples for calibration curve are similarly analyzed by liquid chromatography and a mass analysis method, the area of the detection peak is divided by the area of the internal standard peak for each standard sample for calibration curve, and based on the obtained values, a calibration curve is created. Then, the value obtained by submitting cerebrospinal fluid to liquid chromatography and analyzing an eluate obtained therefrom is interpolated into this calibration curve, and the value that can be regarded as the concentration of Hex4 included in the sample can be measured. According to an embodiment of the invention, when the concentration of Hex4 is mentioned, the concentration of the measured value obtained in this manner. M4 can also be used as the internal standard substance, in place of Glc4.

A specific example of a method for generating a precursor ion and a product ion from *lyso-GM1* by means of a tandem quadrupole type mass analyzer will be described below. First, a precursor ion having a mass-to-charge ratio (m/z) of 640.8 is obtained by ionizing *lyso-GM1*. One of the theoretically conceivable chemical formulas of the precursor ion is represented by the following Formula (XIII):

[Chemical Formula 13]

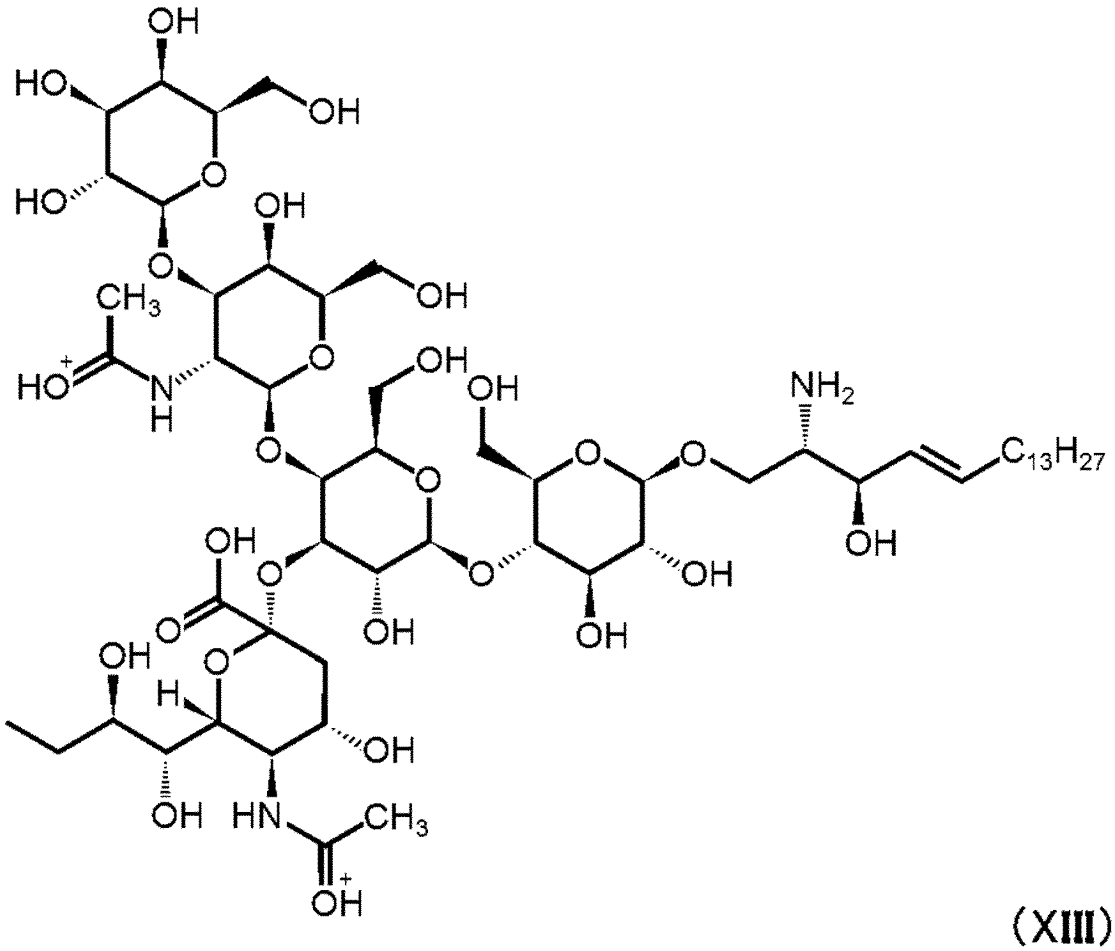

(XIII)

When the above-described precursor ion is separated and cleaved in the collision cell (Q2), a product ion ($[M+2H]^{2+}$) having a mass-to-charge ratio (m/z) of 282.3 is obtained. The theoretically conceivable chemical formula of the product ion ($[M+2H]^{2+}$) is represented by the following Formula (XIV):

[Chemical Formula 14]

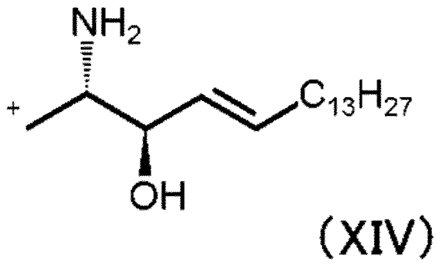

(XIV)

Next, a specific example of a method for generating a precursor ion and a product ion from an internal standard substance, N-Glycinated lyso-ceramide trihexoside, by means of a tandem quadrupole type mass analyzer will be described below. First, a precursor ion having a mass-to-charge ratio (m/z) of 843.5 is obtained by ionizing N-Glycinated lyso-ceramide trihexoside. One of the theoretically conceivable chemical formulas of the precursor ion is represented by the following Formula (XV):

[Chemical Formula 15]

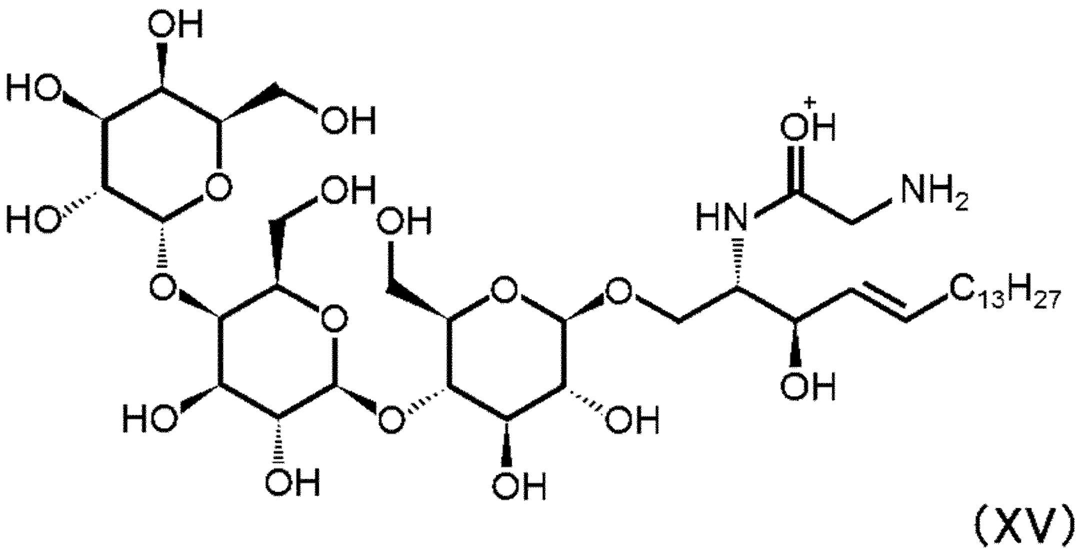

(XV)

When the above-described precursor ion is separated and cleaved in the collision cell (Q2), a product ion ($[M+H]^+$) having a mass-to-charge ratio (m/z) of 264.3 is obtained. The theoretically conceivable chemical formula of the product ion ($[M+H]^+$) is represented by the following Formula (XVI):

[Chemical Formula 16]

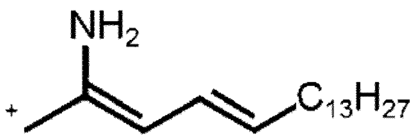

(XVI)

The amount of *lyso-GM1* included in a sample can be measured by measuring the product ion generated from *lyso-GM1* by the above-described method of using liquid chromatography and a tandem quadrupole type mass analyzer. An eluate obtained by submitting cerebrospinal fluid to liquid chromatography is analyzed using a tandem quadrupole type mass analyzer, and thereby the area of a peak (detection peak) detected on the chromatographic chart corresponding to the product ion (XIV) having a mass-to-charge ratio (m/z) of 282.3 is calculated. The value of dividing this area by the area of the peak (internal standard peak) corresponding to a product ion originating from the internal standard substance included in the same eluate is determined. Incidentally, the mass-to-charge ratio (m/z) of the product ion originating from

the internal standard substance is 264.3.

Standard samples for calibration curve are similarly analyzed by liquid chromatography and a mass analysis method, the area of the detection peak is divided by the area of the internal standard peak for each standard sample for calibration curve, and based on the obtained values, a calibration curve is created. Then, the value obtained by submitting cerebrospinal fluid to liquid chromatography and analyzing an eluate obtained therefrom is interpolated into this calibration curve, and the concentration of *lyso-GM1* included in the sample can be measured.

A specific example of a method for generating a precursor ion and a product ion from Fuc-GlcNAc-Asn by a tandem quadrupole type mass analyzer will be described below. First, a precursor ion having a mass-to-charge ratio (m/z) of 482.2 is obtained by ionizing Fuc-GlcNAc-Asn. One of the theoretically conceivable chemical formulas of the precursor ion is represented by the following Formula (XVII):

[Chemical Formula 17]

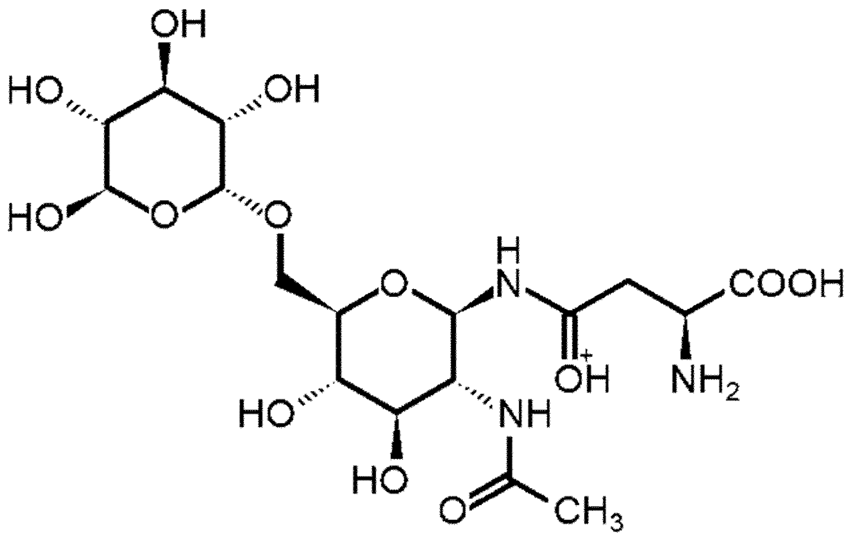

(XVII)

The above-described precursor is separated and cleaved in the collision cell (Q2), and thereby a product ion ($[M+H]^+$) having a mass-to-charge ratio (m/z) of 336.1 is obtained. The theoretically conceivable chemical formula of the product ion ($[M+H]^+$) is represented by the following Formula (XVIII):

[Chemical Formula 18]

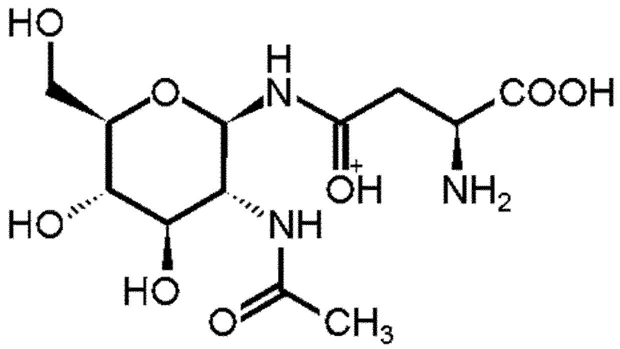

(XVIII)

Next, a specific example of a method for generating a precursor ion and a product ion from an internal standard substance, Fuc-GlcNAc-OMe, by means of a tandem quadrupole type mass analyzer will be described. First, a precursor ion having a mass-to-charge ratio (m/z) of 382.2 is obtained by ionizing Fuc-GlcNAc-OMe. One of the theoretically

conceivable chemical formulas of the precursor ion is represented by the following Formula (XIX):

[Chemical Formula 19]

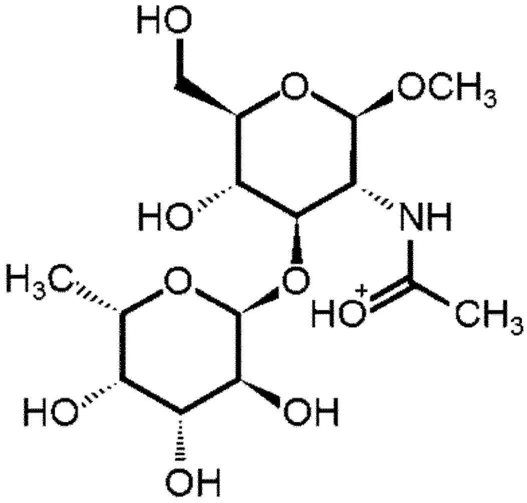

(XIX)

The above-described precursor ion is separated and cleaved in the collision cell (Q2), and thereby a product ion ($[M+H]^+$) having a mass-to-charge ratio (m/z) of 204.1 is obtained. The theoretically conceivable chemical formula of the product ion ($[M+H]^+$) is represented by the following Formula (XX):

[Chemical Formula 20]

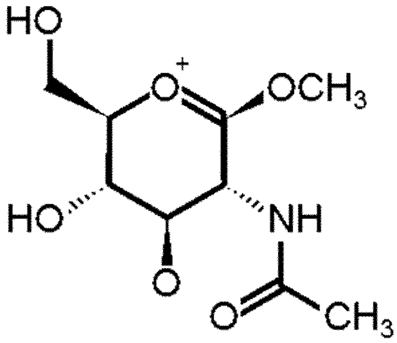

(XX)

The amount of Fuc-GlcNAc-Asn included in a sample can be measured by measuring the product ion produced from Fuc-GlcNAc-Asn by the above-described method of using liquid chromatography and a tandem quadrupole type mass analyzer. An eluate obtained by submitting cerebrospinal fluid to liquid chromatography is analyzed using a tandem quadrupole type mass analyzer, and thereby the area of a peak (detection peak) detected on the chromatographic chart corresponding to the product ion (XVIII) having a mass-to-charge ratio (m/z) of 336.1 is calculated. The value of dividing this area by the area of the peak (internal standard peak) corresponding to a product ion originating from the internal standard substance included in the same eluate is determined. Incidentally, the mass-to-charge ratio (m/z) of the product ion originating from the internal standard substance is 204.1.

Standard samples for calibration curve are similarly analyzed by liquid chromatography and a mass analysis method, the area of the detection peak is divided by the area of the internal standard peak for each standard sample for calibration curve, and based on the obtained values, a calibration curve is created. Then, the value obtained by submitting cerebrospinal fluid to liquid chromatography and analyzing an eluate obtained therefrom is interpolated into this calibration curve, and the concentration of Fuc-GlcNAc-Asn included in the sample can be measured.

A specific example of a method for generating a precursor ion and a product ion from lyso-sulfatide by means of a

tandem quadrupole type mass analyzer will be described below. First, a precursor ion having a mass-to-charge ratio (m/z) of 542.3 is obtained by ionizing lyso-sulfatide. One of the theoretically conceivable chemical formulas of the precursor ion is represented by the following Formula (XXI):

[Chemical Formula 21]

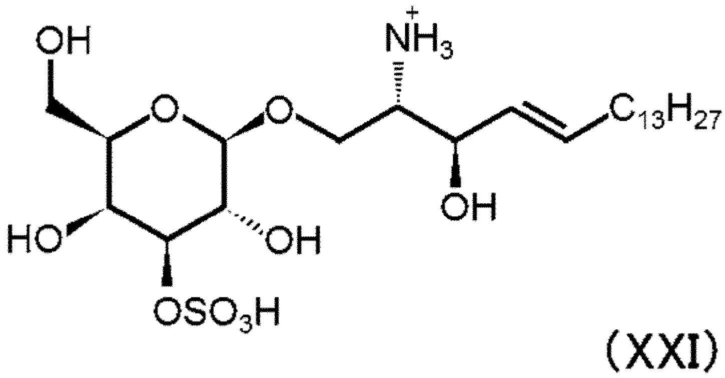

(XXI)

The above-described precursor ion is separated and cleaved in the collision cell (Q2), and thereby a product ion ($[M+H]^+$) having a mass-to-charge ratio (m/z) of 282.3 is obtained. The theoretically conceivable chemical formula of the product ion ($[M+H]^+$) is represented by the following Formula (XXII):

[Chemical Formula 22]

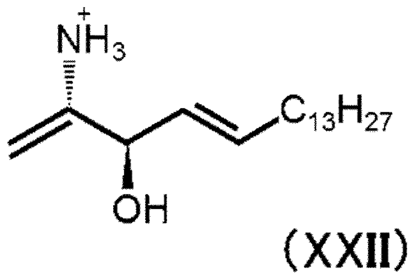

(XXII)

Next, a specific example of a method for generating a precursor ion and a product ion from an internal standard substance, N-Glycinated lyso-sulfatide, by means of a tandem quadrupole type mass analyzer will be described. First, a precursor ion having a mass-to-charge ratio (m/z) of 599.3 is obtained by ionizing N-Glycinated lyso-sulfatide. One of the theoretically conceivable chemical formulas of the precursor ion is represented by the following Formula (XXIII):

[Chemical Formula 23]

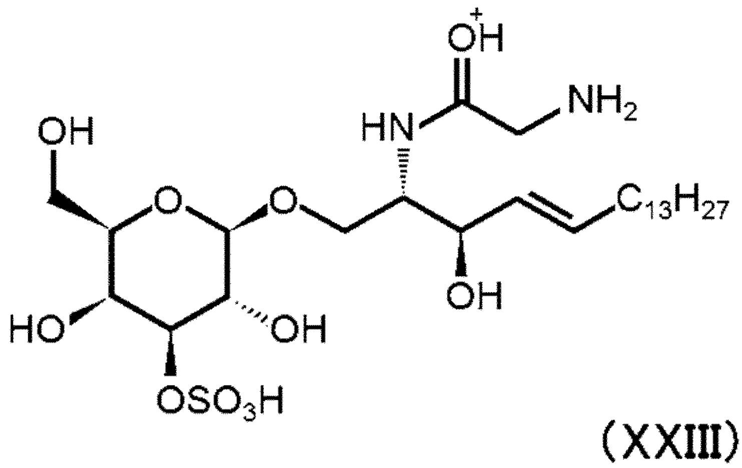

(XXIII)

The above-described precursor ion is separated and cleaved in the collision cell (Q2), and thereby a product ion ($[M+H]^+$) having a mass-to-charge ratio (m/z) of 339.3 is obtained. The theoretically conceivable chemical formula of the product ion ($[M+H]^+$) is represented by the following Formula (XXIV):

[Chemical Formula 24]

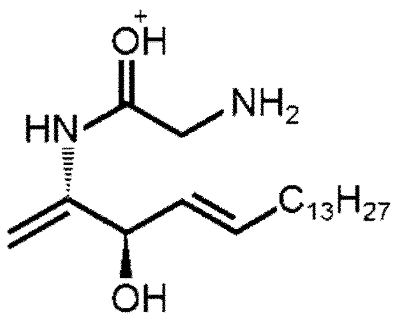

(XXIV)

The amount of lyso-sulfatide included in a sample can be measured by measuring a product ion generated from lyso-sulfatide by means of the above-described method of using liquid chromatography and tandem quadrupole type mass analyzer. An eluate obtained by submitting cerebrospinal fluid to liquid chromatography is analyzed using a tandem quadrupole type mass analyzer, and thereby the area of a peak (detection peak) detected on the chromatographic chart corresponding to the product ion (XXII) having a mass-to-charge ratio (m/z) of 282.3 is calculated. The value of dividing this area by the area of the peak (internal standard peak) corresponding to a product ion originating from the internal standard substance included in the same eluate is determined. Incidentally, the mass-to-charge ratio (m/z) of the product ion originating from the internal standard substance is 339.3.

Standard samples for calibration curve are similarly analyzed by liquid chromatography and a mass analysis method, the area of the detection peak is divided by the area of the internal standard peak for each standard sample for calibration curve, and based on the obtained values, a calibration curve is created. Then, the value obtained by submitting cerebrospinal fluid to liquid chromatography and analyzing an eluate obtained therefrom is interpolated into this calibration curve, and the concentration of lyso-sulfatide included in the sample can be measured.

One of the diseases in which glycogen accumulates in the body is Pompe disease. As a treatment method for Pompe disease, enzyme replacement therapy of replenishing acidic $\alpha$-glucosidase in a patient by performing intravenous drip infusion has been carried out. Regarding a method for evaluating the effect of enzyme replacement therapy, a method of quantitatively determining the glycogen concentration in muscle tissue has been generally carried out. However, with regard to abnormalities in the central nervous system (CNS), the method is not effective because enzymes cannot cross the Blood-Brain Barrier (BBB). Therefore, regarding the purpose of evaluating the effect of conventional enzyme replacement therapy, the concentration of glycogen included in cerebrospinal fluid is not considered to be a target of measurement.

Pompe disease is a disease that genetically partially or completely lacks GAA activity. Due to deficit and deficiency of this enzyme, glycogen accumulates in the body of a patient. Main symptoms of Pompe disease include

invasive symptoms to myocardium, skeletal muscles, and diaphragm. Clinically, Pompe disease is classified into three types, namely, classical infant type, infant type, and adult type; however, in all cases, muscle weakness and respiratory disorders caused by accumulation of glycogen in the myocardium, skeletal muscles, and diaphragm are observed. In recent years, with regard to the respiratory disorders associated with Pompe disease, not only the disorder of the muscular contraction ability of the diaphragm is the cause of the respiratory disorders, but the disorder of the phrenic nerve that controls the respiratory organs is also considered to be one of the causes. Therefore, it is considered to be important to remove the glycogen accumulated in the central nervous system even for Pompe disease, which has been considered not to be accompanied by abnormalities in the central nervous system. However, measuring glycogen accumulated in the tissues of the central nervous system is not practical because it is necessary to perform biopsy of the cells of the central nervous system.

As shown in Example 10, the quantitative value of Hex4 included in cerebrospinal fluid, which is measured by the method of an embodiment of the invention, correlates with the concentration of glycogen included in the brain tissue of the same individual. That is, according to the method of the invention, the amount of glycogen accumulated in the central nervous system can be evaluated by measuring Hex4 included in the cerebrospinal fluid.

As described above, even for Pompe disease, it is considered to be important to remove the glycogen accumulated in the central nervous system. In order to cause a drug administered by intravenous injection or the like to reach the central nervous system, the drug needs to cross the blood-brain barrier. When it is attempted to develop a therapeutic drug for Pompe disease that can cross the blood-brain barrier and can exhibit effects in the central nervous system, there is a need to evaluate the drug efficacy in the central nervous system. The method of the invention meets such a demand. Even in the case of developing a drug of a type that is directly administered into the central nervous system without causing the drug to cross the blood-brain barrier, the same also applies.

Without being limited to Pompe disease, a patient suffering from a disease in which Hex4 and/or glycogen accumulates in the central nervous system can be screened by the method of an embodiment of the invention, by measuring the concentration of Hex4 included in the cerebrospinal fluid and performing screening based on the measured values thus obtained. When the measured value is abnormally higher than the value of a normal person, the test subject can be determined to be a patient suffering from the disease. The method of the invention can be carried out for the purpose of conducting such determination.

When a patient with a disease in which Hex4 and/or glycogen accumulates in the central nervous system is treated, the effect of the treatment can be checked by measuring the concentration of Hex4 included in the cerebrospinal fluid of the patient before the treatment and after the treatment and measuring the amount of decrease of these after the treatment. As the amount of decrease is larger, the therapeutic effect can be considered to be higher. The method of the invention can be carried out for the purpose of conducting confirmation of such therapeutic effect. For example, when the concentration of Hex4 included in the blood of the patient has been decreased preferably by 10% or more, for example, when the concentration has been decreased by 20% or more, 30% or more, or 50% or more, respectively, before and after the treatment, it can be determined that there has been therapeutic effect.

One of the diseases in which monosialoganglioside GM1 accumulates in the body is GM1 gangliosidosis. As a treatment method for GM1 gangliosidosis, enzyme replacement therapy of replenishing β-galactosidase in a patient by performing intravenous drip infusion is assumed; however, currently there is no clinically applied treatment method for that disease. In a case where enzyme replacement therapy has been carried out, regarding a method for evaluating the effect of the therapy, a method of quantitatively determining the concentration of monosialoganglioside GM1 in muscle tissue is assumed. However, the method is not effective for abnormalities in the central nervous system (CNS) because enzymes cannot cross the blood-brain barrier (BBB). Therefore, regarding the purpose of evaluating the effect of conventional enzyme replacement therapy, the concentration of monosialoganglioside GM1 included in the cerebrospinal fluid is not regarded as the target of measurement.

GM1 gangliosidosis is a disease that genetically partially or completely lacks β-galactosidase activity. Due to deficit and deficiency of this enzyme, monosialoganglioside GM1 accumulates in the body of a patient. Main symptoms of GM1 gangliosidosis include central nerve system disorders. Clinically, GM1 gangliosidosis is classified into four types, namely, infant type, juvenile type, adult type, and Morquio's disease type B, and central nervous system disorders are recognized in all of the types except for Morquio's disease type B. Therefore, it is considered important to remove monosialoganglioside GM1 accumulated in the central nervous system for GM1 gangliosidosis. However, measuring monosialoganglioside GM1 accumulated in the tissues of the central nervous system is not practical because it is necessary to perform biopsy of the cells of the central nervous system.

As shown in Example 20, the quantitative value of *lyso-GM1* included in cerebrospinal fluid, which is measured by the method of an embodiment of the invention, correlates with the concentration of monosialoganglioside GM1 included in the brain tissue of the same individual. That is, according to the method of the invention, the amount of monosialoganglioside GM1 accumulated in the central nervous system can be evaluated by measuring *lyso-GM1* included in the cerebrospinal fluid.

As described above, even for GM1 gangliosidosis, it is considered important to remove monosialoganglioside

GM1 accumulated in the central nervous system. In order to cause a drug administered by intravenous injection or the like to reach the central nervous system, the drug needs to cross the blood-brain barrier. When it is attempted to develop a therapeutic drug for GM1 gangliosidosis that can cross the blood-brain barrier and can exhibit effects in the central nervous system, there is a need to evaluate the drug efficacy in the central nervous system. The method of the invention meets such a demand. Even in the case of developing a drug of a type that is directly administered into the central nervous system without causing the drug to cross the blood-brain barrier, the same also applies.

Without being limited to GM1 gangliosidosis, a patient suffering from a disease in which *lyso-GM1* and/or monosialoganglioside GM1 accumulates in the central nervous system can be screened by the method of an embodiment of the invention, by measuring the concentration of *lyso-GM1* included in the cerebrospinal fluid and performing screening based on the measured values thus obtained. When the measured value is abnormally higher than the value of a normal person, the test subject can be determined to be a patient suffering from the disease. The method of the invention can be carried out for the purpose of conducting such determination.

When a patient with a disease in which *lyso-GM1* and/or monosialoganglioside GM1 accumulates in the central nervous system is treated, the effect of the treatment can be checked by measuring the concentration of *lyso-GM1* included in the cerebrospinal fluid of the patient before the treatment and after the treatment and measuring the amount of decrease of these after the treatment. As the amount of decrease is larger, the therapeutic effect can be considered to be higher. The method of the invention can be carried out for the purpose of conducting confirmation of such therapeutic effect. For example, when the concentration of *lyso*-GM1 included in the blood of the patient has been decreased preferably by 10% or more, for example, when the concentration has been decreased by 20% or more, 30% or more, or 50% or more, respectively, before and after the treatment, it can be determined that there has been therapeutic effect.

One of the diseases in which α-L-fucoside accumulates in the body is fucosidosis. As a treatment method for fucosidosis, enzyme replacement therapy of replenishing α-L-fucosidase in a patient by performing intravenous drip infusion is assumed; however, currently there is no clinically applied treatment method for that disease. In a case where enzyme replacement therapy has been carried out, regarding a method for evaluating the effect of the therapy, a method of quantitatively determining the concentration of α-L-fucoside in muscle tissue is assumed. However, the method is not effective for abnormalities in the central nervous system (CNS) because enzymes cannot cross the blood-brain barrier (BBB). Therefore, regarding the purpose of evaluating the effect of conventional enzyme replacement therapy, the concentration of α-L-fucoside included in the cerebrospinal fluid is not regarded as the target of measurement.

Fucosidosis is a disease that genetically partially or completely lacks α-L-fucosidase activity. Due to deficit and deficiency of this enzyme, α-L-fucoside accumulates in the body of a patient. Main symptoms of fucosidosis include central nerve system disorders. Clinically, fucosidosis is classified into two types, namely, severe type and mild type, and central nervous system disorders are recognized in both types. Therefore, it is considered important to remove α-L-fucoside accumulated in the central nervous system for fucosidosis. However, measuring α-L-fucoside accumulated in the tissues of the central nervous system is not practical because it is necessary to perform biopsy of the cells of the central nervous system.

As shown in Example 29, the quantitative value of Fuc-GlcNAc-Asn included in cerebrospinal fluid, which is measured by the method of an embodiment of the invention, correlates with the concentration of Fuc-GlcNAc-Asn included in the brain tissue of the same individual. That is, according to the method of the invention, the amount of Fuc-GlcNAc-Asn accumulated in the central nervous system can be evaluated by measuring Fuc-GlcNAc-Asn included in the cerebrospinal fluid.

As described above, even for fucosidosis, it is considered important to remove α-L-fucoside accumulated in the central nervous system. In order to cause a drug administered by intravenous injection or the like to reach the central nervous system, the drug needs to cross the blood-brain barrier. When it is attempted to develop a therapeutic drug for fucosidosis that can cross the blood-brain barrier and can exhibit effects in the central nervous system, there is a need to evaluate the drug efficacy in the central nervous system. The method of the invention meets such a demand. Even in the case of developing a drug of a type that is directly administered into the central nervous system without causing the drug to cross the blood-brain barrier, the same also applies.

Without being limited to fucosidosis, a patient suffering from a disease in which Fuc-GlcNAc-Asn and/or α-L-fucoside accumulates in the central nervous system can be screened by the method of an embodiment of the invention, by measuring the concentration of Fuc-GlcNAc-Asn included in the cerebrospinal fluid and performing screening based on the measured values thus obtained. When the measured value is abnormally higher than the value of a normal person, the test subject can be determined to be a patient suffering from the disease. The method of the invention can be carried out for the purpose of conducting such determination.

When a patient with a disease in which Fuc-GlcNAc-Asn and/or α-L-fucoside accumulates in the central nervous system is treated, the effect of the treatment can be checked by measuring the concentration of Fuc-GlcNAc-Asn included in the cerebrospinal fluid of the patient before the treatment and after the treatment and measuring the amount of decrease of these after the treatment. As the amount of decrease is larger, the therapeutic effect can be considered to be higher. The method of the invention can be carried out for the purpose of conducting confirmation of such therapeutic effect. For

example, when the concentration of Fuc-GlcNAc-Asn included in the blood of the patient has been decreased preferably by 10% or more, for example, when the concentration has been decreased by 20% or more, 30% or more, or 50% or more, respectively, before and after the treatment, it can be determined that there has been therapeutic effect.

One of the diseases in which sulfatide accumulates in the body is metachromatic leukodystrophy. As a treatment method for metachromatic leukodystrophy, enzyme replacement therapy of replenishing arylsulfatase A (ARSA) in a patient by performing intravenous drip infusion is assumed; however, currently there is no clinically applied treatment method for that disease. In a case where enzyme replacement therapy has been carried out, regarding a method for evaluating the effect of the therapy, a method of quantitatively determining the concentration of sulfatide in muscle tissue is assumed. However, the method is not effective for abnormalities in the central nervous system (CNS) because enzymes cannot cross the blood-brain barrier (BBB). Therefore, regarding the purpose of evaluating the effect of conventional enzyme replacement therapy, the concentration of sulfatide included in the cerebrospinal fluid is not regarded as the target of measurement.

Metachromatic leukodystrophy is a disease that genetically partially or completely lacks arylsulfatase A (ARSA) activity. Due to deficit and deficiency of this enzyme, sulfatide accumulates in the body of a patient. Main symptoms of metachromatic leukodystrophy include central nerve system disorders. Clinically, metachromatic leukodystrophy is classified into three types, namely, infant type, juvenile type, and adult type, and central nervous system disorders are recognized in all of the types. Therefore, it is considered important to remove sulfatide accumulated in the central nervous system for metachromatic leukodystrophy. However, measuring sulfatide accumulated in the tissues of the central nervous system is not practical because it is necessary to perform biopsy of the cells of the central nervous system.

As shown in Example 38, the quantitative value of lyso-sulfatide included in cerebrospinal fluid, which is measured by the method of an embodiment of the invention, correlates with the concentration of lyso-sulfatide included in the brain tissue of the same individual. That is, according to the method of the invention, the amount of lyso-sulfatide accumulated in the central nervous system can be evaluated by measuring lyso-sulfatide included in the cerebrospinal fluid.

As described above, even for metachromatic leukodystrophy, it is considered important to remove sulfatide accumulated in the central nervous system. In order to cause a drug administered by intravenous injection or the like to reach the central nervous system, the drug needs to cross the blood-brain barrier. When it is attempted to develop a therapeutic drug for metachromatic leukodystrophy that can cross the blood-brain barrier and can exhibit effects in the central nervous system, there is a need to evaluate the drug efficacy in the central nervous system. The method of the invention meets such a demand. Even in the case of developing a drug of a type that is directly administered into the central nervous system without causing the drug to cross the blood-brain barrier, the same also applies.

Without being limited to metachromatic leukodystrophy, a patient suffering from a disease in which lyso-sulfatide and/or sulfatide accumulates in the central nervous system can be screened by the method of an embodiment of the invention, by measuring the concentration of lyso-sulfatide included in the cerebrospinal fluid and performing screening based on the measured values thus obtained. When the measured value is abnormally higher than the value of a normal person, the test subject can be determined to be a patient suffering from the disease. The method of the invention can be carried out for the purpose of conducting such determination.

When a patient with a disease in which lyso-sulfatide and/or sulfatide accumulates in the central nervous system is treated, the effect of the treatment can be checked by measuring the concentration of lyso-sulfatide included in the cerebrospinal fluid of the patient before the treatment and after the treatment and measuring the amount of decrease of these after the treatment. As the amount of decrease is larger, the therapeutic effect can be considered to be higher. The method of the invention can be carried out for the purpose of conducting confirmation of such therapeutic effect. For example, when the concentration of *lyso*-sulfatide included in the blood of the patient has been decreased preferably by 10% or more, for example, when the concentration has been decreased by 20% or more, 30% or more, or 50% or more, respectively, before and after the treatment, it can be determined that there has been therapeutic effect.

The method of an embodiment of the invention can also be used at the time of searching for a therapeutic agent for a disease in which Hex4 and/or glycogen accumulates in the central nervous system, using a model animal. When a drug is administered to a model animal with a disease in which Hex4 and/or glycogen accumulates in the central nervous system, the effect of the treatment can be checked by measuring the concentration of Hex4 included in the cerebrospinal fluid of the model animal before the administration and after the administration and measuring the amount of decrease thereof after the administration. It can be determined that as the amount of decrease thereof is larger, the therapeutic effect of the drug is higher. The method of the invention can be carried out for the purpose of conducting verification of such therapeutic effect. For example, when the concentration of Hex4 included in the blood of the model animal has been decreased preferably by 10% or more, for example, when the concentration has been decreased by 20% or more, 30% or more, or 50% or more, respectively, before and after the treatment, it can be determined that there has been therapeutic effect. Here, the animal species of the model animal is not particularly limited; however, examples thereof include monkey, mouse, rat, guinea pig, hamster, rabbit, horse, cattle, pig, dog, and cat, while preferred examples include monkey, mouse, and rat.

That is, the biological species from which the cerebrospinal fluid to be analyzed is acquired is not particularly

limited; however, examples include human being, monkey, mouse, rat, guinea pig, hamster, rabbit, horse, cattle, pig, dog, and cat, while particularly preferred examples include human being, monkey, mouse, and rat.

The method of an embodiment of the invention can also be used at the time of searching for a therapeutic agent for a disease in which *lyso-GM1* and/or monosialoganglioside GM1 accumulates in the central nervous system, using a model animal. When a drug is administered to a model animal with a disease in which *lyso-GM1* and/or monosialoganglioside GM1 accumulates in the central nervous system, the effect of the treatment can be checked by measuring the concentration of *lyso-GM1* included in the cerebrospinal fluid of the model animal before the administration and after the administration and measuring the amount of decrease thereof after the administration. It can be determined that as the amount of decrease thereof is larger, the therapeutic effect of the drug is higher. The method of the invention can be carried out for the purpose of conducting verification of such therapeutic effect. For example, when the concentration of *lyso-GM1* included in the blood of the model animal has been decreased preferably by 10% or more, for example, when the concentration has been decreased by 20% or more, 30% or more, or 50% or more, respectively, before and after the treatment, it can be determined that there has been therapeutic effect. Here, the animal species of the model animal is not particularly limited; however, examples thereof include monkey, mouse, rat, guinea pig, hamster, rabbit, horse, cattle, pig, dog, and cat, while preferred examples include monkey, mouse, and rat.

That is, the biological species from which the cerebrospinal fluid to be analyzed is acquired is not particularly limited; however, examples include human being, monkey, mouse, rat, guinea pig, hamster, rabbit, horse, cattle, pig, dog, and cat, while particularly preferred examples include human being, monkey, mouse, and rat.

The method of an embodiment of the invention can also be used at the time of searching for a therapeutic agent for a disease in which Fuc-GlcNAc-Asn and/or α-L-fucoside accumulates in the central nervous system, using a model animal. When a drug is administered to a model animal with a disease in which Fuc-GlcNAc-Asn and/or α-L-fucoside accumulates in the central nervous system, the effect of the treatment can be checked by measuring the concentration of Fuc-GlcNAc-Asn included in the cerebrospinal fluid of the model animal before the administration and after the administration and measuring the amount of decrease thereof after the administration. It can be determined that as the amount of decrease thereof is larger, the therapeutic effect of the drug is higher. The method of the invention can be carried out for the purpose of conducting verification of such therapeutic effect. For example, when the concentration of Fuc-GlcNAc-Asn included in the blood of the model animal has been decreased preferably by 10% or more, for example, when the concentration has been decreased by 20% or more, 30% or more, or 50% or more, respectively, before and after the treatment, it can be determined that there has been therapeutic effect. Here, the animal species of the model animal is not particularly limited; however, examples thereof include monkey, mouse, rat, guinea pig, hamster, rabbit, horse, cattle, pig, dog, and cat, while preferred examples include monkey, mouse, and rat.

That is, the biological species from which the cerebrospinal fluid to be analyzed is acquired is not particularly limited; however, examples include human being, monkey, mouse, rat, guinea pig, hamster, rabbit, horse, cattle, pig, dog, and cat, while particularly preferred examples include human being, monkey, mouse, and rat.

The method of an embodiment of the invention can also be used at the time of searching for a therapeutic agent for a disease in which lyso-sulfatide and/or sulfatide accumulates in the central nervous system, using a model animal. When a drug is administered to a model animal with a disease in which lyso-sulfatide and/or sulfatide accumulates in the central nervous system, the effect of the treatment can be checked by measuring the concentration of lyso-sulfatide included in the cerebrospinal fluid of the model animal before the administration and after the administration and measuring the amount of decrease thereof after the administration. It can be determined that as the amount of decrease thereof is larger, the therapeutic effect of the drug is higher. The method of the invention can be carried out for the purpose of conducting verification of such therapeutic effect. For example, when the concentration of lyso-sulfatide included in the blood of the model animal has been decreased preferably by 10% or more, for example, when the concentration has been decreased by 20% or more, 30% or more, or 50% or more, respectively, before and after the treatment, it can be determined that there has been therapeutic effect. Here, the animal species of the model animal is not particularly limited; however, examples thereof include monkey, mouse, rat, guinea pig, hamster, rabbit, horse, cattle, pig, dog, and cat, while preferred examples include monkey, mouse, and rat.

That is, the biological species from which the cerebrospinal fluid to be analyzed is acquired is not particularly limited; however, examples include human being, monkey, mouse, rat, guinea pig, hamster, rabbit, horse, cattle, pig, dog, and cat, while particularly preferred examples include human being, monkey, mouse, and rat.

[EXAMPLES]

Hereinafter, the present invention will be described in more detail with reference to Examples; however, the invention is not intended to be limited to the Examples.

In the following description, Examples 1 to 10 relate to the measurement of Hex4.

[Example 1] Preparation of reagent solution

Mobile phase A: 4 mL of 25% ammonium hydroxide solution (Merck & Co., Inc.) and 996 mL of water were mixed, and this mixture was used as mobile phase A.

Mobile phase B: 4 mL of 25% ammonium hydroxide solution (Merck & Co., Inc.) and 996 mL of acetonitrile (for use in LC/MS, Fujifilm Wako Pure Chemical Corp.) were mixed, and this mixture was used as mobile phase B.

[Example 2] Preparation of standard solution for Hex4 measurement

Standard stock solution for Hex4 measurement: 1 mg of 6-a-D-Glucopyranosyl maltotriose (Glc4, Carbosynth Holdings, Ltd.) was dissolved in 0.5 mL of pure water to prepare a 2 mg/mL solution, and this was used as a standard stock solution for Hex4 measurement.

Standard solutions for calibration curve for Hex4 measurement (H-SS-1 to H-SS-8): Serial dilutions of the standard stock solution for Hex4 measurement were prepared using methanol according to the following Table 1, and these were used as standard solutions for calibration curve for Hex4 measurement (H-SS-1 to H-SS-8).

[Table 1]

| Table 1. Preparation of standard solutions for calibration curve for Hex4 measurement | |
|---|---|
| Solution name | Preparation concentration (ng/mL) |
| H-SS-8 | 4800 |
| H-SS-7 | 2000 |
| H-SS-6 | 600 |
| H-SS-5 | 200 |
| H-SS-4 | 60 |
| H-SS-3 | 20 |
| H-SS-2 | 6 |
| H-SS-1 | 2 |

Standard solutions for QC for Hex4 measurement (H-QS-1 to H-QS-4): Serial dilutions of the standard solution for calibration curve for Hex4 measurement H-SS-8 were prepared using methanol according to the following Table 2, and these were used as standard solutions for QC for Hex4 measurement (H-QS-1 to H-QS-4).

[Table 2]

| Table 2. Preparation of standard solutions for QC for Hex4 measurement | |
|---|---|
| Solution name | Preparation concentration (ng/mL) |
| H-QS-4 | 1600 |
| H-QS-3 | 200 |
| H-QS-2 | 20 |
| H-QS-1 | 2 |

[Example 3] Preparation of internal standard solution for Hex4 measurement

Internal standard stock solution for Hex4 measurement: 1 mg of 6-a-D-Glucopyranosyl maltotriose-13C6 (Toronto Research Chemicals, Inc.) (H-IS) was dissolved in 0.5 mL of pure water to prepare a 2 mg/mL solution, and this was used as an internal standard stock solution for Hex4 measurement.

Internal standard solutions for Hex4 measurement (H-IS-1 and HIS-2): Serial dilutions of the internal standard stock solution for Hex4 measurement were prepared using methanol according to the following Table 3, and these were used as internal standard solutions for Hex4 measurement (H-IS-1 and H-IS-2).

[Table 3]

| Table 3. Preparation of internal standard solutions for Hex4 measurement | |
|---|---|
| Solution name | Preparation concentration (ng/mL) |
| H-IS-2 | 2000 |
| H-IS-1 | 200 |

[Example 4] Preparation of brain tissue extract and preparative isolation of CSF

The brains of wild-type mice (C57BL/6, 53 weeks old, Oriental Bio Service, Ltd.) and GAA KO mice (C57BL/6, hTfR KI, GAA KO, 50 weeks old, Oriental Bio Service, Ltd.), which are Pompe disease model mice, were extracted, and the wet weights thereof were weighed. The brain tissue, pure water in an amount 20 times the wet weight of the brain tissue, and fifteen Φ5-mm SUS beads (Taitec Corp.) were introduced into a 5-mL self-standing type mailing tube (Watson, Inc.), and the tissue was crushed (2500 rpm, for 120 seconds) with a bead crusher (μT-12, Taitec Corp.). The tissue liquid after crushing was transferred into a 1.5-mL sampling tube (Sarstedt K.K.), subjected to a heating treatment at 100°C for 5 minutes, and left to stand on ice. Next, centrifugation was performed for 5 minutes using a high-speed microcentrifuge (MX-307, Tomy Seiko Co., Ltd.) under the conditions of 13,000 rpm and 4°C, and a supernatant was collected and used as a brain tissue extract. Furthermore, about 15 μL each of cerebrospinal fluid (CSF) was preparatively isolated from each individual from which brain was collected.

[Example 5] Preparation of various samples

Standard samples for calibration curve for Hex4 measurement (H-S0 to H-S7): 100 μL each of the standard solutions for calibration curve for Hex4 measurement (H-SS-1 to H-SS-8) prepared in Example 2 and 100 μL of the internal standard solution for Hex4 measurement (H-IS-1) prepared in Example 3 were respectively mixed according to the following Table 4 and stirred, and then centrifugation was performed for 5 minutes using a high-speed microcentrifuge (MX-307, Tomy Seiko Co., Ltd.) under the conditions of 16,000 rpm and 20°C. Supernatants thus obtained were each filled into a vial and were used as standard samples for calibration curve for Hex4 measurement (H-S0 to H-S7).

[Table 4]

| Table 4. Preparation of standard samples for calibration curve for Hex4 measurement | | | | | | |
|---|---|---|---|---|---|---|
| Sample name | Standard solution for calibration curve for Hex4 measurement | | | Internal standard solution for Hex4 measurement | | |
| | Solution name | Amount of addition (μL) | Concentration in sample (ng/mL) | Solution name | Amount of addition (μL) | Concentration in sample (ng/mL) |
| H-S7 | H-SS-7 | 100 | 1000 | H-IS-1 | 100 | 100 |
| H-S6 | H-SS-6 | 100 | 300 | H-IS-1 | 100 | 100 |
| H-S5 | H-SS-5 | 100 | 100 | H-IS-1 | 100 | 100 |
| H-S4 | H-SS-4 | 100 | 30 | H-IS-1 | 100 | 100 |
| H-S3 | H-SS-3 | 100 | 10 | H-IS-1 | 100 | 100 |
| H-S2 | H-SS-2 | 100 | 3 | H-IS-1 | 100 | 100 |
| H-S1 | H-SS-1 | 100 | 1 | H-IS-1 | 100 | 100 |
| Zero sample (H-S0) | Methanol | 100 | 0 | Methanol | 100 | 0 |

Standard samples for QC for Hex4 measurement (H-Q1 to H-Q4): 100 μL each of the standard solutions for QC for Hex4 measurement prepared in Example 2 and 100 μL of the internal standard solution for Hex4 measurement (H-IS-1) prepared in Example 3 were respectively mixed according to the following Table 5 and stirred, and then centrifugation was performed for 5 minutes under the conditions of 16,000 rpm and 20°C. Supernatants thus obtained were each filled into a vial and were used as standard samples for QC for Hex4 measurement (H-Q1 to H-Q4).

[Table 5]

| Table 5. Preparation of standard samples for QC for Hex4 measurement | | | | | | |
|---|---|---|---|---|---|---|
| Sample name | Standard solution for calibration curve for Hex4 measurement | | | Internal standard solution for Hex4 measurement | | |
| | Solution name | Amount of addition (μL) | Concentration in sample (ng/mL) | Solution name | Amount of addition (μL) | Concentration in sample (ng/mL) |
| H-Q4 | H-QS-4 | 100 | 800 | H-IS-1 | 100 | 100 |
| H-Q3 | H-QS-3 | 100 | 100 | H-IS-1 | 100 | 100 |
| H-Q2 | H-QS-2 | 100 | 10 | H-IS-1 | 100 | 100 |
| H-Q1 | H-QS-1 | 100 | 1 | H-IS-1 | 100 | 100 |

Measurement sample (brain): 630 μL of methanol was added to 70 μL of the brain tissue extract obtained in Example 4, the mixture was stirred, and then centrifugation was performed for 5 minutes under the conditions of 16,000 rpm and 20°C to obtain a supernatant. 500 μL of the obtained supernatant was preparatively isolated, the solvent was distilled off using a centrifugal concentrator (CC-105, Tomy Seiko Co., Ltd.), and then 25 μL of the internal standard solution for Hex4 measurement H-IS-1 prepared in Example 3 and 25 μL of methanol were added thereto to redissolve the mixture. After stirring, centrifugation was performed for 5 minutes under the conditions of 16,000 rpm and 20°C, and a supernatant thus obtained was filled into a vial and used as a measurement sample.

Measurement sample (CSF): To 2 μL of the CSF obtained in Example 4, 10 μL of the internal standard solution for Hex4 measurement H-IS-1 prepared in Example 3 and 8 μL of methanol were added, and the mixture was stirred. Subsequently, centrifugation was performed for 5 minutes under the conditions of 16,000 rpm and 20°C, and a supernatant thus obtained was filled into a vial and was used as a measurement sample.

[Example 6] LC/MS/MS analysis

LC/MS/MS analysis was carried out using hydrophilic interaction ultrahigh performance liquid chromatography and a tandem quadrupole type mass analyzer. QTRAP5500 (AB Sciex Pte., Ltd.) was used as a mass analyzer (MS/MS apparatus), and Nexera X2 (SHIMADZU CORPORATION) was mounted as an HPLC apparatus on the mass analyzer. Furthermore, ACQUITY UPLC BEH Amide Column, 2.1 mm × 5 mm (Nihon Waters K.K.), was used as an analytic column, and ACQUITY UPLC BEH Amide VanGuard Pre-column, 2.1 mm × 50 mm (Nihon Waters K.K.), was used as a guide column. The mobile phase A and the mobile phase B prepared in Example 1 were used as mobile phases. Furthermore, the column temperature was set to 40°C.

The columns were equilibrated with a mixed liquid composed of 45% (v/v) of the mobile phase A and 55% (v/v) of the mobile phase B, subsequently 10 μL of a sample was injected in, and chromatography was performed under the conditions of the mobile phase shown in Table 6. Incidentally, the flow rate of the mobile phase was set to 0.4 mL/min.

[Table 6]

| Table 6. Conditions for liquid chromatography for Hex4 measurement | | |
|---|---|---|
| Time lapsed after sample injection (min) | Mobile phase A (% (v/v)) | Mobile phase B (% (v/v)) |
| 0.0 | 45 | 55 |
| 1.5 | Stop | |

The ion source parameters, MS internal parameters, and valve switching program of the MS/MS apparatus were respectively set as shown in Table 7 to Table 9, according to the instruction manual of QTRAP5500 (AB Sciex Pte., Ltd.).

[Table 7]

| Table 7. Ion source parameters for MS/MS apparatus for Hex4 measurement | |
|---|---|
| Ion Source | ESI |
| Polarity | Negative |

(continued)

| Table 7. Ion source parameters for MS/MS apparatus for Hex4 measurement | |
|---|---|
| Scan Type | MRM |
| Curtain Gas (CUR) | 30 |
| Collision Gas (CAD) | 8 |
| IonSpray Voltage (IS) | -4500 |
| Temperature (TEM) | 600 |
| Ion Source Gas 1 (GS1) | 30 |
| Ion Source Gas 2 (GS2) | 60 |

[Table 8]

| Table 8. MS internal parameters for MS/MS apparatus for Hex4 measurement | | | | | | | | | |
|---|---|---|---|---|---|---|---|---|---|
| | Detection ion | Theoretical value | Q1 Mass (Da) | Q3 Mass (Da) | Time (msec) | DP (volts) | EP (volts) | CE (volts) | CXP (volts) |
| Glc4 | $[M-H]^-$ | 665.2 | 665.1 | 179.0 | 500 | -210 | -10 | -34 | -13 |
| H-IS | $[M-H]^-$ | 671.2 | 671.1 | 185.0 | 500 | -210 | -10 | -34 | -13 |

[Table 9]

| Table 9. Valve switching program for MS/MS apparatus for Hex4 measurement | |
|---|---|
| Time (min) | Valve location |
| 0.0 to 0.1 | A |
| 0.1 to 1.4 | B |
| 1.4 to 1.5 | A |

The measured values (ng/mL) and trueness (%) were respectively set to three-digit significant numbers. Furthermore, the trueness (%) was calculated based on the following calculation formula. Trueness (%) = Measured value/theoretical concentration $\times$ 100 Incidentally, the theoretical concentration in the above-described calculation formula means the concentration of Hex4 added to a standard sample for calibration curve for Hex4 measurement or a standard sample for QC for Hex4 measurement.

[Example 7] Evaluation of calibration curve for Hex4 measurement and quantification range

The standard samples for calibration curve for Hex4 measurement and the standard samples for QC for Hex4 measurement prepared in Example 5 were measured, and the areas of the peaks (detection peaks) detected on the chromatographic charts of the product ions derived from Hex4 included in the standard samples for calibration curve for Hex4 measurement and the standard samples for QC for Hex4 measurement were determined. Furthermore, the area of the detection peak of the product ion derived from the internal standard solution was determined. The trueness for each preparation concentration (theoretical concentration) of the standard sample for calibration curve for Hex4 measurement is shown in Table 10, and the trueness for each preparation concentration (theoretical concentration) of the standard sample for QC for Hex4 measurement is shown in Table 11. In the range of 1 to 1000 ng/mL, the standard samples for calibration curve for Hex4 measurement could be measured at a trueness of 96.8% to 109%, and the standard samples for QC for Hex4 measurement could be measured at a trueness of 93.8% to 103%.

[Table 10]

| Table 10. Measurement results and trueness evaluation for standard samples for calibration curve for Hex4 measurement | | | |
|---|---|---|---|
| Sample name | Preparation concentration (ng/mL) | Calculated concentration (ng/mL) | Trueness (%) |
| H-S7 | 1000 | 1090 | 109 |
| H-S6 | 300 | 300 | 100 |
| H-S5 | 100 | 98.6 | 98.6 |
| H-S4 | 30 | 30.8 | 103 |
| H-S3 | 10 | 10.3 | 103 |
| H-S2 | 3 | 2.97 | 98.9 |
| H-S1 | 1 | 0.968 | 96.8 |

[Table 11]

| Table 11. Measurement results and trueness evaluation for standard samples for QC for Hex4 measurement | | | |
|---|---|---|---|
| Sample name | Preparation concentration (ng/mL) | Calculated concentration (ng/mL) | Trueness (%) |
| H-Q4 | 800 | 824 | 103 |
| H-Q3 | 100 | 93.8 | 93.8 |
| H-Q2 | 10 | 10.3 | 103 |
| H-Q1 | 1 | 0.959 | 95.9 |

Furthermore, the area ratio (Hex4 detection peak area/H-IS detection peak area) of the area (Hex4 detection peak area) of the detection peak originating from Hex4 included in each standard sample for calibration curve for Hex4 measurement with respect to the area (H-IS detection peak area) of the detection peak originating from the internal standard solution for Hex4 measurement was determined. This value was plotted on the ordinate axis, the concentration of Hex4 (Hex4 concentration) of each standard sample for calibration curve for Hex4 measurement was plotted on the abscissa axis, a regression equation was calculated using a quadratic programming method, and a calibration curve for Hex4 measurement was created. The obtained calibration curve for Hex4 measurement showed a satisfactory linearity in the range of 1 to 1000 ng/mL (Fig. 1). The correlation coefficient (r) was 1.0000.

[Example 8] Measurement of Hex4 concentration in brain and CSF

The Hex4 concentrations in the brain and CSF of each of wild-type mice and Pompe disease model mice were calculated using the calibration curve for Hex4 measurement obtained in Example 7 and the measurement samples prepared in Example 5. The measurement results are respectively shown in Fig. 2 and Fig. 3. In both the brain and the CSF, results that the concentration of Hex4 was higher in the Pompe disease model mice as compared to the wild-type mice were obtained. These results show that the concentrations of Hex4 in the brain and the CSF increase in the Pompe disease model mice as compared to the wild-type mice.

[Example 9] Measurement of glycogen concentration in brain

In order to evaluate the correlation between the measured values of the Hex4 concentration in the brain and the CSF obtained in Example 8 and the glycogen concentration in the brain, the glycogen concentration in the brain of each of wild-type mice and Pompe disease model mice was measured. The measurement of the glycogen concentration in the brain was carried out using a Glycogen Assay Kit (BioVision, Inc.). The Glycogen Assay Kit quantifies glycogen by breaking down glycogen included in a sample into glucose and quantifying this glucose.
The brain tissue extract and the CSF used for the measurement of the Hex4 concentration in the brain and the CSF, which had been prepared in Example 4, were used as samples for measuring the glycogen concentration. Incidentally, any sample for measuring the glycogen concentration, which was expected to exceed the calibration curve upper limit concentration, was appropriately diluted.
Preparation of standard solution for glycogen calibration curve: 10 μL of the standard solution in the Glycogen Assay Kit and 990 μL of water were mixed, and a 20 μg/mL glycogen solution was prepared. Next, the 20 μg/mL glycogen

solution was diluted according to the following Table 12 using the Hydrolysis Buffer in the Glycogen Assay Kit to prepare standard solutions for glycogen calibration curve (STD. 1 to STD. 7, Blank).

[Table 12]

| Table 12. Preparation of standard solutions for calibration curve for glycogen measurement | | | | |
|---|---|---|---|---|
| STD. No. | Amount of addition of STD. 0 | Amount of addition of Hydrolysis Buffer | Final concentration (ng/mL) | Amount of Glycogen (ng/well) |
| STD. 1 | 54 μL | 96 μL | 7200 | 360 |
| STD. 2 | 42 μL | 108 μL | 5600 | 280 |
| STD. 3 | 30 μL | 120 μL | 4000 | 200 |
| STD. 4 | 18 μL | 132 μL | 2400 | 120 |
| STD. 5 | 12 μL | 138 μL | 1600 | 80 |
| STD. 6 | 6.0 μL | 144 μL | 800 | 40 |
| STD. 7 | 3.0 μL | 147 μL | 400 | 20 |
| Blank | - | 150 μL | 0 | 0 |

Measurement of glycogen concentration in brain: To each well of an F96 Black plate (Thermo Fisher Scientific, Inc.), 50 μL each of the standard solutions for glycogen calibration curve and a sample for glycogen concentration measurement were added. The measurement was carried out by using two wells each for all standard samples for glycogen calibration curve and the tissue extracts for glycogen concentration measurement. 1 μL each of the Hydrolysis Enzyme Mix included in the Glycogen Assay Kit was added and mixed into two wells of the standard solutions for glycogen calibration curve and one well of the tissue extract for glycogen concentration measurement, and the mixtures were caused to react for 30 minutes at room temperature. In order to measure the concentration of endogenous glycose included in the samples, wells where the Hydrolysis Enzyme Mix was not added were prepared. The Development Buffer, Development Enzyme Mix, and OxiRed Probe in the Glycogen Assay Kit were mixed at the proportions of 48.7 : 1 : 0.3, and the mixture was used as a reaction solution. 50 μL each of the reaction solution was added to all the wells and was caused to react for 30 minutes at room temperature under light shielding. Fluorescence measurement was performed by using a fluorescence plate reader (Gemini XPS, Molecular Devices Japan K.K.) (Ex/Em = 535/587 nm).

Calculation of glycogen concentration in brain: A calibration curve was created from the theoretical concentrations of the standard solutions for glycogen calibration curve and the signal average values by means of a Linear fit curve, and each glucose concentration was calculated. A diluted sample was corrected by the dilution ratio thereof. The value obtained by subtracting the glucose concentration of a measurement sample, to which the Hydrolysis Enzyme Mix was not added (endogenous glucose concentration value), from the value of a measurement sample, to which the Hydrolysis Enzyme Mix was added (total glucose concentration value), was defined as the glycogen concentration.

Results: The results of measuring the glycogen concentration in the brain are shown in Fig. 4. It was shown that the glycogen concentration in the brain was the same as the Hex4 concentration in the brain (Fig. 2) and the Hex4 concentration in the CSF (Fig. 3) obtained in Example 8, and the value of the glycogen concentration in the brain increased in the Pompe disease model mice as compared to the wild-type mice.

[Example 10] Comparison of Hex4 concentration in brain and CSF with glycogen concentration in brain

The results of plotting the relationship between the glycogen concentration in the brain and the Hex4 concentration in the brain in the same mouse individuals, the relationship between the glycogen concentration in the brain and the Hex4 concentration in the CSF, and the relationship between the Hex4 concentration in the brain and the Hex4 concentration in the CSF are shown in Fig. 5 to Fig. 7, respectively. The coefficients of determination ($R^2$) were 0.9227, 0.9345, and 0.9467, respectively, and high correlativity was recognized in all cases.

These results show that the Hex4 concentration in the CSF reflects the Hex4 concentration and the glycogen concentration in the brain, and that the Hex4 concentration in the CSF can be used as a biomarker in the central nervous system for a disease in which Hex4 and/or glycogen accumulates in the brain. Particularly, the results show that the Hex4 concentration in the CSF can be used as a biomarker in the central nervous system for Pompe disease.

Hereinafter, Examples 11 to 20 relate to the measurement of *lyso*-GM1.

[Example 11] Preparation of reagent solution

Mobile phase C: 1 mL of formic acid (Fujifilm Wako Pure Chemical Corp.) and 499 mL of water were mixed, and this mixture was used as mobile phase C.
Mobile phase D: 1 mL of formic acid (Fujifilm Wako Pure Chemical Corp.) and 499 mL of acetonitrile (for use in LC/MS, Fujifilm Wako Pure Chemical Corp.) were mixed, and this mixture was used as mobile phase D.
60% Methanol solution: Methanol and water were mixed at the proportions of 60 : 40 (v/v), and this mixture was used as a 60% methanol solution.
65% Methanol solution: Methanol and water were mixed at the proportions of 65 : 35 (v/v), and this mixture was used as a 65% methanol solution.
90% Methanol solution: Methanol and water were mixed at the proportions of 90 : 10 (v/v), and this mixture was used as a 90% methanol solution.

[Example 12] Preparation of standard solution for *lyso*-GM1 measurement

Standard stock solution for *lyso*-GM1 measurement: 0.5 mg of *lyso*-Monosialoganglioside GM1 ($NH_4^+$ salt) (*lyso*-GM1, Matreya, LLC) was dissolved in 0.5 mL of methanol to prepare a 1 mg/mL solution, and this was used as a standard stock solution for *lyso*-GM1 measurement.
Standard solutions for calibration curve for *lyso*-GM1 (G-SS-1 to G-SS-8): Serial dilutions of the standard stock solution for *lyso*-GM1 measurement were prepared using methanol according to the following Table 13, and these were used as standard solutions for calibration curve for *lyso*-GM1 measurement (G-SS-1 to G-SS-8).

[Table 13]

| Table 13. Preparation of standard solutions for calibration curve for *lyso*-GM1 measurement | |
|---|---|
| Solution name | Preparation concentration (ng/mL) |
| G-SS-8 | 4800 |
| G-SS-7 | 2000 |
| G-SS-6 | 600 |
| G-SS-5 | 200 |
| G-SS-4 | 60 |
| G-SS-3 | 20 |
| G-SS-2 | 6 |
| G-SS-1 | 2 |

Standard solutions for QC for *lyso*-GM1 measurement (G-QS-1 to G-QS-4): Serial dilutions of the standard solution for calibration curve for *lyso*-GM1 measurement G-SS-8 were prepared using methanol according to the following Table 14, and these were used as standard solutions for QC for *lyso*-GM1 measurement (G-QS-1 to G-QS-4).

[Table 14]

| Table 14. Preparation of standard solutions for QC for *lyso*-GM1 measurement | |
|---|---|
| Solution name | Preparation concentration (ng/mL) |
| G-QS-4 | 1600 |
| G-QS-3 | 200 |
| G-QS-2 | 20 |
| G-QS-1 | 2 |

[Example 13] Preparation of internal standard solution for *lyso*-GM1 measurement

Internal standard stock solution for *lyso*-GM1 measurement: 1 mg of N-glycinated *lyso*-ceramide trihexoside (Matreya, LLC) (G-IS) was dissolved in 1 mL of pure water to prepare a 1 mg/mL solution, and this was used as an internal

standard stock solution for *lyso*-GM1 measurement.

Internal standard solutions for *lyso*-GM1 measurement (G-IS-1 to G-IS-3): Serial dilutions of the internal standard stock solution for *lyso*-GM1 measurement were prepared using methanol according to the following Table 15, and these were used as internal standard solutions for *lyso*-GM1 measurement (G-IS-1 to G-IS-3).

[Table 15]

Table 15. Preparation of internal standard solutions for *lyso*-GM1 measurement

| Solution name | Preparation concentration (ng/mL) |
|---|---|
| G-IS-3 | 2000 |
| G-IS-2 | 600 |
| G-IS-1 | 200 |

[Example 14] Preparation of tissue extract, blood plasma, and CSF

Various tissues (brain, spinal cord, lung, liver, spleen, kidney, heart, and quadriceps femoris muscle) of β-Gal KO homozygous mice (C57BL/6, 12 weeks old and 38 weeks old, Oriental Bio Service, Ltd.), β-Gal KO heterozygous mice (C57BL/6, 12 weeks old, Oriental Bio Service, Ltd.), and wild-type mice (C57BL/6, 12 weeks old and 38 weeks old, Oriental Bio Service, Ltd.) were extracted. The wet weight of each of the tissues was weighed, the tissue, pure water in an amount 9 times the wet weight of the tissue, and fifteen Φ5-mm SUS beads (Taitec Corp.) were introduced into a 5-mL self-standing type mailing tube (Watson, Inc.), and the tissue was crushed (2500 rpm, for 300 seconds) with a bead crusher (μT-12, Taitec Corp.). About 1 mL each of the tissue liquid after crushing was preparatively isolated and used as a tissue extract. For the brain and the spinal cord, a 10-fold dilution of the tissue liquid was also prepared using pure water, and the dilution was appropriately used for measurement. Furthermore, about 300 μL each of blood plasma was preparatively isolated from each individual. In addition, about 15 μL each of the cerebrospinal fluid (CSF) was preparatively isolated from each individual.

[Example 15] Preparation of various samples

Standard samples for calibration curve for *lyso*-GM1 measurement (G-S0 to G-S7): 150 μL each of the standard solutions for calibration curve for *lyso*-GM1 measurement (G-SS-1 to G-SS-7) prepared in Example 12 and 150 μL of the internal standard solution for *lyso*-GM1 measurement (G-IS-1) prepared in Example 13 were respectively mixed according to the following Table 16 and stirred, and then centrifugation was performed for 5 minutes using a high-speed microcentrifuge (MX-307, Tomy Seiko Co., Ltd.) under the conditions of 16,000×g and 20°C. Supernatants thus obtained were each filled into a vial and were used as standard samples for calibration curve (G-S0 to G-S7).

[Table 16]

Table 16. Preparation of standard samples for calibration curve for *lyso*-GM1 measurement

| Sample name | Standard solution for calibration curve for *lyso*-GM1 measurement | | | Internal standard solution for *lyso*-GM1 measurement | | |
|---|---|---|---|---|---|---|
| | Solution name | Amount of addition (μL) | Concentration in sample (ng/mL) | Solution name | Amount of addition (μL) | Concentration in sample (ng/mL) |
| G-S7 | G-SS-7 | 150 | 1000 | G-IS-1 | 150 | 100 |
| G-S6 | G-SS-6 | 150 | 300 | G-IS-1 | 150 | 100 |
| G-S5 | G-SS-5 | 150 | 100 | G-IS-1 | 150 | 100 |
| G-S4 | G-SS-4 | 150 | 30 | G-IS-1 | 150 | 100 |
| G-S3 | G-SS-3 | 150 | 10 | G-IS-1 | 150 | 100 |
| G-S2 | G-SS-2 | 150 | 3 | G-IS-1 | 150 | 100 |
| G-S1 | G-SS-1 | 150 | 1 | G-IS-1 | 150 | 100 |

(continued)

| Table 16. Preparation of standard samples for calibration curve for *lyso*-GM1 measurement | | | | | | |
|---|---|---|---|---|---|---|
| Sample name | Standard solution for calibration curve for *lyso*-GM1 measurement | | | Internal standard solution for *lyso*-GM1 measurement | | |
| | Solution name | Amount of addition (μL) | Concentration in sample (ng/mL) | Solution name | Amount of addition (μL) | Concentration in sample (ng/mL) |
| Zero sample (G-S0) | Methanol | 150 | 0 | Methanol | 150 | 0 |

Standard samples for QC for *lyso*-GM1 (G-Q1 to G-Q4): 150 μL each of the standard solutions for QC for *lyso*-GM1 measurement (G-QS-1 to G-QS-4) prepared in Example 12 and 150 μL of the internal standard solution for *lyso*-GM1 measurement (G-IS-1) prepared in Example 13 were respectively mixed according to the following Table 17 and stirred, and then centrifugation was performed for 5 minutes under the conditions of 16,000×g and 20°C. Supernatants thus obtained were each filled into a vial and were used as standard samples for QC (G-Q1 to G-Q4).

[Table 17]

| Table 17. Preparation of standard samples for QC for *lyso*-GM1 measurement | | | | | | |
|---|---|---|---|---|---|---|
| Sample name | Standard solution for calibration curve for *lyso*-GM1 measurement | | | Internal standard solution for *lyso*-GM1 measurement | | |
| | Solution name | Amount of addition (μL) | Concentration in sample (ng/mL) | Solution name | Amount of addition (μL) | Concentration in sample (ng/mL) |
| G-Q4 | G-QS-4 | 150 | 800 | G-IS-1 | 150 | 100 |
| G-Q3 | G-QS-3 | 150 | 100 | G-IS-1 | 150 | 100 |
| G-Q2 | G-QS-2 | 150 | 10 | G-IS-1 | 150 | 100 |
| G-Q1 | G-QS-1 | 150 | 1 | G-IS-1 | 150 | 100 |

Measurement samples (brain, spinal cord, lung, liver, kidney, quadriceps femoris muscle, and blood plasma): To 70 μL of each of the tissue extracts of brain, spinal cord, lung, liver, kidney, and quadriceps femoris muscle, and the blood plasma obtained in Example 14, 210 μL of pure water and 408 μL of methanol were added and stirred, subsequently centrifugation was performed for 5 minutes under the conditions of 16,000×g and 20°C, and a supernatant was obtained. 590 μL of the obtained supernatant was preparatively isolated, 10 μL of the internal standard solution for *lyso*-GM1 measurement G-IS-2 prepared in Example 13 was added, and 500 μL from 600 μL of the solution thus obtained was loaded into an Oasis HLB 1 cc Vac Cartridge (30 mg, 30 μm, Nihon Waters K.K.). The solution was washed with 1 mL of a 60% methanol solution and then was eluted with 1 mL of a 90% methanol solution. The solvent of 800 μL of the eluted fraction was distilled off using a centrifugal concentrator (CC-105, Tomy Seiko Co., Ltd.), subsequently 40 μL of methanol was added thereto, and the mixture was redissolved. After stirring, centrifugation was performed for 5 minutes under the conditions of 16,000×g and 20°C, and the obtained supernatant was filled into a vial and used as a measurement sample.

Measurement sample (heart): To 70 μL of a tissue extract of the heart obtained in Example 14, 210 μL of pure water and 408 μL of methanol were added, the mixture was stirred, subsequently centrifugation was performed for 5 minutes under the conditions of 16,000×g and 20°C, and a supernatant was obtained. 590 μL of the obtained supernatant was preparatively isolated, 10 μL of the internal standard solution for *lyso*-GM1 measurement G-IS-2 prepared in Example 13 was added thereto, and 500 μL from 600 μL of the obtained solution thus obtained was loaded into an Oasis HLB 1 cc Vac Cartridge (30 mg, 30 μm, Nihon Waters K.K.). The solution was washed with 1 mL of a 60% methanol solution and subsequently with 250 μL of a 65% methanol solution and was eluted with 1 mL of a 90% methanol solution. The solvent of 800 μL of the eluted fraction was distilled off using a centrifugal concentrator (CC-105, Tomy Seiko Co., Ltd.), subsequently 40 μL of methanol was added thereto, and the mixture was redissolved. After stirring, centrifugation was performed for 5 minutes under the conditions of 16,000×g and 20°C, and the obtained supernatant was filled into a vial and used as a measurement sample.

Measurement sample (spleen): To 70 μL of the tissue extract of spleen obtained in Example 14, 210 μL of pure water and 408 μL of methanol were added, the mixture was stirred, subsequently centrifugation was performed for 5

minutes under the conditions of 16,000×g and 20°C, and a supernatant was obtained. 590 μL of the obtained supernatant was preparatively isolated, 10 μL of the internal standard solution for *lyso*-GM1 measurement G-IS-2 prepared in Example 13 was added thereto, and 500 μL from 600 μL of the obtained solution thus obtained was loaded into an Oasis HLB 1 cc Vac Cartridge (30 mg, 30 μm, Nihon Waters K.K.). The solution was washed with 1 mL of a 60% methanol solution and then was eluted with 400 μL of a 90% methanol solution. The solvent of 320 μL of the eluted fraction was distilled off using a centrifugal concentrator (CC-105, Tomy Seiko Co., Ltd.), subsequently 40 μL of methanol was added thereto, and the mixture was redissolved. After stirring, centrifugation was performed for 5 minutes under the conditions of 16,000×g and 20°C, a supernatant thus obtained was filled into a vial, and this was used as a measurement sample.

Measurement sample (CSF): To 2 μL of the CSF obtained in Example 14, 10 μL of the internal standard solution for *lyso*-GM1 measurement G-IS-1 prepared in Example 13 and 8 μL of methanol were added, the mixture was stirred, subsequently centrifugation was performed for 5 minutes under the conditions of 16,000×g and 20°C, and a supernatant thus obtained was filled into a vial and was used as a measurement sample.

[Example 16] LC/MS/MS analysis

LC/MS/MS analysis was carried out using a combination of reverse phase chromatography and a tandem quadrupole type mass analyzer. QTRAP5500 or TripleQuad6500+ (AB Sciex Pte., Ltd.) was used as a mass analyzer (MS/MS apparatus), and Nexera X2 (SHIMADZU CORPORATION) was mounted as an HPLC apparatus on the mass analyzer. Furthermore, Cadenza CW-C18 2 mm × 150 mm (Imtakt Corp.) was used as an analytic column, and CW-C18 5 × 2 mm (Imtakt Corp.) was used as a guide column. The mobile phase C and the mobile phase D prepared in Example 11 were used as mobile phases. Furthermore, the column temperature was set to 40°C.

The columns were equilibrated with a mixed liquid composed of 62.5% (v/v) of the mobile phase C and 37.5% (v/v) of the mobile phase D, subsequently 10 μL of a sample was injected in, and chromatography was performed under the conditions of the mobile phase shown in Table 18. Incidentally, the flow rate of the mobile phase was set to 0.4 mL/min.

[Table 18]

| Table 18. Conditions for liquid chromatography for *lyso*-GM1 measurement | | |
|---|---|---|
| Time lapsed after sample injection (min) | Mobile phase C (% (V/V)) | Mobile phase D (% (V/V)) |
| 0.0 | 62.5 | 37.5 |
| 1.0 | 62.5 | 37.5 |
| 2.0 | 5 | 95 |
| 3.4 | 5 | 95 |
| 3.5 | 62.5 | 37.5 |
| 5.5 | Stop | |

The ion source parameters, MS internal parameters, and valve switching program of the MS/MS apparatus were respectively set as shown in Table 19 to Table 21, according to the instruction manual of QTRAP5500 or TripleQuad6500+ (AB Sciex Pte., Ltd.).

[Table 19]

| Table 19. Ion source parameters for MS/MS apparatus for *lyso*-GM1 measurement | |
|---|---|
| Ion Source | ESI |
| Polarity | Positive |
| Scan Type | MRM |
| Curtain Gas (CUR) | 40 |
| Collision Gas (CAD) | 12 |
| IonSpray Voltage (IS) | 5500 |
| Temperature (TEM) | 300 |
| Ion Source Gas 1 (GS1) | 80 |

(continued)

| Table 19. Ion source parameters for MS/MS apparatus for *lyso*-GM1 measurement | |
|---|---|
| Ion Source Gas 2 (GS2) | 80 |

[Table 20]

Table 20. MS internal parameters for MS/MS apparatus for *lyso*-GM1 measurement

| | Detection ion | Theoretical value | Q1 Mass (Da) | Q3 Mass (Da) | Time (msec) | DP (volts) | EP (volts) | CE (volts) | CXP (volts) |
|---|---|---|---|---|---|---|---|---|---|
| *lyso*-GM1 | $[M+2H]^{2+}$ | 640.8 | 641.0 | 282.3 | 500 | 81 | 10 | 45 | 16 |
| G-IS | $[M+H]^{+}$ | 843.5 | 843.3 | 264.2 | 500 | 186 | 10 | 71 | 8 |

[Table 21]

Table 21. Valve switching program for MS/MS apparatus for *lyso*-GM1 measurement

| Time (min) | Valve location |
|---|---|
| 0.0 to 0.1 | A |
| 0.1 to 5.4 | B |
| 5.4 to 5.5 | A |

The measured values (ng/mL) and trueness (%) were respectively set to three-digit significant numbers. Furthermore, the trueness (%) was calculated based on the following calculation formula. Trueness (%) = Measured value/theoretical concentration × 100 Incidentally, the theoretical concentration in the above-described calculation formula means the concentration of *lyso*-GM1 added to a standard sample for calibration curve for *lyso*-GM1 measurement or a standard sample for QC for *lyso*-GM1 measurement.

[Example 17] Evaluation of calibration curve for *lyso*-GM1 measurement and quantification range

The standard samples for calibration curve for *lyso*-GM1 measurement and the standard samples for QC for *lyso*-GM1 measurement prepared in Example 15 were measured, and the areas of the peaks (detection peaks) detected on the chromatographic charts of the product ions derived from *lyso*-GM1 included in the standard samples for calibration curve for *lyso*-GM1 measurement and the standard samples for QC for *lyso*-GM1 measurement were determined. Furthermore, the area of the detection peak of the product ion derived from the internal standard solution for *lyso*-GM1 measurement was determined. The trueness for each preparation concentration (theoretical concentration) of the standard sample for calibration curve for *lyso*-GM1 measurement is shown in Table 22, and the trueness for each preparation concentration (theoretical concentration) of the standard sample for QC for *lyso*-GM1 measurement is shown in Table 23. In the range of 1 to 1000 ng/mL, the standard samples for calibration curve for *lyso*-GM1 measurement could be measured at a trueness of 91.1% to 105%, and the standard samples for QC for *lyso*-GM1 measurement could be measured at a trueness of 98.2% to 107%.

[Table 22]

Table 22. Measurement results and trueness evaluation for standard samples for calibration curve for *lyso*-GM1 measurement

| Sample name | Preparation concentration (ng/mL) | Calculated concentration (ng/mL) | Trueness (%) |
|---|---|---|---|
| G-S7 | 1000 | 1000 | 100 |
| G-S6 | 300 | 300 | 100 |
| G-S5 | 100 | 98.7 | 98.7 |
| G-S4 | 30 | 30.9 | 103 |
| G-S3 | 10 | 10.2 | 102 |

(continued)

| Table 22. Measurement results and trueness evaluation for standard samples for calibration curve for *lyso*-GM1 measurement | | | |
|---|---|---|---|
| Sample name | Preparation concentration (ng/mL) | Calculated concentration (ng/mL) | Trueness (%) |
| G-S2 | 3 | 3.15 | 105 |
| G-S1 | 1 | 0.911 | 91.1 |

[Table 23]

| Table 23. Measurement results and trueness evaluation for standard samples for QC for *lyso*-GM1 measurement | | | |
|---|---|---|---|
| Sample name | Preparation concentration (ng/mL) | Calculated concentration (ng/mL) | Trueness (%) |
| G-Q4 | 800 | 823 | 103 |
| G-Q3 | 100 | 107 | 107 |
| G-Q2 | 10 | 10.5 | 105 |
| G-Q1 | 1 | 0.982 | 98.2 |

Furthermore, the area ratio (*lyso*-GM1 detection peak area/G-IS detection peak area) of the area (*lyso*-GM1 detection peak area) of the detection peak originating from *lyso*-GM1 included in each standard sample for calibration curve for *lyso*-GM1 measurement with respect to the area (G-IS detection peak area) of the detection peak originating from the internal standard solution for *lyso*-GM1 measurement was determined. This value was plotted on the ordinate axis, the concentration of *lyso*-GM1 (*lyso*-GM1 concentration) of each standard sample for calibration curve for *lyso*-GM1 measurement was plotted on the abscissa axis, a regression equation was calculated using a quadratic programming method, and a calibration curve for *lyso*-GM1 measurement was created. The obtained calibration curve for *lyso*-GM1 measurement showed a satisfactory linearity in the range of 1 to 1000 ng/mL (Fig. 8). The correlation coefficient (r) was 0.9999.

[Example 18] Measurement of *lyso*-GM1 concentration in various tissues, CSF, and blood plasma of wild-type mouse and model mouse

The *lyso*-GM1 concentrations in various tissues, CSF, and blood plasma of each of wild-type mice, β-Gal KO homozygous mice, and β-Gal KO heterozygous mice were calculated using the calibration curve for *lyso*-GM1 measurement obtained in Example 17 and the measurement samples prepared in Example 15. The measurement results are respectively shown in Fig. 9 to Fig. 18. In all of the tissues, blood plasma, and CSF, results that the concentration of *lyso*-GM1 was higher in the β-Gal KO homozygous mice as compared to the wild-type mice were obtained. Furthermore, the concentration of *lyso*-GM1 was lower in the β-Gal KO heterozygous mice, and the concentration of *lyso*-GM1 was similar in the wild-type mice. These results show that the concentrations of *lyso*-GM1 in the various tissues, blood plasma, and CSF increase in the β-Gal KO homozygous mice as compared to the wild-type mice. Incidentally, in the brain, CSF, and spinal cord, that is, the central nervous system, of the β-Gal KO homozygous mice, an age-dependent increase in the concentration of *lyso*-GM1 can be confirmed.

[Example 19] Measurement of GM1 concentration in brain tissue of wild-type mouse and model mouse

In order to evaluate the correlation between the measured values of the *lyso*-GM1 concentration in the brain and the CSF obtained in Example 18 and the GM1 concentration in the brain tissue, the GM1 concentration in the brain tissue of each of wild-type mice, β-Gal KO homozygous mice, and β-Gal KO heterozygous mice was measured.

The measurement sample (brain) prepared in Example 15, which was used for the measurement of the *lyso*-GM1 concentration in the brain and the CSF, was used as a GM1 concentration measurement sample in the brain tissue, and the following measurement was carried out.

[Preparation of reagent solution for GM1 measurement]

0.5 M ammonium acetate solution: Water was added to 19.27 g of ammonium acetate (Fujifilm Wako Pure Chemical Corp.), the total amount was made up to 500 mL, and this was used as a 0.5 M ammonium acetate solution.

25 mM ammonium acetate solution: 25 mL of the 0.5 M ammonium acetate solution and 475 mL of water were

mixed, and the mixture was used as a 25 mM ammonium acetate solution.

50 mM ammonium acetate solution: 1 mL of the 0.5 M ammonium acetate solution and 9 mL of water were mixed, and the mixture was used as a 50 mM ammonium acetate solution.

Mobile phase C': 25 mL of the 0.5 M ammonium acetate solution and 400 mL of acetonitrile (Fujifilm Wako Pure Chemical Corp.) were mixed, subsequently water was added to make up a total amount of 500 mL, and this was used as mobile phase C'.

Mobile phase D': 25 mL of the 0.5 M ammonium acetate solution and 350 mL of acetonitrile (Fujifilm Wako Pure Chemical Corp.) were mixed, subsequently water was added to make up a total amount of 500 mL, and this was used as mobile phase D'.

[Preparation of standard solutions for GM1 measurement]

Standard stock solution for GM1 measurement: 5 mg of Monosialoganglioside GM1 ($NH_4^+$ salt) (GM1, Matreya, LLC) was dissolved in 1 mL of methanol to prepare a 5 mg/mL solution, and this was used as a standard stock solution for GM1 measurement.

Standard solutions for calibration curve for GM1 measurement (GM1-SS-1 to GM1-SS-8): Serial dilutions of the standard stock solution for GM1 measurement were prepared using dimethyl sulfoxide (DMSO) according to the following Table 24, and these were used as standard solutions for calibration curve for GM1 measurement (GM1-SS-1 to GM1-SS-8).

[Table 24]

Table 24. Preparation of standard solutions for calibration curve for GM1 measurement

| Solution name | Preparation concentration (ng/mL) |
|---|---|
| GM1-SS-8 | 20000 |
| GM1-SS-7 | 10000 |
| GM1-SS-6 | 2000 |
| GM1-SS-5 | 1000 |
| GM1-SS-4 | 200 |
| GM1-SS-3 | 100 |
| GM1-SS-2 | 20 |
| GM1-SS-1 | 10 |

[Preparation of internal standard solution for GM1 measurement]

Internal standard stock solution for GM1 measurement: 0.5 mg of N-omega-CD3-Octadecanoyl monosialoganglioside GM1 ($NH_4^+$ salt) (Matreya, LLC) (hereinafter, referred to as GM1-IS) was dissolved in 0.5 mL of methanol to prepare a 1 mg/mL solution, and this was used as an internal standard stock solution for GM1 measurement.

Internal standard solutions for GM1 measurement (GM1-IS-1 and GM1-IS-2): Internal standard solutions for GM1 measurement (GM1-IS-1 and GM1-IS-2) were prepared by preparing serial dilutions of the internal standard stock solution for GM1 measurement using DMSO according to the following Table 25.

[Table 25]

Table 25. Preparation of internal standard solutions for GM1 measurement

| Solution name | Preparation concentration (ng/mL) |
|---|---|
| GM1-IS-2 | 5000 |
| GM1-IS-1 | 1000 |

[Preparation of various samples]

Standard samples for calibration curve for GM1 measurement (GM1-S0 to GM1-G8): 50 μL of each of the standard solutions for calibration curve for GM1 measurement (GM1-SS-1 to GM1-SS-8), 50 μL of the internal standard

solution for GM1 measurement (GM1-IS-1), 100 μL of the 25 mM ammonium acetate solution, and 800 μL of acetonitrile were respectively mixed according to the following Table 26, and standard samples for calibration curve for GM1 measurement (GM1-S0 to GM1-S8) were obtained.

[Table 26]

Table 26. Preparation of standard samples for calibration curve for GM1 measurement

| Sample name | Standard solution for calibration curve for GM1 measurement | | | Internal standard solution for GM1 measurement | | | 25 mM ammonium acetate solution | Acetonitrile |
|---|---|---|---|---|---|---|---|---|
| | Solution name | Amount of addition (μL) | Concentration in sample (ng/mL) | Solution name | Amount of addition (μL) | Concentration in sample (ng/mL) | Amount of addition (μL) | Amount of addition (μL) |
| GM1-S8 | GM1-SS-8 | 50 | 10000 | GM1-IS-1 | 50 | 50 | 100 | 800 |
| GM1-S7 | GM1-SS-7 | 50 | 5000 | GM1-IS-1 | 50 | 50 | 100 | 800 |
| GM1-S6 | GM1-SS-6 | 50 | 1000 | GM1-IS-1 | 50 | 50 | 100 | 800 |
| GM1-S5 | GM1-SS-5 | 50 | 500 | GM1-IS-1 | 50 | 50 | 100 | 800 |
| GM1-S4 | GM1-SS-4 | 50 | 100 | GM1-IS-1 | 50 | 50 | 100 | 800 |
| GM1-S3 | GM1-SS-3 | 50 | 50 | GM1-IS-1 | 50 | 50 | 100 | 800 |
| GM1-S2 | GM1-SS-2 | 50 | 10 | GM1-IS-1 | 50 | 50 | 100 | 800 |
| GM1-S1 | GM1-SS-1 | 50 | 5 | GM1-IS-1 | 50 | 50 | 100 | 800 |
| Zero sample (GM1-S0) | DMSO | 50 | 0 | DMSO | 50 | 0 | 100 | 800 |

[LC/MS/MS analysis]

LC/MS/MS analysis was carried out using a combination of reverse phase chromatography and a tandem quadrupole type mass analyzer. QTRAP5500 (AB Sciex Pte., Ltd.) was used as a mass analyzer (MS/MS apparatus), and Nexera LC-30A (SHIMADZU CORPORATION) was mounted as an HPLC apparatus on the mass analyzer. Furthermore, UK amino column 2.1 mm × 150 mm (Imtakt Corp.) was used as a primary column, and Kinetex HILIC 2.1 × 150 mm (Phenomenex, Inc.) was used as a secondary column. The mobile phase C' and the mobile phase D' prepared as described above were used as mobile phases. Furthermore, the column temperature was set to 60°C.

The primary column and the secondary column were equilibrated with the mobile phase C' and the mobile phase D', respectively, 10 μL of a sample was injected, and chromatography was performed under the conditions for the mobile phase as shown in Table 27. Incidentally, the flow rate of the mobile phase C' was set to 0.2 mL/min, and the flow rate of the mobile phase D' was set to 0.25 mL/min.

[Table 27]

| Table 27. Conditions for liquid chromatography for GM1 measurement | | |
|---|---|---|
| Time lapsed after sample injection (min) | Primary column | Secondary column |
| 0.0 to 4.0 | Equilibration with mobile phase C' | Equilibration with mobile phase D' |
| 4.0 to 5.5 | Sample adsorption with mobile phase A' | |
| 5.5 to 12.5 | Washing with mobile phase C' | Elution with mobile phase D' |
| 12.5 to 16.0 | Equilibration with mobile phase C' | Equilibration with mobile phase D' |

MS/MS settings, GM1 detection ion species, various GM1 ion species detection conditions, and ESI parameters were set as shown in Table 28 to Table 31, respectively, according to the instruction manual of QTRAP5500 (AB Sciex Pte., Ltd.). For the detection ions, peaks on the ion chromatogram detected for each Isoform (GM1_d18:1_C18:0, GM1_d20:1_C18:0, GM1_d18:1_C16:0, GM1_d16:1_C18:0) were integrated, and then the resultant was used as one GM1 peak for the analysis.

[Table 28]

| Table 28. MS/MS settings for GM1 measurement | |
|---|---|
| Scan Type | MRM |
| Polarity | Positive |
| Ion Source | Turbo Spray |
| Resolution Q1 | High |
| Resolution Q3 | High |

[Table 29]

| Table 29. GM1 detection ion species | | | | |
|---|---|---|---|---|
| | Isoform | $[M+2H]^{2+}$ | Q1 Mass (Da) | Q3 Mass (Da) |
| GM1 | GM1_d18:1_C18:0 | m/z: 773.9 | 774.2 | 264.3 |
| | GM1_d20:1_C18:0 | m/z: 788.0 | 788.2 | 292.3 |
| | GM1_d18:1_C16:0 | m/z: 759.9 | 759.9 | 264.3 |
| | GM1_d16:1_C18:0 | m/z: 759.9 | 759.9 | 236.0 |
| GM1-IS | GM1_d18:1_C18:0_CD3 | m/z: 775.4 | 775.7 | 551.6 |

[Table 30]

| Table 30. Detection conditions for each GM1 ion species | | | | | |
|---|---|---|---|---|---|
| | Isoform | Parameter | | | |
| | | DP | CE | CXP | EP |
| GM1 | GM1_d18:1_C18:0 | 91.00 | 47.00 | 24.00 | 10.00 |
| | GM1_d20:1_C18:0 | 101.00 | 51.00 | 16.00 | 10.00 |
| | GM1_d18:1_C16:0 | 96.00 | 51.00 | 8.00 | 10.00 |
| | GM1_d16:1_C18:0 | 151.00 | 53.00 | 18.00 | 10.00 |
| GM1-IS | GM1_d18:1_C18:0_CD3 | 106.00 | 31.00 | 12.00 | 10.00 |

[Table 31]

| Table 31. ESI parameters for GM1 measurement | |
|---|---|
| CUR | 50.0 psi |
| IS | 5500 V |
| Temp | 600°C |
| GS1 | 70 psi |
| GS2 | 70 psi |
| CAD | 8 psi |

The measured values (ng/mL) were set to three-digit significant numbers.

The area ratio (GM1 detection peak area/GM1-IS detection peak area) of the area (GM1 detection peak area) of the detection peak originating from GM1 included in each standard sample for calibration curve for GM1 measurement with respect to the detection peak (GM1-IS detection peak area) originating from the GM1 internal standard solution was determined. This value was plotted on the ordinate axis, the concentration of GM1 (GM1 concentration) of each standard sample for calibration curve for GM1 measurement was plotted on the abscissa axis, a regression equation was calculated using a quadratic programming method, and a calibration curve for GM1 measurement was created.

[Measurement of GM1 concentration in brain tissue of wild-type mouse and model mouse]

The GM1 concentration in the brain tissue of each of wild-type mice, β-Gal KO homozygous mice, and β-Gal KO heterozygous mice was calculated using the calibration curve for GM1 measurement obtained as described above and the measurement samples (brain) prepared in Example 15. The measurement results are shown in Fig. 19. Results in which the concentration of GM1 was higher in the β-Gal KO homozygous mice as compared to the wild-type mice were obtained. Furthermore, the concentration of GM1 was lower in the β-Gal KO heterozygous mice, and the concentration was similar in the wild-type mice. These results show that the concentration of GM1 in the brain tissue increases in the β-Gal KO homozygous mice as compared to the wild-type mice. Incidentally, an age-dependent increase in the concentration of GM1 can be confirmed in the β-Gal KO homozygous mice.

[Example 20] Comparison of *lyso*-GM1 concentration in brain and CSF and GM1 concentration in brain

The results of plotting the relationship between the *lyso*-GM1 concentration in the brain and the *lyso*-GM1 concentration, the relationship between the GM1 concentration in the brain and the *lyso*-GM1 concentration in the CSF, and the relationship between the *lyso*-GM1 concentration in the brain and the GM1 concentration in the brain in the CSF in the same mouse individuals are shown in Fig. 20 to Fig. 22, respectively. The coefficients of determination ($R^2$) were 0.0.9909, 0.9051, and 0.9375, respectively, and high correlativity was recognized in all cases.

These results show that the *lyso*-GM1 concentration in the CSF reflects the *lyso*-GM1 concentration and the GM1 concentration in the brain, and that the *lyso*-GM1 concentration in the CSF can be used as a biomarker in the central nervous system for a disease in which *lyso*-GM1 and/or GM1 accumulates in the brain. Particularly, the results show that the *lyso*-GM1 concentration in the CSF can be used as a biomarker in the central nervous system for GM1 gangliosidosis.

Hereinafter, Examples 21 to 29 relate to the measurement of Fuc-GlcNAc-Asn.

[Example 21] Preparation of reagent solution

Mobile phase E: 2 mL of formic acid (Fujifilm Wako Pure Chemical Corp.) and 998 mL of water were mixed, and this mixture was used as mobile phase E.

Mobile phase F: 2 mL of formic acid (Fujifilm Wako Pure Chemical Corp.) and 998 mL of acetonitrile (for LC/MS, Fujifilm Wako Pure Chemical Corp.) were mixed, and this mixture was used as mobile phase F.

[Example 22] Preparation of standard solution for Fuc-GlcNAc-Asn measurement

Standard stock solution for Fuc-GlcNAc-Asn measurement: 3.3 mg of Fuc1-α-6GlcNAc1-β-Asn (Fuc-GlcNAc-Asn, Peptide Institute, Inc.) was dissolved in 16.5 mL of pure water to prepare a 0.2 mg/mL solution, and this was used as a standard stock solution for Fuc-GlcNAc-Asn measurement.

Standard solutions for calibration curve for Fuc-GlcNAc-Asn measurement (F-SS-1 to F-SS-7): Serial dilutions of

the standard stock solution for Fuc-GlcNAc-Asn measurement were prepared using pure water according to the following Table 32, and these were used as standard solutions for Fuc-GlcNAc-Asn measurement (F-SS-1 to F-SS-7).

[Table 32]

| Table 32. Preparation of standard solutions for calibration curve for Fuc-GlcNAc-Asn measurement | |
|---|---|
| Solution name | Preparation concentration (ng/mL) |
| F-SS-7 | 1500 |
| F-SS-6 | 500 |
| F-SS-5 | 150 |
| F-SS-4 | 50 |
| F-SS-3 | 15 |
| F-SS-2 | 5 |
| F-SS-1 | 1.5 |

Standard solutions for QC for Fuc-GlcNAc-Asn measurement (F-QS-1 to F-QS-4): Serial dilutions of the standard solution for calibration curve for Fuc-GlcNAc-Asn measurement F-SS-7 were prepared using pure water according to the following Table 33, and these were used as standard solutions for QC for Fuc-GlcNAc-Asn measurement (F-QS-1 to F-QS-4).

[Table 33]

| Table 33. Preparation of standard solutions for QC for Fuc-GlcNAc-Asn measurement | |
|---|---|
| Solution name | Preparation concentration (ng/mL) |
| F-QS-4 | 1200 |
| F-QS-3 | 50 |
| F-QS-2 | 1.5 |
| F-QS-1 | 0.5 |

[Example 23] Preparation of internal standard solution for Fuc-GlcNAc-Asn measurement

Internal standard stock solution for Fuc-GlcNAc-Asn measurement: 2 mg of Fuc1-α-3GlcNAc1-β-OMe (Fuc-GlcNAc-OMe, Toronto Research Chemicals, Inc.) was dissolved in 1 mL of methanol to prepare a 2 mg/mL solution, and this was used as an internal standard stock solution for Fuc-GlcNAc-Asn measurement.

Internal standard solutions for Fuc-GlcNAc-Asn measurement (F-IS-1 to F-IS-3): Serial dilutions of the internal standard stock solution for Fuc-GlcNAc-Asn measurement were prepared using acetonitrile according to the following Table 34, and these were used as internal standard solutions for Fuc-GlcNAc-Asn measurement (F-IS-1 to F-IS-3).

[Table 34]

| Table 34. Preparation of internal standard solutions for Fuc-GlcNAc-Asn measurement | |
|---|---|
| Solution name | Preparation concentration (ng/mL) |
| F-IS-2 | 4000 |
| F-IS-1 | 12.5 |

[Example 24] Preparation of tissue extracts and preparative isolation of blood plasma, CSF, and urine

Various tissues (brain, liver, kidney, and spleen) of wild-type mice (C57BL/6, 13 to 15 weeks old, Oriental Bio Service, Ltd.) and FUCA1 KO mice (C57BL/6, 13 to 15 weeks old, Oriental Bio Service, Ltd.), which are fucosidosis model mice, were extracted. Each of the tissues was freeze-dried and then weighed, the tissue, pure water in an amount 39 times the freeze-dried weight of the tissue, and fifteen Φ5-mm SUS beads (Taitec Corp.) were introduced into a 5-mL self-standing type mailing tube (Watson, Inc.), and the tissue was crushed (2500 rpm, for 120 seconds) with a bead crusher (μT-12, Taitec Corp.). About 1 mL each of the tissue liquid after crushing was preparatively isolated and was used as a tissue extract. Furthermore, about 300 μL each of blood plasma, about 15 μL each of cerebrospinal fluid (CSF), and about 1 mL each of urine were preparatively isolated from each individual.

[Example 25] Preparation of various samples

Standard samples for calibration curve for Fuc-GlcNAc-Asn measurement (F-S0 to F-S7): 20 μL of each of the standard solutions for calibration curve for Fuc-GlcNAc-Asn measurement (F-SS-1 to F-SS-7) prepared in Example 22 and 80 μL of the internal standard solution for Fuc-GlcNAc-Asn measurement (F-IS-1) prepared in Example 23 were respectively mixed according to the following Table 35 and stirred, and then centrifugation was performed for 5 minutes using a high-speed microcentrifuge (MX-307, Tomy Seiko Co., Ltd.) under the conditions of 16,000 rpm and 20°C. Supernatants thus obtained were each filled into a vial and were used as standard samples for calibration curve for Fuc-GlcNAc-Asn measurement (F-S0 to F-S7).

[Table 35]

| Table 35. Preparation of standard samples for calibration curve for Fuc-GlcNAc-Asn measurement | | | | | | |
|---|---|---|---|---|---|---|
| Sample name | Standard solution for calibration curve for Fuc-GlcNAc-Asn measurement | | | Internal standard solution for Fuc-GlcNAc-Asn measurement | | |
| | Solution name | Amount of addition (μL) | Concentration in sample (ng/mL) | Solution name | Amount of addition (μL) | Concentration in sample (ng/mL) |
| F-S7 | SS-7 | 20 | 300 | F-IS-1 | 80 | 10 |
| F-S6 | SS-6 | 20 | 100 | F-IS-1 | 80 | 10 |
| F-S5 | SS-5 | 20 | 30 | F-IS-1 | 80 | 10 |
| F-S4 | SS-4 | 20 | 10 | F-IS-1 | 80 | 10 |
| F-S3 | SS-3 | 20 | 3 | F-IS-1 | 80 | 10 |
| F-S2 | SS-2 | 20 | 1 | F-IS-1 | 80 | 10 |
| F-S1 | SS-1 | 20 | 0.3 | F-IS-1 | 80 | 10 |
| Zero sample (F-S0) | Pure water | 20 | 0 | Acetonitrile | 80 | 0 |

Standard samples for QC for Fuc-GlcNAc-Asn measurement (F-Q1 to F-Q4): 20 μL of each of the standard solutions for QC for Fuc-GlcNAc-Asn measurement (F-QS-1 to F-QS-4) prepared in Example 22 and 80 μL of the internal standard solution for Fuc-GlcNAc-Asn measurement (F-IS-1) prepared in Example 23 were respectively mixed according to the following Table 36 and stirred, and then centrifugation was performed for 5 minutes under the conditions of 16,000×g and 20°C. Supernatants thus obtained were each filled into a vial and were used as standard samples for QC for Fuc-GlcNAc-Asn measurement (F-Q1 to F-Q4).

[Table 36]

| Table 36. Preparation of standard samples for QC for Fuc-GlcNAc-Asn measurement | | | | | | |
|---|---|---|---|---|---|---|
| Sample name | Standard solution for calibration curve for Fuc-GlcNAc-Asn measurement | | | Internal standard solution for Fuc-GlcNAc-Asn measurement | | |
| | Solution name | Amount of addition (μL) | Concentration in sample (ng/mL) | Solution name | Amount of addition (μL) | Concentration in sample (ng/mL) |
| F-Q4 | F-QS-4 | 20 | 240 | F-IS-1 | 80 | 10 |
| F-Q3 | F-QS-3 | 20 | 10 | F-IS-1 | 80 | 10 |
| F-Q2 | F-QS-2 | 20 | 0.3 | F-IS-1 | 80 | 10 |

(continued)

| Table 36. Preparation of standard samples for QC for Fuc-GlcNAc-Asn measurement | | | | | | |
|---|---|---|---|---|---|---|
| Sample name | Standard solution for calibration curve for Fuc-GlcNAc-Asn measurement | | | Internal standard solution for Fuc-GlcNAc-Asn measurement | | |
| | Solution name | Amount of addition (μL) | Concentration in sample (ng/mL) | Solution name | Amount of addition (μL) | Concentration in sample (ng/mL) |
| F-Q1 | F-QS-1 | 20 | 0.1 | F-IS-1 | 80 | 10 |

Measurement samples (brain, liver, kidney, spleen): The tissue extracts of brain, liver, kidney, and spleen obtained in Example 24 were diluted 100 times with pure water as necessary. To 4 μL of each of the tissue extracts (or each of the tissue extracts diluted 100 times), 4 μL of pure water and 32 μL of the internal standard solution for Fuc-GlcNAc-Asn measurement F-IS-1 prepared in Example 23 were respectively added, and the mixtures were stirred. Subsequently, centrifugation was performed for 5 minutes under the conditions of 16,000×g and 20°C, and a supernatant thus obtained was filled into a vial and was used as a measurement sample.

Measurement samples (CSF and blood plasma): The CSF and blood plasma obtained in Example 24 were diluted 10 times with pure water as necessary. To 4 μL of the CSF or the blood plasma (or the CSF or blood plasma diluted 10 times), 4 μL of pure water and 32 μL of the internal standard solution for Fuc-GlcNAc-Asn measurement F-IS-1 prepared in Example 23 were added, and the mixture was stirred. Subsequently, centrifugation was performed for 5 minutes under the conditions of 16,000×g and 20°C, and supernatants thus obtained were each filled into a vial and were used as measurement samples.

Measurement sample (urine): 18 μL of pure water was added to 2 μL of the urine obtained in Example 24 to prepare 10-fold diluted urine. The 10-fold diluted urine was further diluted 100 times with pure water as necessary to obtain 1000-fold diluted urine. To 4 μL of the 10-fold diluted urine (or 1000-fold diluted urine), 4 μL of pure water and 32 μL of the internal standard solution for Fuc-GlcNAc-Asn measurement F-IS-1 prepared in Example 24 were added, and the mixture was stirred. Subsequently, centrifugation was performed for 5 minutes under the conditions of 16,000×g and 20°C. A supernatant thus obtained was filled into a vial and was used as a measurement sample.

[Example 26] LC/MS/MS analysis

LC/MS/MS analysis was carried out using a combination of normal phase chromatography and a tandem quadrupole type mass analyzer. QTRAP5500 (AB Sciex Pte., Ltd.) was used as a mass analyzer (MS/MS apparatus), and Nexera X2 (SHIMADZU CORPORATION) was mounted as an HPLC apparatus on the mass analyzer. Furthermore, Unison UK-Amino 2 mm × 150 mm (Imtakt Corp.) was used as an analytic column, and UK-Amino 5 × 2 mm (Imtakt Corp.) was used as a guide column. The mobile phase E and the mobile phase F prepared in Example 21 were used as mobile phases. Furthermore, the column temperature was set to 40°C.

The columns were equilibrated with a mixed liquid composed of 48% (v/v) of the mobile phase E and 52% (v/v) of the mobile phase F, subsequently 10 μL of a sample was injected in, and chromatography was performed under the conditions of the mobile phase shown in Table 37. Incidentally, the flow rate of the mobile phase was set to 0.4 mL/min.

[Table 37]

| Table 37. Conditions for liquid chromatography for Fuc-GlcNAc-Asn measurement | | |
|---|---|---|
| Time lapsed after sample injection (min) | Mobile phase E (% (V/V)) | Mobile phase F (% (V/V)) |
| 0.0 | 48 | 52 |
| 2.0 | Stop | |

The ion source parameters, MS internal parameters, and valve switching program of the MS/MS apparatus were set as shown in Table 38 to Table 40, respectively, according to the instruction manual of QTRAP5500 (AB Sciex Pte., Ltd.).

[Table 38]

| Table 38. Ion source parameters for MS/MS apparatus for Fuc-GlcNAc-Asn measurement | |
|---|---|
| Ion Source | ESI |

(continued)

| Table 38. Ion source parameters for MS/MS apparatus for Fuc-GlcNAc-Asn measurement | |
|---|---|
| Polarity | Negative |
| Scan Type | MRM |
| Curtain Gas (CUR) | 30 |
| Collision Gas (CAD) | 10 |
| IonSpray Voltage (IS) | 5500 |
| Temperature (TEM) | 600 |
| Ion Source Gas 1 (GS1) | 80 |
| Ion Source Gas 2 (GS2) | 80 |

[Table 39]

| Table 39. MS internal parameters for MS/MS apparatus for Fuc-GlcNAc-Asn measurement | | | | | | | | | |
|---|---|---|---|---|---|---|---|---|---|
| | Detection ion | Theoretical value | Q1 Mass (Da) | Q3 Mass (Da) | Time (msec) | DP (volts) | EP (volts) | CE (volts) | CXP (volts) |
| Fuc-GlcNAc-Asn | $[M+H]^+$ | 482.2 | 482.0 | 336.1 | 150 | 81 | 10 | 21 | 22 |
| F-IS | $[M+H]^+$ | 382.2 | 382.0 | 204.1 | 150 | 61 | 10 | 27 | 12 |

[Table 40]

| Table 40. Valve switching program for MSIMS apparatus for Fuc-GlcNAc-Asn measurement | |
|---|---|
| Time (min) | Valve location |
| 0.0 to 0.1 | A |
| 0.1 to 1.9 | B |
| 1.9 to 2.0 | A |

The measured values (ng/mL) and trueness (%) were respectively set to three-digit significant numbers. Furthermore, the trueness (%) was calculated based on the following calculation formula. Trueness (%) = Measured value/theoretical concentration × 100 Incidentally, the theoretical concentration in the above-described calculation formula means the concentration of Fuc-GlcNAc-Asn added to a standard sample for calibration curve for Fuc-GlcNAc-Asn measurement or a standard sample for QC for Fuc-GlcNAc-Asn measurement.

[Example 27] Evaluation of calibration curve for Fuc-GlcNAc-Asn measurement and quantification range

The standard samples for calibration curve for Fuc-GlcNAc-Asn measurement and the standard samples for QC for Fuc-GlcNAc-Asn measurement prepared in Example 25 were measured, and the areas of the peaks (detection peaks) detected on the chromatographic charts of the product ions derived from Fuc-GlcNAc-Asn included in the standard samples for calibration curve for Fuc-GlcNAc-Asn measurement and the standard samples for QC for Fuc-GlcNAc-Asn measurement were determined. Furthermore, the area of the detection peak of the product ion derived from the internal standard solution was determined. The trueness for each preparation concentration (theoretical concentration) of the standard sample for calibration curve for Fuc-GlcNAc-Asn measurement is shown in Table 41, and the trueness for each preparation concentration (theoretical concentration) of the standard sample for QC for Fuc-GlcNAc-Asn measurement is shown in Table 42. In the range of 0.3 to 300 ng/mL, the standard samples for calibration curve for Fuc-GlcNAc-Asn measurement could be measured at a trueness of 95.7% to 107%, and the standard samples for QC for Fuc-GlcNAc-Asn measurement could be measured at a trueness of 93.1% to 106%.

[Table 41]

| Table 41. Measurement results and trueness evaluation for standard samples for calibration curve for Fuc-GlcNAc-Asn measurement | | | |
|---|---|---|---|
| Sample name | Preparation concentration (ng/mL) | Calculated concentration (ng/mL) | Trueness (%) |
| F-S7 | 300 | 299 | 99.7 |
| F-S6 | 100 | 102 | 102 |
| F-S5 | 30 | 29.3 | 97.5 |
| F-S4 | 10 | 9.79 | 97.9 |
| F-S3 | 3 | 2.87 | 95.7 |
| F-S2 | 1 | 1 | 100 |
| F-S1 | 0.3 | 0.321 | 107 |

[Table 42]

| Table 42. Measurement results and trueness evaluation for standard samples for QC for Fuc-GlcNAc-Asn measurement | | | |
|---|---|---|---|
| Sample name | Preparation concentration (ng/mL) | Calculated concentration (ng/mL) | Trueness (%) |
| F-Q4 | 240 | 223 | 93.1 |
| F-Q3 | 10 | 10.2 | 102 |
| F-Q2 | 0.3 | 0.319 | 106 |
| F-Q1 | 0.1 | 0.104 | 104 |

Furthermore, the area ratio (Fuc-GlcNAc-Asn detection peak area/F-IS detection peak area) of the area (Fuc-GlcNAc-Asn detection peak area) of the detection peak originating from Fuc-GlcNAc-Asn included in each standard sample for calibration curve for Fuc-GlcNAc-Asn measurement with respect to the area (F-IS detection peak area) of the detection peak originating from the internal standard solution for Fuc-GlcNAc-Asn measurement was determined. This value was plotted on the ordinate axis, the concentration of Fuc-GlcNAc-Asn (Fuc-GlcNAc-Asn concentration) of each standard sample for calibration curve for Fuc-GlcNAc-Asn measurement was plotted on the abscissa axis, a regression equation was calculated using a quadratic programming method, and a calibration curve for Fuc-GlcNAc-Asn measurement was created. The obtained calibration curve for Fuc-GlcNAc-Asn measurement showed a satisfactory quadratic curvilinearity in the range of 0.3 to 300 ng/mL (Fig. 23). The correlation coefficient (r) was 0.9999.

[Example 28] Measurement of Fuc-GlcNAc-Asn concentration in various tissues, CSF, blood plasma, and urine of wild-type mouse and model mouse

The Fuc-GlcNAc-Asn concentrations in various tissues, blood plasma, urine, and CSF of each of wild-type mice and FUCA1 KO mice were calculated using the calibration curve obtained in Example 27 and the measurement samples prepared in Example 25. The measurement results are respectively shown in Fig. 24 to Fig. 30. In all of the tissues, blood plasma, urine, and CSF, results that the concentration of Fuc-GlcNAc-Asn was higher in the FUCA1 KO mice as compared to the wild-type mice were obtained. These results show that the concentrations of Fuc-GlcNAc-Asn in the various tissues, blood plasma, urine, and CSF increase in the FUCA1 KO mice as compared to the wild-type mice.

[Example 29] Comparison of Fuc-GlcNAc-Asn concentrations in brain and CSF

With regard to the relationship between the Fuc-GlcNAc-Asn concentration in the brain and the Fuc-GlcNAc-Asn concentration in the CSF in the same mouse individuals, the results of plotting are shown in Fig. 31. The coefficient of determination ($R^2$) was 0.981, and high correlativity was recognized.

These results show that the Fuc-GlcNAc-Asn concentration in the CSF reflects the Fuc-GlcNAc-Asn concentration in the brain, and that the Fuc-GlcNAc-Asn concentration in the CSF can be used as a biomarker in the central nervous system for a disease in which Fuc-GlcNAc-Asn and/or $\alpha$-L-fucoside accumulates in the brain.

Hereinafter, Examples 30 to 38 relate to the measurement of lyso-sulfatide.

[Example 30] Preparation reagent solution

Mobile phase G: 1 mL of formic acid (Fujifilm Wako Pure Chemical Corp.) and 499 mL of pure water were mixed, and this mixture was used as mobile phase G.
Mobile phase H: 1 mL of formic acid (Fujifilm Wako Pure Chemical Corp.) and 499 mL of acetonitrile (for LC/MS, Fujifilm Wako Pure Chemical Corp.) were mixed, and this mixture was used as mobile phase H.
60% Methanol solution: Methanol and water were mixed at proportions of 60 : 40 (v/v), and the mixture was used as a 60% methanol solution.
90% Methanol solution: Methanol and water were mixed at proportions of 90 : 10 (v/v), and the mixture was used as a 90% methanol solution.

[Example 31] Preparation of standard solutions for *lyso*-sulfatide measurement

Standard stock solution for *lyso*-sulfatide measurement: 1 mg of *lyso*-Sulfatide ($NH_4^+$ salt) (*lyso*-sulfatide, Matreya, LLC) was dissolved in 1 mL of methanol to prepare a 1 mg/mL solution, and this was used as a standard stock solution for lyso-sulfatide measurement.
Standard solutions for calibration curve for *lyso*-sulfatide measurement (S-SS-1 to S-SS-8): Serial dilutions of the standard stock solution for *lyso*-sulfatide measurement were prepared using methanol according to the following Table 43 to prepare standard solutions for calibration curve for *lyso*-sulfatide measurement (S-SS-1 to S-SS-8).

[Table 43]

Table 43. Preparation of standard solutions for calibration curve for *lyso*-sulfatide measurement

| Solution name | Preparation concentration (ng/mL) |
|---|---|
| S-SS-8 | 2000 |
| S-SS-7 | 20 |
| S-SS-6 | 6 |
| S-SS-5 | 2 |
| S-SS-4 | 0.6 |
| S-SS-3 | 0.2 |
| S-SS-2 | 0.06 |
| S-SS-1 | 0.02 |

Standard solutions for QC for *lyso*-sulfatide measurement (S-QS-1 to S-QS-4): Serial dilutions of the standard solution for calibration curve for *lyso*-sulfatide measurement S-SS-8 were prepared using methanol according to the following Table 44 to prepare standard solutions for QC for *lyso*-sulfatide measurement (S-QS-1 to S-QS-4).

[Table 44]

Table 44. Preparation of standard solutions for QC for *lyso*-sulfatide measurement

| Solution name | Preparation concentration (ng/mL) |
|---|---|
| S-QS-4 | 16 |
| S-QS-3 | 2 |
| S-QS-2 | 0.06 |
| S-QS-1 | 0.02 |

[Example 32] Preparation of internal standard solutions for *lyso*-sulfatide measurement

Internal standard stock solution for *lyso*-sulfatide measurement: 1 mg of N-glycinated *lyso*-sulfatide (S-IS, Matreya, LLC) was dissolved in 1 mL of methanol to prepare a 1 mg/mL solution, and this was used as an internal standard stock solution for *lyso*-sulfatide measurement.
Internal standard solutions for *lyso*-sulfatide measurement (SIS-1 to S-IS-3): Serial dilutions of the internal

standard stock solution for *lyso*-sulfatide measurement were prepared using methanol according to the following Table 45 to prepare internal standard solutions for *lyso*-sulfatide measurement (S-IS-1 to SIS-3).

[Table 45]

| Table 45. Preparation of internal standard solution for *lyso*-sulfatide measurement | |
|---|---|
| Solution name | Preparation concentration (ng/mL) |
| S-IS-3 | 2000 |
| S-IS-2 | 50 |
| S-IS-1 | 2 |

[Example 33] Preparation of tissue extracts, blood plasma, and CSF

Various tissues (brain, spinal cord, sciatic nerve, and kidney) of each of ARSA KO homozygous mice (C57BL/6, 20 to 23 weeks old and 93 to 100 weeks old, Oriental Bio Service, Ltd.), ARSA KO heterozygous mice (C57BL/6, 93 to 100 weeks old, Oriental Bio Service, Ltd.), and wild-type mice (C57BL/6, 20 to 23 weeks old and 93 to 100 weeks old, Oriental Bio Service, Ltd.) were extracted and freeze-dried, and then dry weights thereof were respectively weighed. Each of the tissues, pure water in an amount 39 times the dry weight of the tissue, and three or fifteen Φ5-mm SUS beads (Taitec Corp.) were introduced into a 2-mL Shatter Resistant tube (Scientific Specialties, Inc.) or a 5-mL self-standing type mailing tube (Watson, Inc.), and the tissue was crushed (2500 rpm, for 300 seconds) with a bead crusher (μT-12, Taitec Corp.). The tissue liquid after crushing was left to stand on ice for 30 minutes or longer while being stirred as needed, and about 1 mL each of the tissue liquid was preparatively isolated and was used as a tissue extract. Furthermore, about 300 μL each of blood plasma was preparatively isolated from each individual. Furthermore, about 15 μL each of cerebrospinal fluid (CSF), was preparatively isolated from each individual.

[Example 34] Preparation of various samples

Standard samples for calibration curve for *lyso*-sulfatide measurement (S-S0 to S-S7): 50 μL of each of the standard solutions for calibration curve for *lyso*-sulfatide measurement (S-SS-1 to S-SS-7) prepared in Example 31 and 50 μL of the internal standard solution for *lyso*-sulfatide measurement (S-IS-1) prepared in Example 32 were respectively mixed according to the following Table 46, and the mixtures were stirred. Subsequently, centrifugation was performed for 5 minutes using a high-speed microcentrifuge (MX-307, Tomy Seiko Co., Ltd.) under the conditions of 16,000×g and 20°C, and supernatants thus obtained were each filled into a vial and were used as standard samples for calibration curve for *lyso*-sulfatide measurement (S-S0 to S-S7).

[Table 46]

| Table 46. Preparation of standard samples for calibration curve for *lyso*-sulfatide measurement | | | | | | |
|---|---|---|---|---|---|---|
| Sample name | Standard solution for calibration curve for *lyso*-sulfatide measurement | | | Internal standard solution for *lyso*-sulfatide measurement | | |
| | Solution name | Amount of addition (μL) | Concentration in sample (ng/mL) | Solution name | Amount of addition (μL) | Concentration in sample (ng/mL) |
| S-S7 | S-SS-7 | 50 | 10 | S-IS-1 | 50 | 1 |
| S-S6 | S-SS-6 | 50 | 3 | S-IS-1 | 50 | 1 |
| S-S5 | S-SS-5 | 50 | 1 | S-IS-1 | 50 | 1 |
| S-S4 | S-SS-4 | 50 | 0.3 | S-IS-1 | 50 | 1 |
| S-S3 | S-SS-3 | 50 | 0.1 | S-IS-1 | 50 | 1 |
| S-S2 | S-SS-2 | 50 | 0.03 | S-IS-1 | 50 | 1 |
| S-S1 | S-SS-1 | 50 | 0.01 | S-IS-1 | 50 | 1 |
| Zero sample (S-S0) | Methanol | 50 | 0 | Methanol | 50 | 0 |

Standard samples for QC for *lyso*-sulfatide measurement (S-Q1 to S-Q4): 50 μL of each of the standard solutions for QC for *lyso*-sulfatide measurement (S-QS-1 to S-QS-4) prepared in Example 31 and 50 μL of the internal standard solution for *lyso*-sulfatide measurement (S-IS-1) prepared in Example 31 were respectively mixed according to the following Table 47, and the mixtures were stirred. Subsequently, centrifugation was performed for 5 minutes under the conditions of 16,000×g and 20°C, and supernatants thus obtained were each filled into a vial and were used as standard samples for QC for *lyso*-sulfatide measurement (S-Q1 to S-Q4).

[Table 47]

Table 47. Preparation of standard samples for QC for *lyso*-sulfatide measurement

| Sample name | Standard solution for calibration curve for *lyso*-sulfatide measurement | | | Internal standard solution for *lyso*-sulfatide measurement | | |
|---|---|---|---|---|---|---|
| | Solution name | Amount of addition (μL) | Concentration in sample (ng/mL) | Solution name | Amount of addition (μL) | Concentration in sample (ng/mL) |
| S-Q4 | S-QS-4 | 50 | 8 | S-IS-1 | 50 | 1 |
| S-Q3 | S-QS-3 | 50 | 1 | S-IS-1 | 50 | 1 |
| S-Q2 | S-QS-2 | 50 | 0.03 | S-IS-1 | 50 | 1 |
| S-Q1 | S-QS-1 | 50 | 0.01 | S-IS-1 | 50 | 1 |

Measurement sample (brain, spinal cord, sciatic nerve, and kidney): To 80 μL of each of the tissue extracts of brain, spinal cord, sciatic nerve, and kidney obtained in Example 33, 240 μL of pure water and 467.2 μL of methanol were added, and the mixture was stirred. Subsequently, centrifugation was performed for 5 minutes under the conditions of 16,000×g and 20°C, and a supernatant was obtained. 590.4 μL of the obtained supernatant was preparatively isolated, 9.6 μL of the internal standard solution for *lyso*-sulfatide measurement S-IS-2 was added thereto, and 500 μL from 600 μL of the obtained solution was loaded into an Oasis HLB 1 cc Vac Cartridge (30 mg, 30 μm, Nihon Waters K.K.). The solution was washed with 1 mL of the 60% methanol solution and then was eluted with 1 mL of the 90% methanol solution. After stirring, centrifugation was performed for 5 minutes under the conditions of 16,000×g and 20°C, and 20 μL of the obtained supernatant was filled into a sample cup IA (Shinwa Chemical Industries, Ltd.) and was used as a measurement sample.

Measurement sample (blood plasma): To 80 μL of the blood plasma obtained in Example 33, 240 μL of pure water and 467.2 μL of methanol were added, and the mixture was stirred. Subsequently, centrifugation was performed for 5 minutes under the conditions of 16,000×g and 20°C, and a supernatant was obtained. 590.4 μL of the obtained supernatant was preparatively isolated, 9.6 μL of the internal standard solution for lyso-sulfatide measurement SIS-2 was added thereto, and 500 μL from 600 μL of the obtained solution was loaded into an Oasis HLB 1 cc Vac Cartridge (30 mg, 30 μm, Nihon Waters K.K.). The solution was washed with 1 mL of the 60% methanol solution and then was eluted with 1 mL of the 90% methanol solution. The solvent of 400 μL of the eluted fraction was distilled off using a centrifugal concentrator (CC-105, Tomy Seiko Co., Ltd.), subsequently 40 μL of methanol was added thereto, and the mixture was redissolved. After stirring, centrifugation was performed for 5 minutes under the conditions of 16,000×g and 20°C, and 20 μL of the obtained supernatant was filled into a sample cup IA (Shinwa Chemical Industries, Ltd.) and was used as a measurement sample.

Measurement sample (CSF): To 4 μL of the CSF obtained in Example 33, 10 μL of the internal standard solution for *lyso*-sulfatide measurement prepared in Example 32 and 6 μL of methanol were added, and the mixture was stirred. Subsequently, centrifugation was performed for 5 minutes under the conditions of 16,000×g and 20°C, and the obtained supernatant was filled into a sample cup IA (Shinwa Chemical Industries, Ltd.) and was used as a measurement sample.

[Example 35] LC/MS/MS analysis

LC/MS/MS analysis was carried out using a combination of reverse phase chromatography and a tandem quadrupole type mass analyzer. QTRAP5500 (AB Sciex Pte., Ltd.) was used as a mass analyzer (MS/MS apparatus), and Nexera X2 (SHIMADZU CORPORATION) was mounted as an HPLC apparatus on the mass analyzer. Furthermore, Cadenza CW-C18 2 mm × 150 mm (Imtakt Corp.) was used as an analytic column, and CW-C18 5 × 2 mm (Imtakt Corp.) was used as a guide column. The mobile phase G and the mobile phase H prepared in Example 30 were used as mobile phases. Furthermore, the column temperature was set to 40°C.

The columns were equilibrated with a mixed liquid composed of 62.5% (v/v) of the mobile phase G and 37.5% (v/v) of the mobile phase H, subsequently 10 μL of a sample was injected, and chromatography was performed under the

conditions of the mobile phase as shown in Table 48. Incidentally, the flow rate of the mobile phase was set to 0.4 mL/min.

[Table 48]

| Table 48. Conditions for liquid chromatography for *lyso*-sulfatide measurement | | |
|---|---|---|
| Time lapsed after sample injection (min) | Mobile phase G (% (V/V)) | Mobile phase H (% (V/V)) |
| 0.0 | 62.5 | 37.5 |
| 1.0 | 62.5 | 37.5 |
| 1.5 | 5 | 95 |
| 2.9 | 5 | 95 |
| 3.0 | 62.5 | 37.5 |
| 5.0 | Stop | |

The ion source parameters, MS internal parameters, and valve switching program of the MS/MS apparatus wee respectively set as shown in Table 49 to Table 51 according to the instruction manual of QTRAP5500 (AB Sciex Pte., Ltd.).

[Table 49]

| Table 49. Ion source parameters for MS/MS apparatus for *lyso*-sulfatide measurement | |
|---|---|
| Ion Source | ESI |
| Polarity | Positive |
| Scan Type | MRM |
| Curtain Gas (CUR) | 40 |
| Collision Gas (CAD) | 9 |
| IonSpray Voltage (IS) | 5500 |
| Temperature (TEM) | 500 |
| Ion Source Gas 1 (GS1) | 60 |
| Ion Source Gas 2 (GS2) | 70 |

[Table 50]

| Table 50. MS internal parameters for MS/MS apparatus for *lyso*-sulfatide measurement | | | | | | | | | |
|---|---|---|---|---|---|---|---|---|---|
| | Detection ion | Theoretical value | Q1 Mass (Da) | Q3 Mass (Da) | Time (msec) | DP (volts) | EP (volts) | CE (volts) | CXP (volts) |
| *lyso*-sulfatide | $[M+H]^+$ | 542.3 | 542.1 | 282.2 | 150 | 181 | 10 | 39 | 18 |
| S-IS | $[M+H]^+$ | 599.3 | 599.1 | 339.2 | 150 | 131 | 10 | 31 | 24 |

[Table 51]

| Table 51. Valve switching program for MS/MS apparatus for *lyso*-sulfatide measurement | |
|---|---|
| Time (min) | Valve location |
| 0.0 to 0.1 | A |
| 0.1 to 4.9 | B |
| 4.9 to 5.0 | A |

The measured values (ng/mL) and trueness (%) were respectively set to three-digit significant numbers. Furthermore, the trueness (%) was calculated based on the following calculation formula.

Trueness (%) = Measured value/theoretical concentration × 100

Incidentally, the theoretical concentration in the above-described calculation formula means the concentration of *lyso*-sulfatide added to a standard sample for calibration curve for *lyso*-sulfatide measurement or a standard sample for QC for *lyso*-sulfatide measurement.

[Example 36] Evaluation of calibration curve for *lyso*-sulfatide measurement and quantification range

The standard samples for calibration curve for *lyso*-sulfatide measurement and the standard samples for QC for *lyso*-sulfatide measurement prepared in Example 34 were measured, and the areas of the peaks (detection peaks) detected on the chromatographic charts of the product ions derived from *lyso*-sulfatide included in the standard samples for calibration curve for *lyso*-sulfatide measurement and the standard samples for QC for *lyso*-sulfatide measurement were determined. Furthermore, the area of the detection peak of the product ion derived from the internal standard solution for *lyso*-sulfatide measurement was determined. The trueness for each preparation concentration (theoretical concentration) of the standard sample for calibration curve for *lyso*-sulfatide measurement is shown in Table 52, and the trueness for each preparation concentration (theoretical concentration) of the standard sample for QC for *lyso*-sulfatide measurement is shown in Table 53. In the range of 0.01 to 10 ng/mL, the standard samples for calibration curve for *lyso*-sulfatide measurement could be measured at a trueness of 95.3% to 105%, and the standard samples for QC for *lyso*-sulfatide measurement could be measured at a trueness of 98.3% to 103%.

[Table 52]

Table 52. Measurement results and trueness evaluation for standard samples for calibration curve for *lyso*-sulfatide measurement

| Sample name | Preparation concentration (ng/mL) | Calculated concentration (ng/mL) | Trueness (%) |
|---|---|---|---|
| S-S7 | 10 | 10.0 | 100 |
| S-S6 | 3 | 2.99 | 99.6 |
| S-S5 | 1 | 0.996 | 99.6 |
| S-S4 | 0.3 | 0.309 | 103 |
| S-S3 | 0.1 | 0.105 | 105 |
| S-S2 | 0.03 | 0.0286 | 95.3 |
| S-S1 | 0.01 | 0.00973 | 97.3 |

[Table 53]

Table 53. Measurement results and trueness evaluation for standard samples for QC for lyso-sulfatide measurement

| Sample name | Preparation concentration (ng/mL) | Calculated concentration (ng/mL) | Trueness (%) |
|---|---|---|---|
| S-Q4 | 8 | 7.99 | 99.9 |
| S-Q3 | 1 | 1.03 | 103 |
| S-Q2 | 0.03 | 0.0295 | 98.3 |
| S-Q1 | 0.01 | 0.0102 | 102 |

Furthermore, the area ratio (*lyso*-sulfatide detection peak area/S-IS detection peak area) of the area (*lyso*-sulfatide detection peak area) of the detection peak originating from *lyso*-sulfatide included in each standard sample for calibration curve for *lyso*-sulfatide measurement with respect to the area (S-IS detection peak area) of the detection peak originating from the internal standard solution for *lyso*-sulfatide measurement was determined. This value was plotted on the ordinate axis, the concentration of *lyso*-sulfatide (*lyso*-sulfatide concentration) of each standard sample for calibration curve for *lyso*-sulfatide measurement was plotted on the abscissa axis, a regression equation was calculated using a quadratic programming method, and a calibration curve for *lyso*-sulfatide measurement was created. A satisfactory fitted curve was obtained in the range of 0.01 to 10 ng/mL (Fig. 32). The correlation coefficient (r) was 0.9999.

[Example 37] Measurement of *lyso*-sulfatide concentration in various tissues, blood plasma, and CSF of wild-type mouse and model mouse

The *lyso*-sulfatide concentrations in various tissues, blood plasma, and CSF of each of wild-type mice, ARSA KO homozygous mice, and ARSA KO heterozygous mice were calculated using the calibration curve for *lyso*-sulfatide measurement obtained in Example 36 and the measurement samples prepared in Example 34. The measurement results are respectively shown in Fig. 33 to Fig. 38. In all of the tissues, blood plasma, and CSF, results that the concentration of *lyso*-sulfatide was higher in the ARSA KO homozygous mice as compared to the wild-type mice were obtained. Furthermore, the concentration of *lyso*-sulfatide was lower in the ARSA KO heterozygous mice, and the concentration was similar in the wild-type mice. These results show that the concentration of *lyso*-sulfatide in the various tissues, blood plasma, and CSF increases in the ARSA KO homozygous mice as compared to the wild-type mice. Incidentally, an age-dependent increase in the concentration of *lyso*-sulfatide can be confirmed in the various tissues, CSF, and blood plasma of the ARSA KO homozygous mice.

[Example 38] Comparison of *lyso*-sulfatide concentrations in brain and CSF

With regard to the relationship between the *lyso*-sulfatide concentration in the brain and the *lyso*-sulfatide concentration in the CSF in the same mouse individuals, the results of plotting are shown in Fig. 39. The coefficient of determination ($R^2$) was 0.9187, and high correlativity was recognized.
These results show that the *lyso*-sulfatide concentration in the CSF reflects the *lyso*-sulfatide concentration in the brain, and that the *lyso*-sulfatide concentration in the CSF can be used as a biomarker in the central nervous system for a disease in which *lyso*-sulfatide and/or sulfatide accumulates in the brain.

[INDUSTRIAL APPLICABILITY]

According to the invention, diagnosis of a disease that develops as glucose tetrasaccharide and/or glycogen accumulates in the central nervous system can be conducted, and a method for investigating the effect of treatment carried out in order to reduce the glucose tetrasaccharide and/or glycogen accumulated in the central nervous system, the treatment being conducted for a disease such as described above, and a diagnostic kit can be provided.
Furthermore, according to the invention, diagnosis of a disease that develops as *lyso*-monosialoganglioside GM1 and/or monosialoganglioside GM1 accumulates in the central nervous system can be conducted, and a method for investigating the effect of treatment carried out in order to reduce *lyso*-monosialoganglioside GM1 and/or monosialoganglioside GM1 accumulated in the central nervous system, the treatment being conducted for a disease such as described above, and a diagnostic kit can be provided.
Furthermore, according to the invention, diagnosis of a disease that develops as Fuc-GlcNAc-Asn and/or α-L-fucoside accumulates in the central nervous system can be conducted, and a method for investigating the effect of treatment carried out in order to reduce Fuc-GlcNAc-Asn and/or α-L-fucoside accumulated in the central nervous system, the treatment being conducted for a disease such as described above, and a diagnostic kit can be provided.
Furthermore, according to the invention, diagnosis of a disease that develops as *lyso*-sulfatide and/or sulfatide accumulates in the central nervous system can be conducted, and a method for investigating the effect of treatment carried out in order to reduce *lyso*-sulfatide and/or sulfatide accumulated in the central nervous system, the treatment being conducted for a disease such as described above, and a diagnostic kit can be provided.

## Claims

**1.** A method for quantifying Hex4 contained in an isolated cerebrospinal fluid, the method comprising:

a step of adding an internal standard substance to a solution containing the isolated cerebrospinal fluid;
a step of applying the solution containing the isolated cerebrospinal fluid, to which the internal standard substance has been added, to liquid chromatography to obtain an eluate; and
a step of subjecting the eluate to mass analysis;
wherein the Hex4 is measured in comparison with the internal standard substance; and
wherein the internal standard substance is represented by either the following Formula (VII):

[Chemical Formula 7]

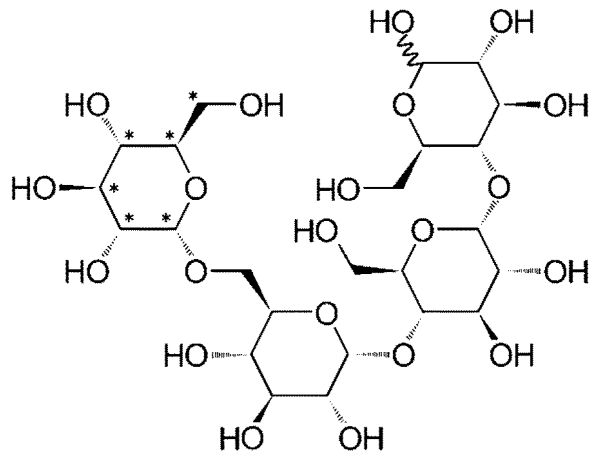

(VII)

wherein at least one of the carbon atoms marked by asterisks is carbon-13, or by the following Formula (XXV):

[Chemical Formula 25]

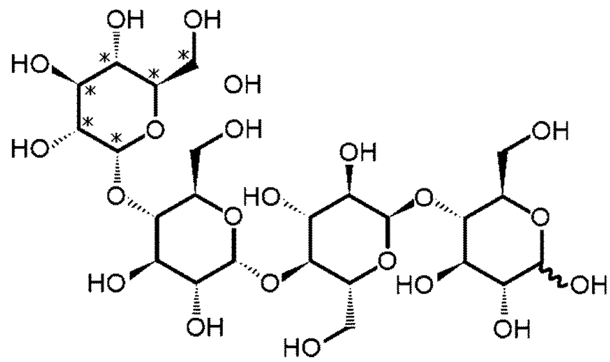

(XXV)

wherein at least one of the carbon atoms marked by asterisks is carbon-13.

2. The method according to claim 1, wherein the liquid chromatography is hydrophilic interaction liquid chromatography.

3. The method according to any claim 1 or 2, wherein the isolated cerebrospinal fluid is obtained from a patient with a disease in which Hex4 and/or glycogen accumulates in the body, optionally wherein the disease is Pompe disease, further optionally wherein the isolated cerebrospinal fluid is obtained from a patient who has previously received treatment to reduce Hex4 and/or glycogen present in the body.

4. A method for quantifying *lyso*-monosialoganglioside GM1 contained in an isolated cerebrospinal fluid, the method comprising:

a step of adding an internal standard substance to a solution containing the isolated cerebrospinal fluid;
a step of applying the solution containing the isolated cerebrospinal fluid, to which the internal standard substance has been added, to liquid chromatography to obtain an eluate; and
a step of subjecting the eluate to mass analysis;
wherein the *lyso*-monosialoganglioside GM1 is measured in comparison with the internal standard substance; and
wherein the internal standard substance is represented by the following Formula (VIII):

[Chemical Formula 8]

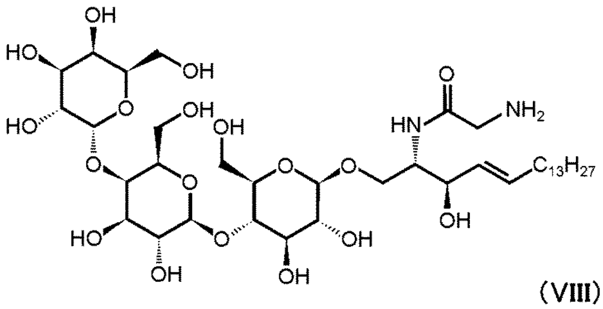

(VIII)

5. The method according to claim 4, wherein the liquid chromatography is reverse phase chromatography.

6. The method according to claim 4 or 5, wherein the isolated cerebrospinal fluid is obtained from a patient with a disease in which *lyso*-monosialoganglioside GM1 and/or monosialoganglioside GM1 accumulates in the body, optionally wherein the disease is GM1 gangliosidosis.

7. The method according to claim 6, wherein the isolated cerebrospinal fluid is obtained from a patient who has previously received treatment to reduce *lyso*-monosialoganglioside GM1 and/or monosialoganglioside GM1 present in the body.

8. A method for quantifying Fuc-GlcNAc-Asn contained in an isolated cerebrospinal fluid, the method comprising:

a step of adding an internal standard substance to a solution containing the isolated cerebrospinal fluid;
a step of applying the solution containing the isolated cerebrospinal fluid, to which the internal standard substance has been added, to liquid chromatography to obtain an eluate; and
a step of subjecting the eluate to mass analysis;
wherein the Fuc-GlcNAc-Asn is measured in comparison with the internal standard substance; and
wherein the internal standard substance is represented by the following Formula (IX):

[Chemical Formula 9]

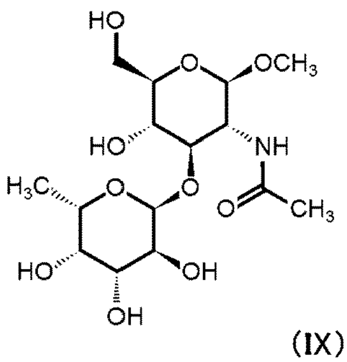

(IX)

9. The method according to claim 8, wherein the liquid chromatography is normal phase chromatography or anion exchange chromatography.

10. The method according to claim 8 or 9, wherein the isolated cerebrospinal fluid is obtained from a patient with a disease in which Fuc-GlcNAc-Asn and/or α-L-fucoside accumulates in the body, optionally wherein the disease is fucoidosis.

11. The method according to claim 10, wherein the isolated cerebrospinal fluid is obtained from a patient who has previously received treatment to reduce Fuc-GlcNAc-Asn and/or α-L-fucoside present in the body.

12. A method for quantifying *lyso*-sulfatide contained in an isolated cerebrospinal fluid, the method comprising:

a step of adding an internal standard substance to a solution containing the isolated cerebrospinal fluid;
a step of applying the solution containing the isolated cerebrospinal fluid, to which the internal standard substance has been added, to liquid chromatography to obtain an eluate; and
a step of subjecting the eluate to mass analysis;
wherein the *lyso*-sulfatide_is measured in comparison with the internal standard substance; and
wherein the internal standard substance is represented by the following Formula (X):

[Chemical Formula 10]

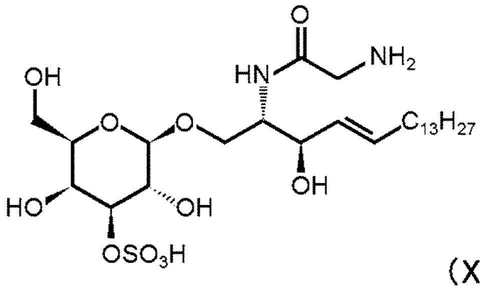

(X)

**13.** The method according to claim 12, wherein the liquid chromatography is reverse phase chromatography.

**14.** The method according to claim 12 or 13, wherein the isolated cerebrospinal fluid is obtained from a patient with a disease in which *lyso*-sulfatide and/or sulfatide accumulates in the body, optionally wherein the disease is metachromatic leukodystrophy.

**15.** The method according to claim 14, wherein the isolated cerebrospinal fluid is obtained from a patient who has previously received treatment to reduce *lyso*-sulfatide and/or sulfatide present in the body.

## Patentansprüche

**1.** Verfahren zur Quantifizierung von Hex4, enthalten in einer isolierten Cerebrospinalflüssigkeit, wobei das Verfahren umfasst:

einen Schritt des Hinzufügens einer internen Standardsubstanz zu einer Lösung, die die isolierte Cerebrospinalflüssigkeit enthält;
einen Schritt der Anwendung der Lösung, die die isolierte Cerebrospinalflüssigkeit enthält, zu der die interne Standardsubstanz hinzugefügt wurde, auf eine Flüssigchromatographie, um ein Eluat zu erhalten;
und einen Schritt des Unterziehens des Eluats einer Massenanalyse;
wobei das Hex4 im Vergleich zu der internen Standardsubstanz gemessen wird; und
wobei die interne Standardsubstanz entweder durch die folgende Formel (VII) dargestellt wird:

[Chemische Formel 7]

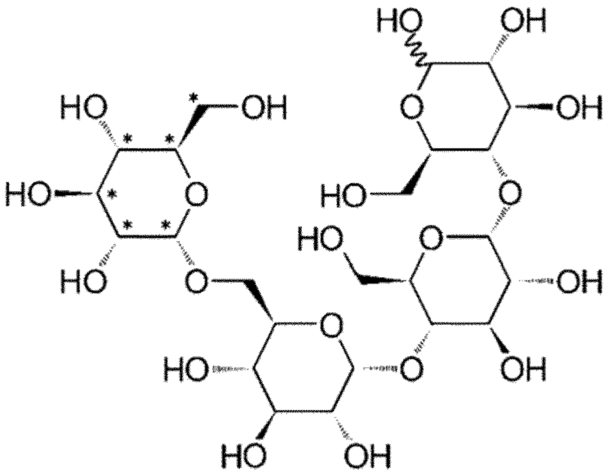

(VII)

wobei mindestens eines der mit Sternchen markierten Kohlenstoffatome Kohlenstoff-13 ist, oder durch die folgende Formel (XXV):

[Chemische Formel 25]

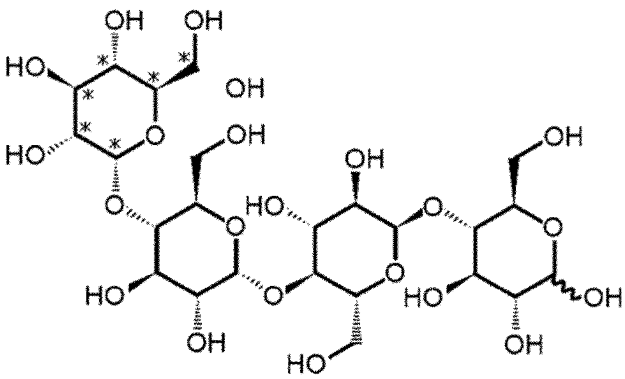

(XXV)

wobei mindestens eines der mit Sternchen markierten Kohlenstoffatome Kohlenstoff-13 ist.

2. Verfahren nach Anspruch 1, wobei die Flüssigchromatographie eine hydrophile Wechselwirkungs-Flüssigchromatographie ist.

3. Verfahren nach einem der Ansprüche 1 oder 2, wobei die isolierte Cerebrospinalflüssigkeit von einem Patienten mit einer Krankheit gewonnen wird, bei der sich Hex4 und/oder Glykogen im Körper ansammelt, optional wobei die Krankheit die Pompe'sche Krankheit ist, weiter optional wobei die isolierte Cerebrospinalflüssigkeit von einem Patienten gewonnen wird, der zuvor eine Behandlung zur Verringerung von Hex4 und/oder Glykogen im Körper erhalten hat.

4. Verfahren zur Quantifizierung von Lyso-Monosialogangliosid GM1, enthalten in einer isolierten Cerebrospinalflüssigkeit, wobei das Verfahren umfasst:

einen Schritt des Hinzufügens einer internen Standardsubstanz zu einer Lösung, die die isolierte Cerebrospinalflüssigkeit enthält;
einen Schritt der Anwendung der Lösung, die die isolierte Cerebrospinalflüssigkeit enthält, zu der die interne Standardsubstanz hinzugefügt wurde, auf eine Flüssigchromatographie, um ein Eluat zu erhalten; und
einen Schritt des Unterwerfens des Eluats einer Massenanalyse;
wobei das Lyso-Monosialogangliosid GM1 im Vergleich zu der internen Standardsubstanz gemessen wird; und

wobei die interne Standardsubstanz durch die folgende Formel (VIII) dargestellt wird:

[Chemische Formel 8]

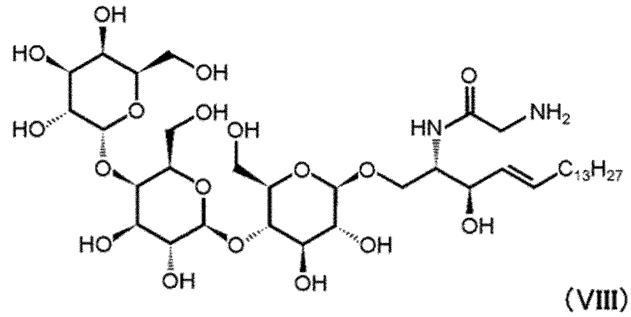

(VIII)

5. Verfahren nach Anspruch 4, wobei die Flüssigchromatographie eine Umkehrphasenchromatographie ist.

6. Verfahren nach Anspruch 4 oder 5, wobei die isolierte Cerebrospinalflüssigkeit von einem Patienten mit einer Krankheit erhalten wird, bei der Lyso-Monosialogangliosid GM1 und/oder Monosialogangliosid GM1 im Körper akkumuliert, wobei die Krankheit optional GM1-Gangliosidose ist.

7. Verfahren nach Anspruch 6, wobei die isolierte Cerebrospinalflüssigkeit von einem Patienten gewonnen wird, der zuvor eine Behandlung zur Verringerung des im Körper vorhandenen Lyso-Monosialogangliosid GM1 und/oder Monosialogangliosid GM1 erhalten hat.

8. Verfahren zur Quantifizierung von Fuc-GlcNAc-Asn, enthalten in einer isolierten Cerebrospinalflüssigkeit, wobei das Verfahren umfasst:

einen Schritt des Hinzufügens einer internen Standardsubstanz zu einer Lösung, die die isolierte Cerebrospinalflüssigkeit enthält;
einen Schritt der Anwendung der Lösung, die die isolierte Cerebrospinalflüssigkeit enthält, zu der die interne Standardsubstanz hinzugefügt wurde, auf eine Flüssigchromatographie, um ein Eluat zu erhalten; und
einen Schritt, bei dem das Eluat einer Massenanalyse unterzogen wird;
wobei das Fuc-GlcNAc-Asn im Vergleich zu der internen Standardsubstanz gemessen wird; und wobei die interne Standardsubstanz durch die folgende Formel (IX) dargestellt wird:

[Chemische Formel 9]

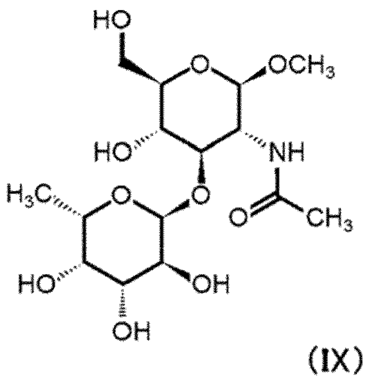

(IX)

9. Verfahren nach Anspruch 8, wobei die Flüssigchromatographie eine Normalphasenchromatographie oder eine Anionenaustauschchromatographie ist.

10. Verfahren nach Anspruch 8 oder 9, wobei die isolierte Cerebrospinalflüssigkeit von einem Patienten mit einer Krankheit gewonnen wird, bei der sich Fuc-GlcNAc-Asn und/oder α-L-Fucosid im Körper ansammelt, wobei die Krankheit optional Fucosidose ist.

**11.** Verfahren nach Anspruch 10, wobei die isolierte Cerebrospinalflüssigkeit von einem Patienten gewonnen wird, der zuvor eine Behandlung zur Verringerung von im Körper vorhandenem Fuc-GlcNAc-Asn und/oder α-L-Fucosid erhalten hat.

**12.** Verfahren zur Quantifizierung von Lyso-Sulfatid, enthalten in einer isolierten Cerebrospinalflüssigkeit, wobei das Verfahren umfasst:

einen Schritt des Hinzufügens einer internen Standardsubstanz zu einer Lösung, die die isolierte Cerebrospinalflüssigkeit enthält;
einen Schritt der Anwendung der Lösung, die die isolierte Cerebrospinalflüssigkeit enthält, zu der die interne Standardsubstanz hinzugefügt wurde, auf eine Flüssigchromatographie, um ein Eluat zu erhalten; und
einen Schritt des Unterziehens des Eluats einer Massenanalyse;
wobei das Lyso-Sulfatid im Vergleich zu der internen Standardsubstanz gemessen wird; und wobei die interne Standardsubstanz durch die folgende Formel (X) dargestellt wird:

[Chemische Formel 10]

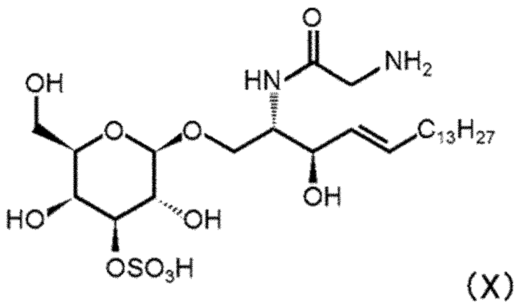

(X)

**13.** Verfahren nach Anspruch 12, wobei die Flüssigchromatographie eine Umkehrphasenchromatographie ist.

**14.** Verfahren nach Anspruch 12 oder 13, wobei die isolierte Cerebrospinalflüssigkeit von einem Patienten mit einer Krankheit gewonnen wird, bei der sich Lyso-Sulfatid und/oder Sulfatid im Körper anreichern, wobei die Erkrankung optional eine metachromatische Leukodystrophie ist.

**15.** Verfahren nach Anspruch 14, wobei die isolierte Cerebrospinalflüssigkeit von einem Patienten gewonnen wird, der zuvor eine Behandlung zur Verringerung des im Körper vorhandenen Lyso-Sulfatids und/oder Sulfatids erhalten hat.

## Revendications

**1.** Procédé de quantification d'Hex4 contenu dans un liquide céphalorachidien isolé, le procédé comprenant :

une étape d'ajout d'une substance étalon interne à une solution contenant le liquide céphalorachidien isolé ;
une étape d'application de la solution contenant le liquide céphalorachidien isolé, à laquelle la substance étalon interne a été ajoutée, à une chromatographie liquide pour obtenir un éluat ; et
une étape de soumission de l'éluat à une analyse de masse ;
dans lequel l'Hex4 est mesuré par rapport à la substance étalon interne ; et
dans lequel la substance étalon interne est représentée soit par la Formule (VII) suivante :

[Formule Chimique 7]

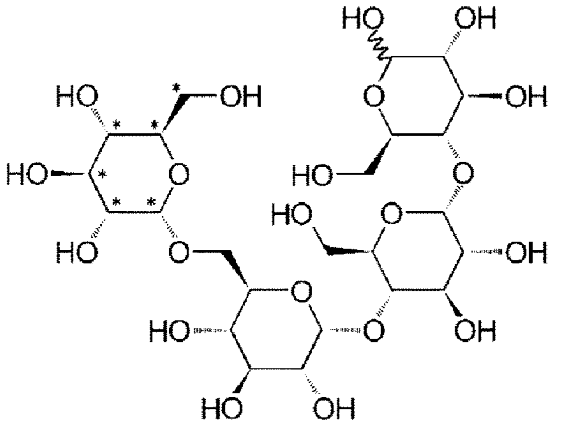

(VII)

dans laquelle au moins l'un des atomes de carbone marqués d'astérisques est du carbone 13, soit par la Formule (XXV) suivante :

[Formule Chimique 25]

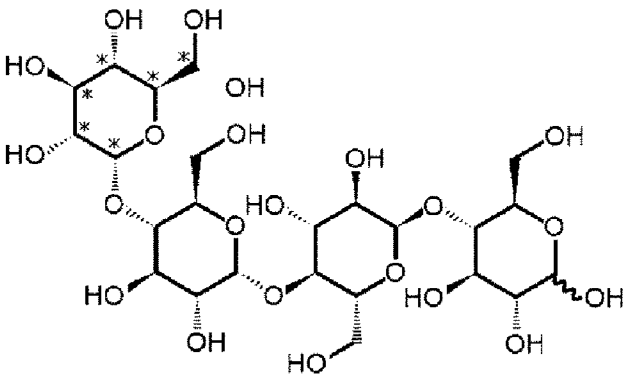

(XXV)

dans laquelle au moins l'un des atomes de carbone marqués d'astérisques est du carbone 13.

2. Procédé selon la revendication 1, dans lequel la chromatographie liquide est une chromatographie liquide à interaction hydrophile.

3. Procédé selon l'une des revendications 1 ou 2, dans lequel le liquide céphalorachidien isolé est obtenu auprès d'un patient atteint d'une maladie dans laquelle Hex4 et/ou du glycogène s'accumulent dans le corps, éventuellement dans lequel la maladie est la maladie de Pompe, en outre éventuellement dans lequel le liquide céphalorachidien isolé est obtenu auprès d'un patient ayant préalablement reçu un traitement visant à réduire Hex4 et/ou le glycogène dans le corps.

4. Procédé de quantification de lyso-monosialoganglioside GM1 contenu dans un liquide céphalorachidien isolé, le procédé comprenant :

une étape d'ajout d'une substance étalon interne à une solution contenant le liquide céphalorachidien isolé ;
une étape d'application de la solution contenant le liquide céphalorachidien isolé, à laquelle la substance étalon interne a été ajoutée, à une chromatographie liquide pour obtenir un éluat ; et
une étape de soumission de l'éluat à une analyse de masse ;

dans lequel le lyso-monosialoganglioside GM1 est mesuré par rapport à la substance étalon interne ; et
dans lequel la substance étalon interne est représentée par la Formule (VIII) suivante :

[Formule Chimique 8]

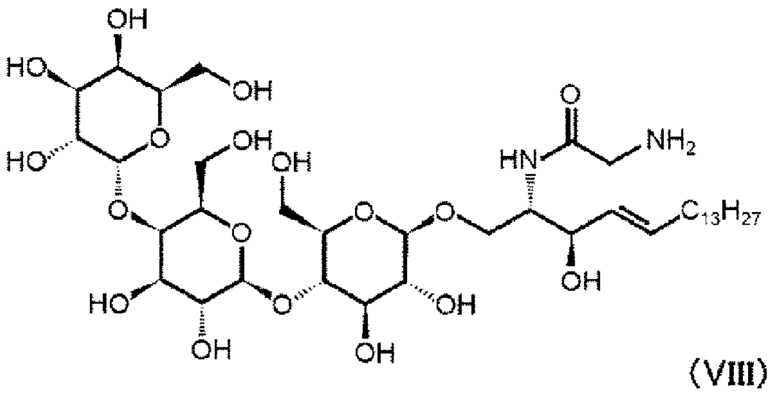

(VIII)

5. Procédé selon la revendication 4, dans lequel la chromatographie liquide est une chromatographie en phase inverse.

6. Procédé selon la revendication 4 ou 5, dans lequel le liquide céphalorachidien isolé est obtenu auprès d'un patient atteint d'une maladie dans laquelle le lyso-monosialoganglioside GM1 et/ou le monosialoganglioside GM1 s'accumule(nt) dans le corps, éventuellement dans lequel la maladie est la gangliosidose à GM1.

7. Procédé selon la revendication 6, dans lequel le liquide céphalorachidien isolé est obtenu auprès d'un patient ayant préalablement reçu un traitement visant à réduire le lyso-monosialoganglioside GM1 et/ou le monosialoganglioside GM1 présent(s) dans le corps.

8. Procédé de quantification de Fuc-GlcNAc-Asn contenu dans un liquide céphalorachidien isolé, le procédé comprenant :

une étape d'ajout d'une substance étalon interne à une solution contenant le liquide céphalorachidien isolé ;
une étape d'application de la solution contenant le liquide céphalorachidien isolé, à laquelle la substance étalon interne a été ajoutée, à une chromatographie liquide pour obtenir un éluat ; et
une étape de soumission de l'éluat à une analyse de masse ;
dans lequel Fuc-GlcNAc-Asn est mesuré par rapport à la substance étalon interne ; et
dans lequel la substance étalon interne est représentée par la Formule (IX) suivante :

[Formule Chimique 9]

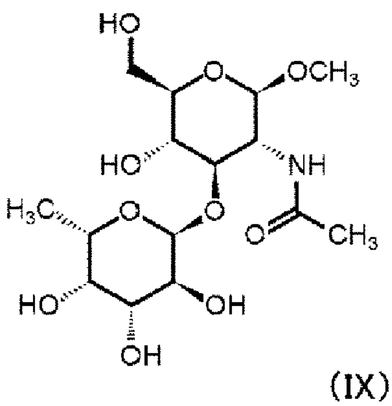

(IX)

9. Procédé selon la revendication 8, dans lequel la chromatographie liquide est une chromatographie en phase normale ou une chromatographie par échange d'anions.

10. Procédé selon la revendication 8 ou 9, dans lequel le liquide céphalorachidien isolé est obtenu auprès d'un patient atteint d'une maladie dans laquelle Fuc-GlcNAc-Asn et/ou l'α-L-fucoside s'accumule(nt) dans le corps, éventuellement dans lequel la maladie est la fucosidose.

11. Procédé selon la revendication 10, dans lequel le liquide céphalorachidien isolé est obtenu auprès d'un patient ayant préalablement reçu un traitement visant à réduire Fuc-GlcNAc-Asn et/ou l'α-L-fucoside présent(s) dans le corps.

12. Procédé de quantification de lyso-sulfatide contenu dans un liquide céphalorachidien isolé, comprenant :

    une étape d'ajout d'une substance étalon interne à une solution contenant le liquide céphalorachidien isolé ;
    une étape d'application de la solution contenant le liquide céphalorachidien isolé, à laquelle la substance étalon interne a été ajoutée, à une chromatographie liquide pour obtenir un éluat ; et
    une étape de soumission de l'éluat à une analyse de masse ;
    dans lequel le lyso-sulfatide est mesuré par rapport à la substance étalon interne ; et
    dans lequel la substance étalon interne est représentée par la Formule (X) suivante :

[Formule Chimique 10]

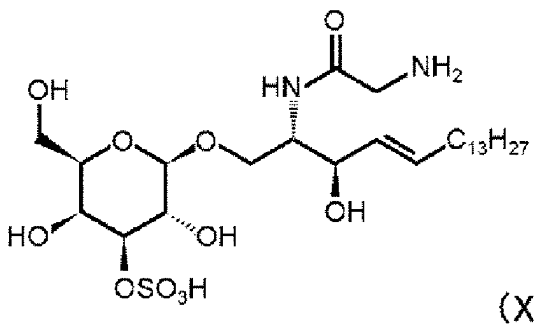

(X)

13. Procédé selon la revendication 12, dans lequel la chromatographie liquide est une chromatographie en phase inverse.

14. Procédé selon la revendication 12 ou 13, dans lequel le liquide céphalorachidien isolé est obtenu auprès d'un patient atteint d'une maladie dans laquelle le lyso-sulfatide et/ou le sulfatide s'accumule(nt) dans le corps, éventuellement dans lequel la maladie est la leucodystrophie métachromatique.

15. Procédé selon la revendication 14, dans lequel le liquide céphalorachidien isolé est obtenu auprès d'un patient ayant préalablement reçu un traitement visant à réduire le lyso-sulfatide et/ou le sulfatide présent(s) dans le corps.

[FIG. 1]

FIG. 1

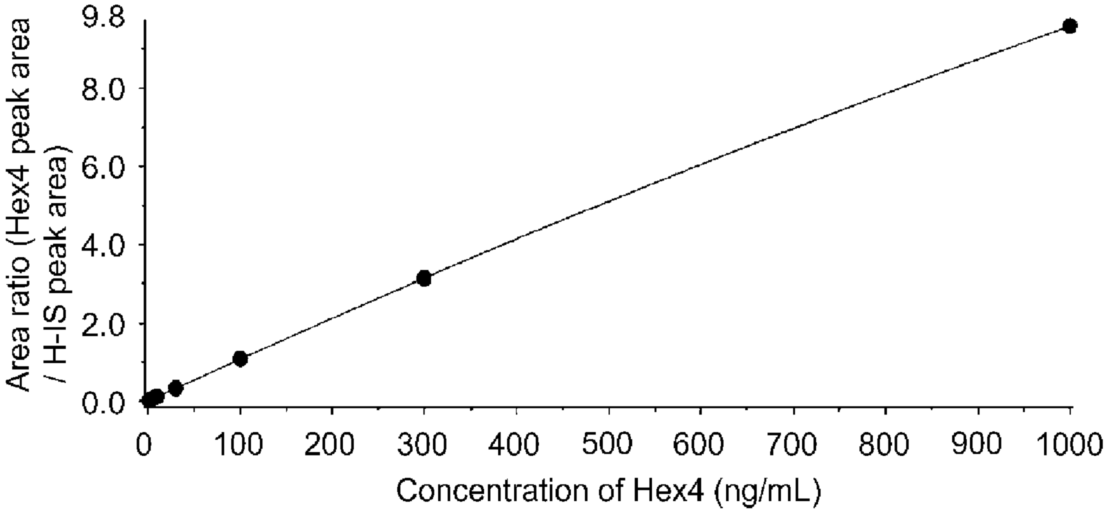
9.8
8.0
6.0
4.0
2.0
0.0
Area ratio (Hex4 peak area / H-IS peak area)
0
100
200
300
400
500
600
700
800
900
1000
Concentration of Hex4 (ng/mL)

[FIG. 2]

FIG. 2

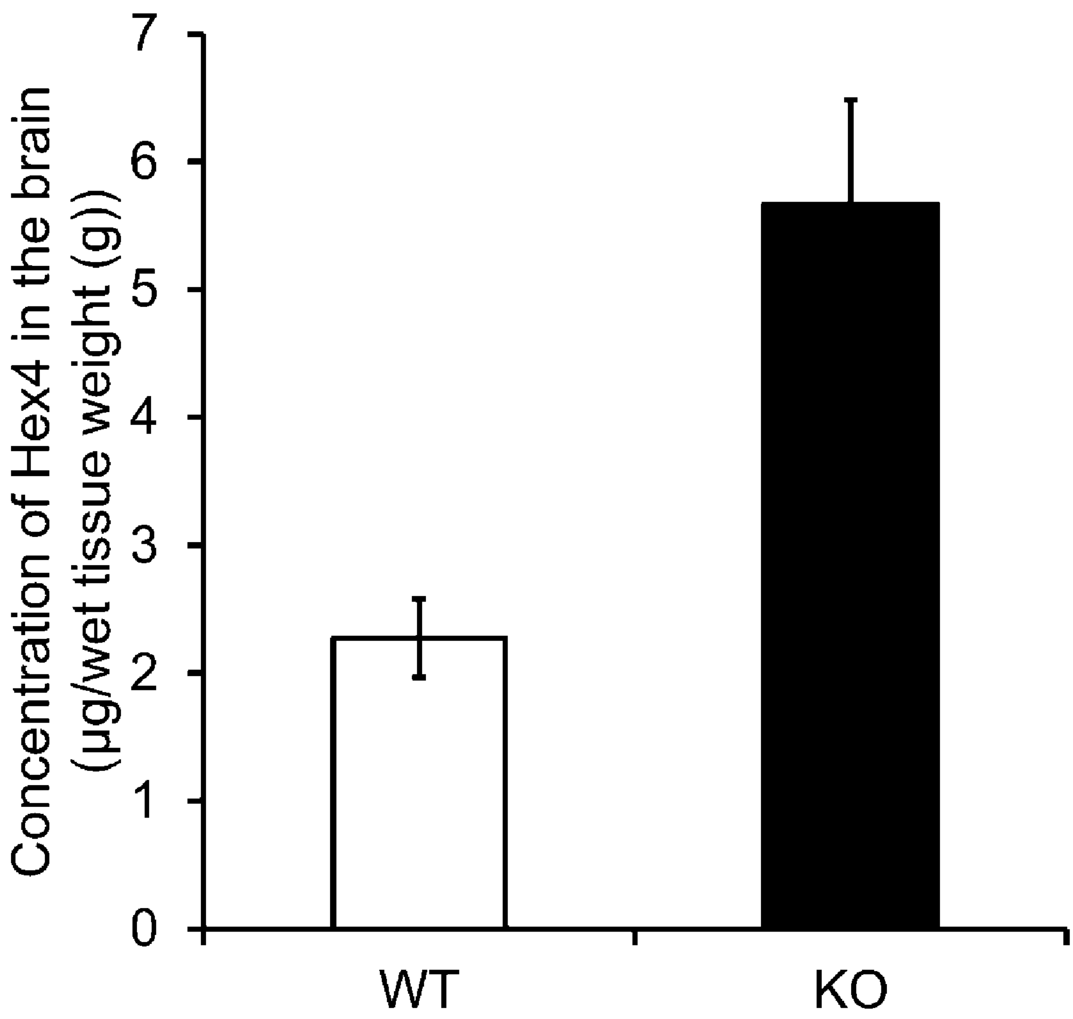
7
6
5
4
3
2
1
0
Concentration of Hex4 in the brain
(μg/wet tissue weight (g))
WT
KO

[FIG. 3]

FIG.3

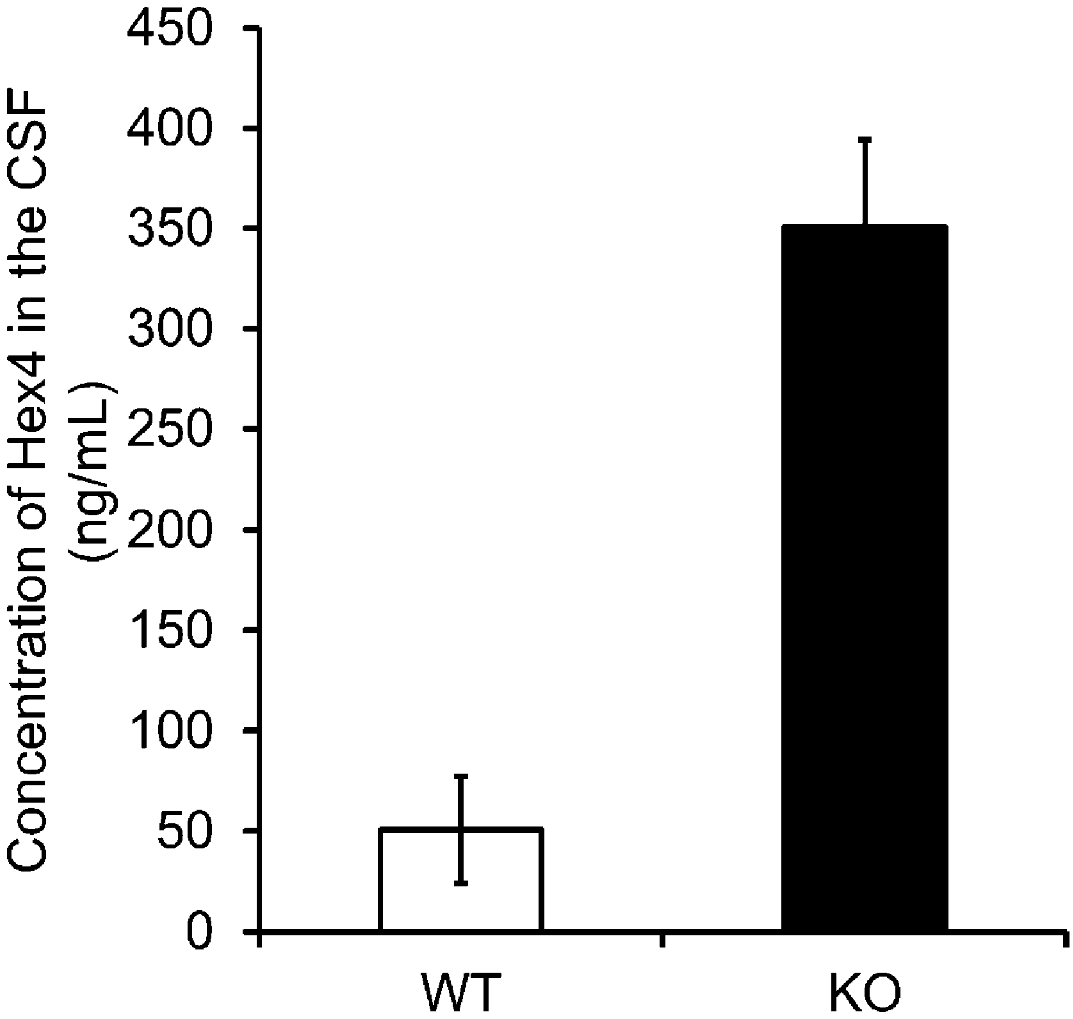
Concentration of Hex4 in the CSF (ng/mL)
450
400
350
300
250
200
150
100
50
0
WT
KO

[FIG. 4]

FIG. 4

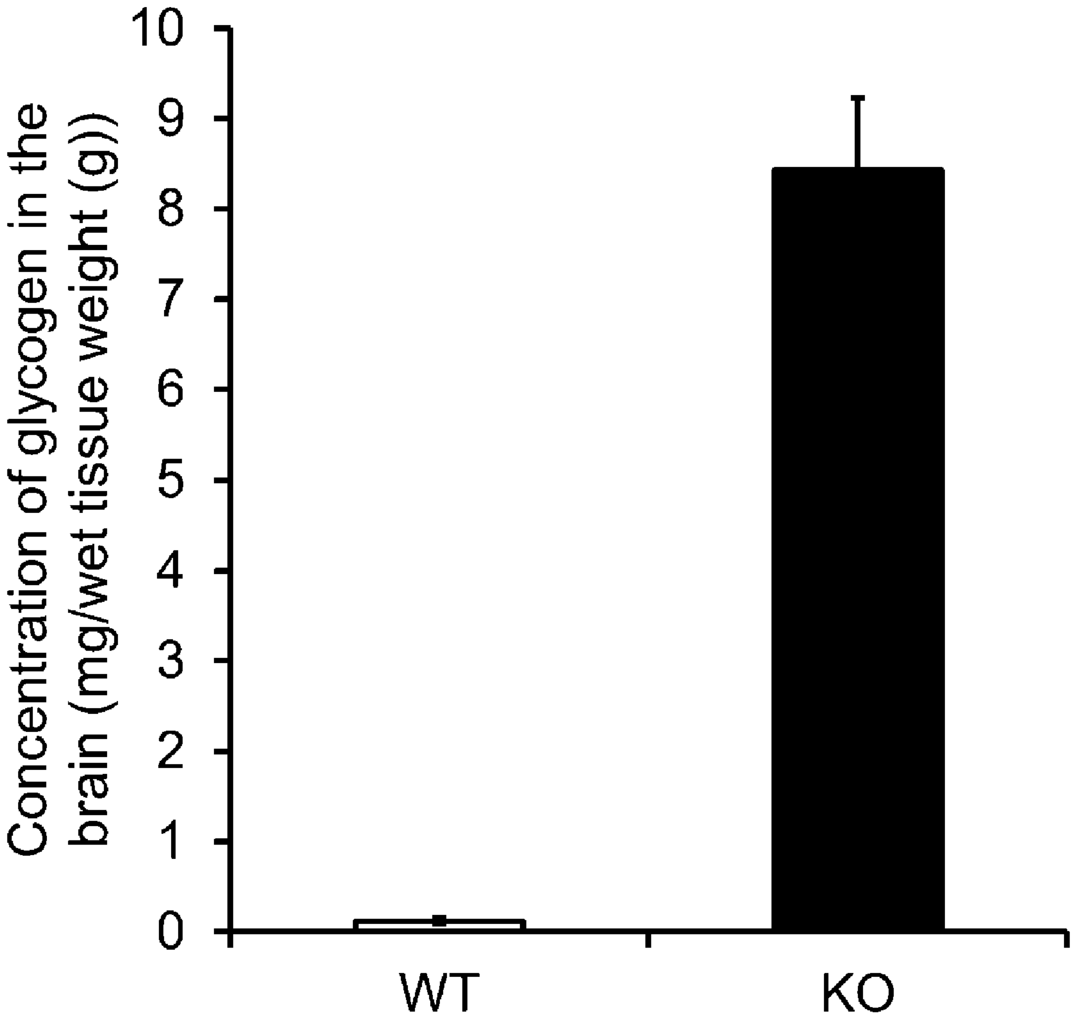
Concentration of glycogen in the brain (mg/wet tissue weight (g))
10
9
8
7
6
5
4
3
2
1
0
WT
KO

[FIG. 5]

FIG. 5

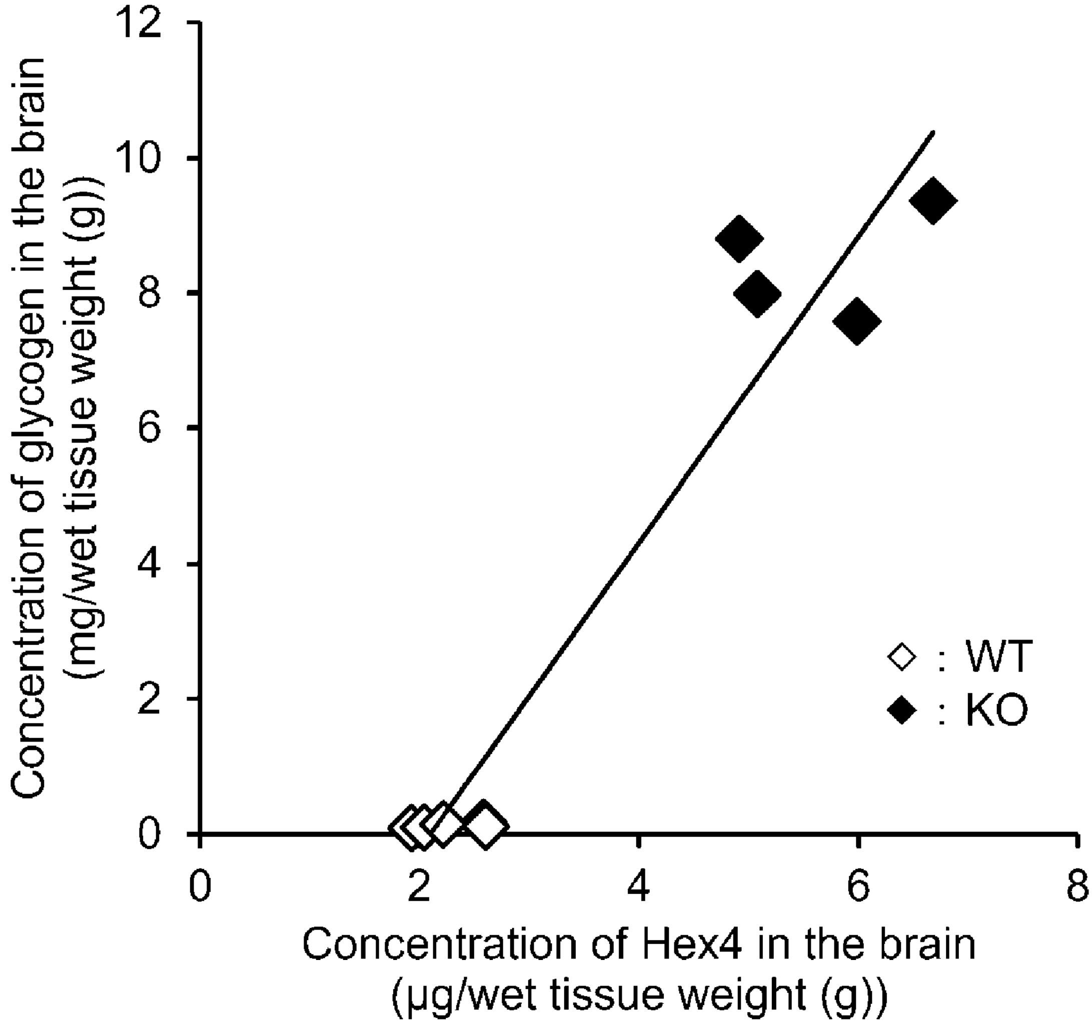

Concentration of glycogen in the brain (mg/wet tissue weight (g))
12
10
8
6
4
2
0
0
2
4
6
8
Concentration of Hex4 in the brain (µg/wet tissue weight (g))
◇ : WT
◆ : KO

[FIG. 6]

FIG. 6

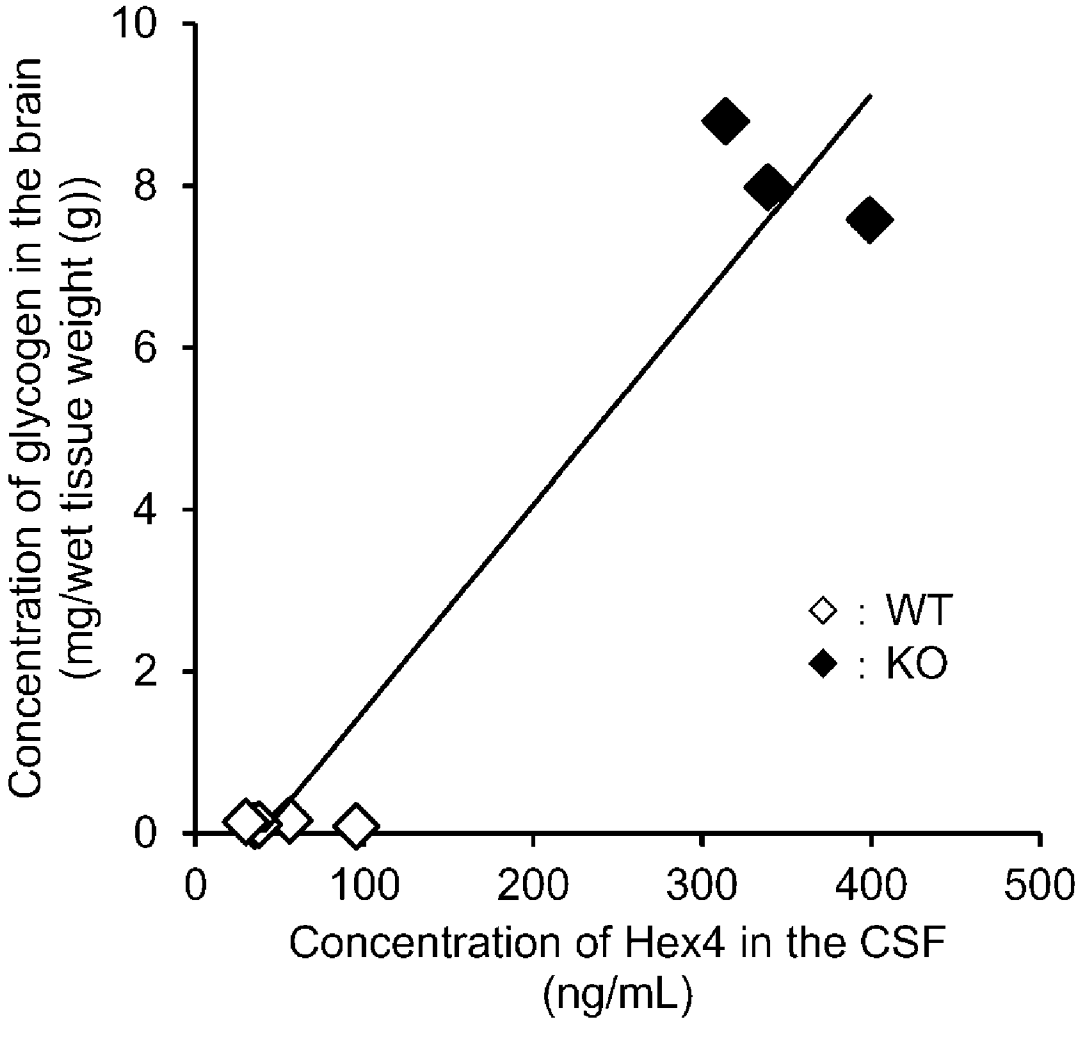

Concentration of glycogen in the brain (mg/wet tissue weight (g))
10
8
6
4
2
0
◇ : WT
◆ : KO
0
100
200
300
400
500
Concentration of Hex4 in the CSF (ng/mL)

[FIG. 7]

FIG. 7

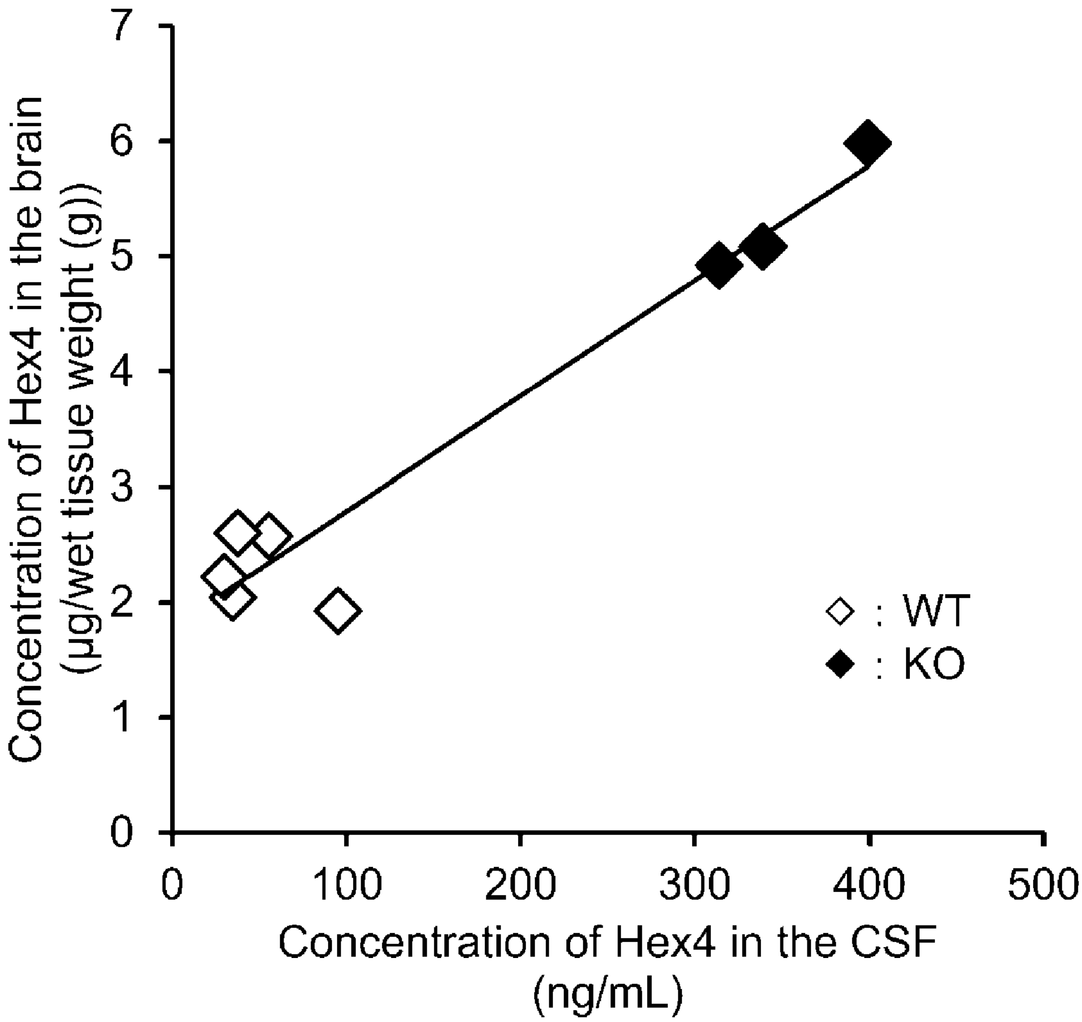
Concentration of Hex4 in the brain (µg/wet tissue weight (g))
0
1
2
3
4
5
6
7
0
100
200
300
400
500
Concentration of Hex4 in the CSF (ng/mL)
◇ : WT
◆ : KO

[FIG. 8]

FIG. 8

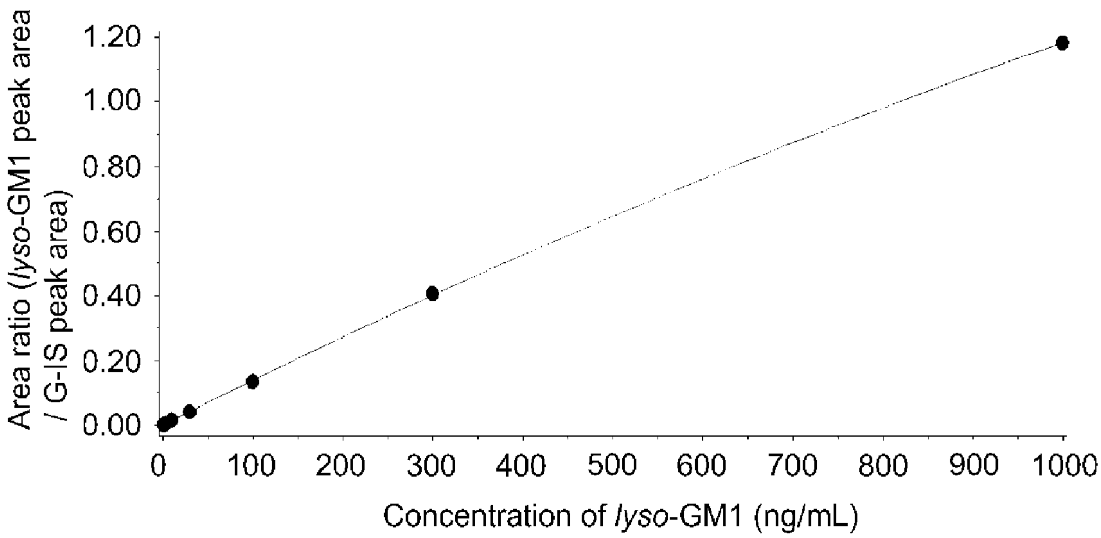
Area ratio (lyso-GM1 peak area / G-IS peak area)
1.20
1.00
0.80
0.60
0.40
0.20
0.00
0
100
200
300
400
500
600
700
800
900
1000
Concentration of lyso-GM1 (ng/mL)

[FIG. 9]

FIG. 9

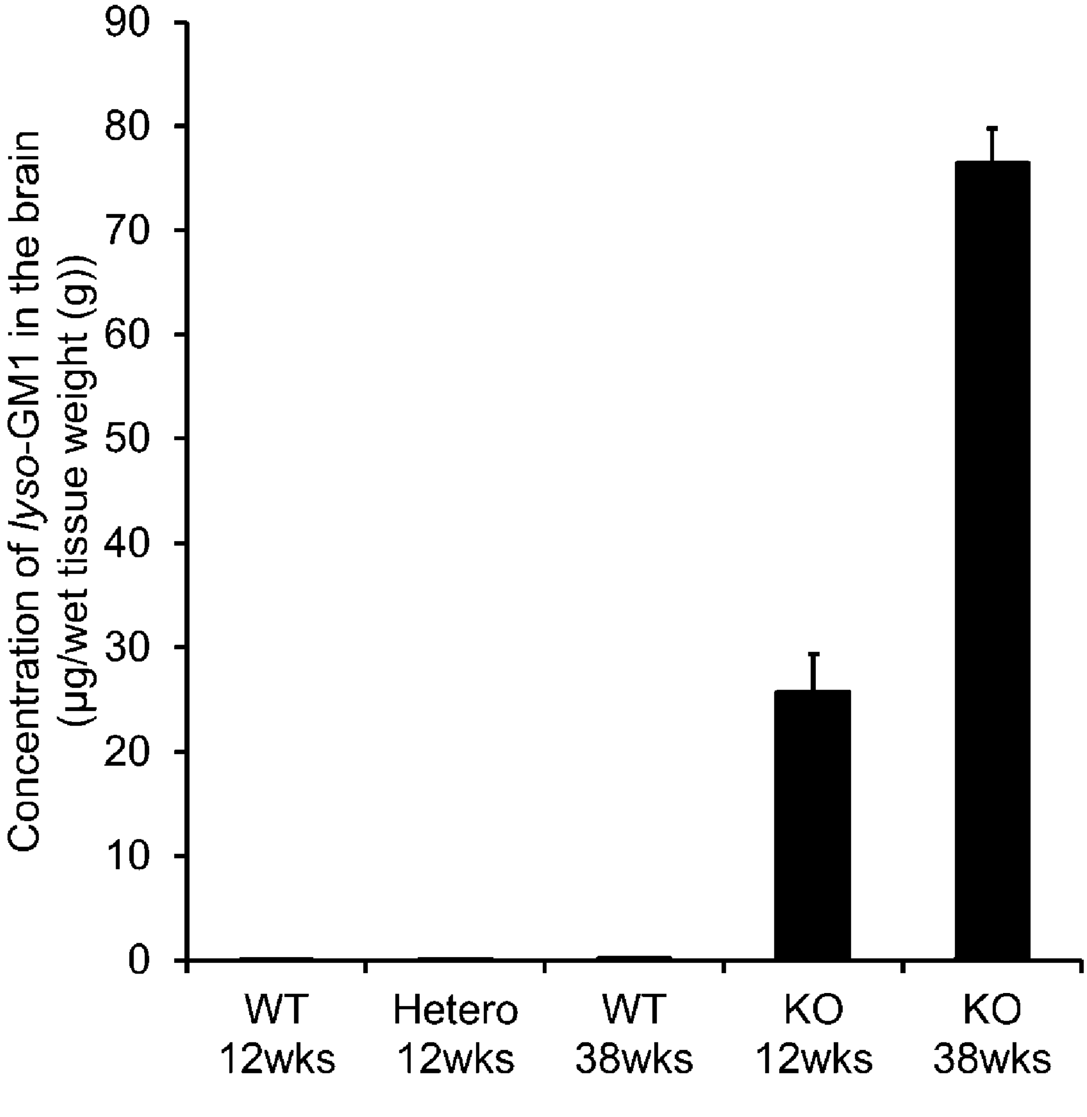
Concentration of *lyso*-GM1 in the brain
(μg/wet tissue weight (g))
90
80
70
60
50
40
30
20
10
0
WT
12wks
Hetero
12wks
WT
38wks
KO
12wks
KO
38wks

[FIG. 10]

FIG. 10

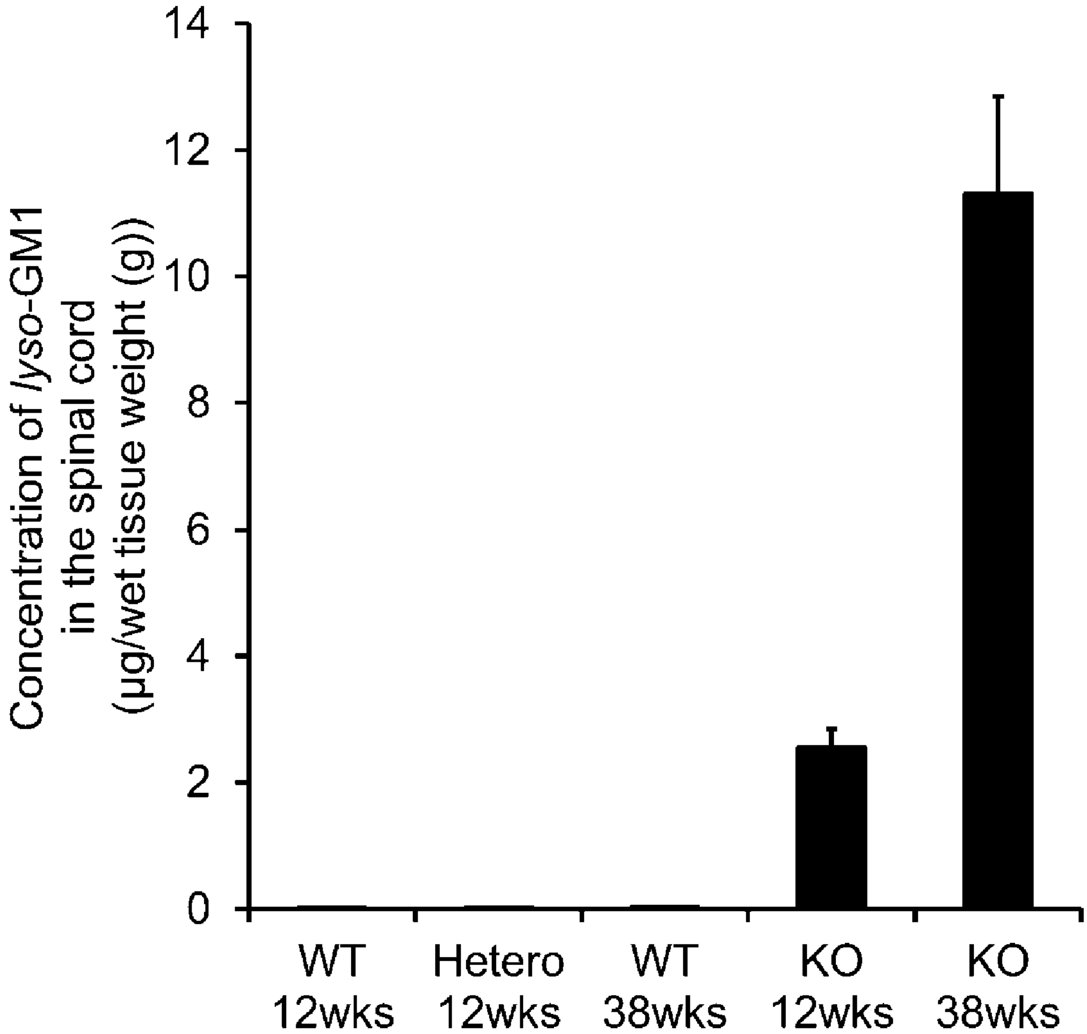
14
12
10
8
6
4
2
0
Concentration of lyso-GM1 in the spinal cord (μg/wet tissue weight (g))
WT 12wks
Hetero 12wks
WT 38wks
KO 12wks
KO 38wks

[FIG. 11]

FIG. 11

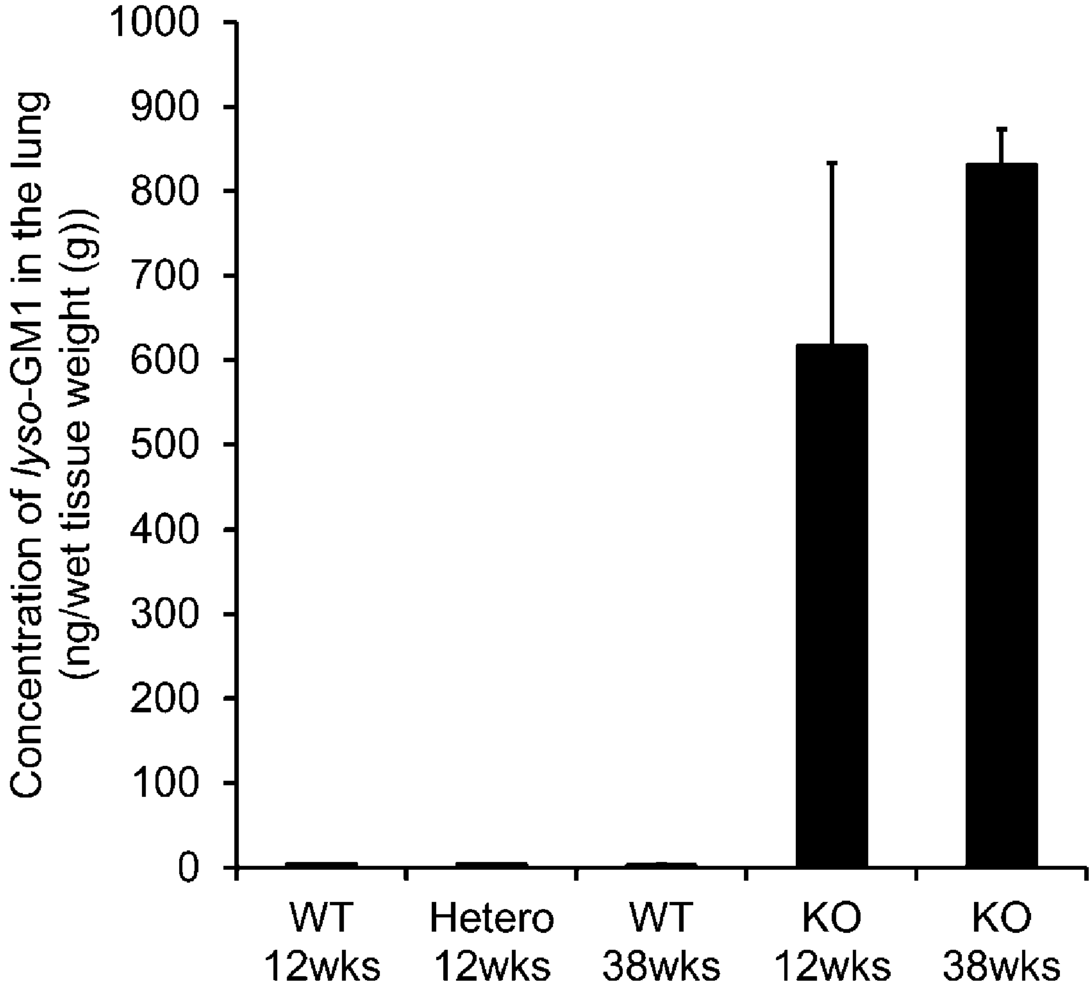
Concentration of lyso-GM1 in the lung (ng/wet tissue weight (g))
1000
900
800
700
600
500
400
300
200
100
0
WT 12wks
Hetero 12wks
WT 38wks
KO 12wks
KO 38wks

[FIG. 12]

FIG. 12

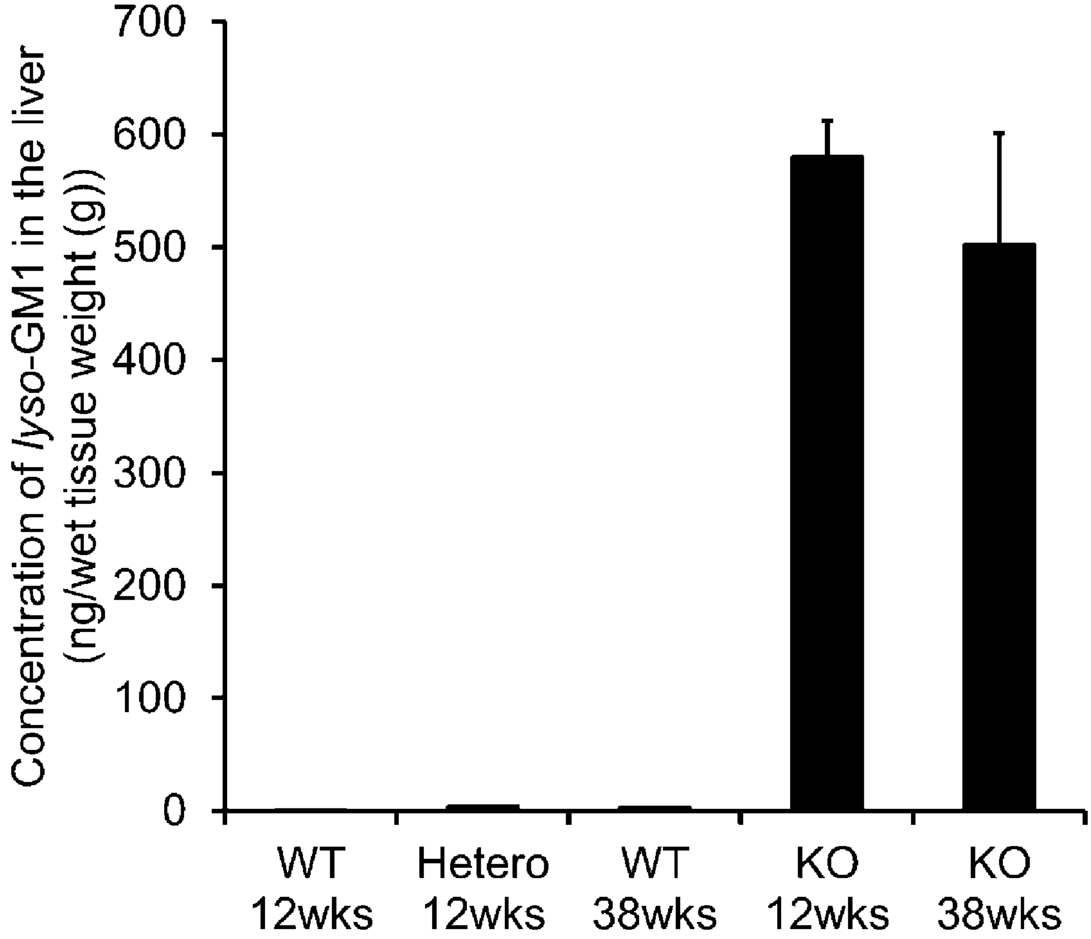
Concentration of lyso-GM1 in the liver
(ng/wet tissue weight (g))
700
600
500
400
300
200
100
0
WT 12wks
Hetero 12wks
WT 38wks
KO 12wks
KO 38wks

[FIG. 13]

FIG. 13

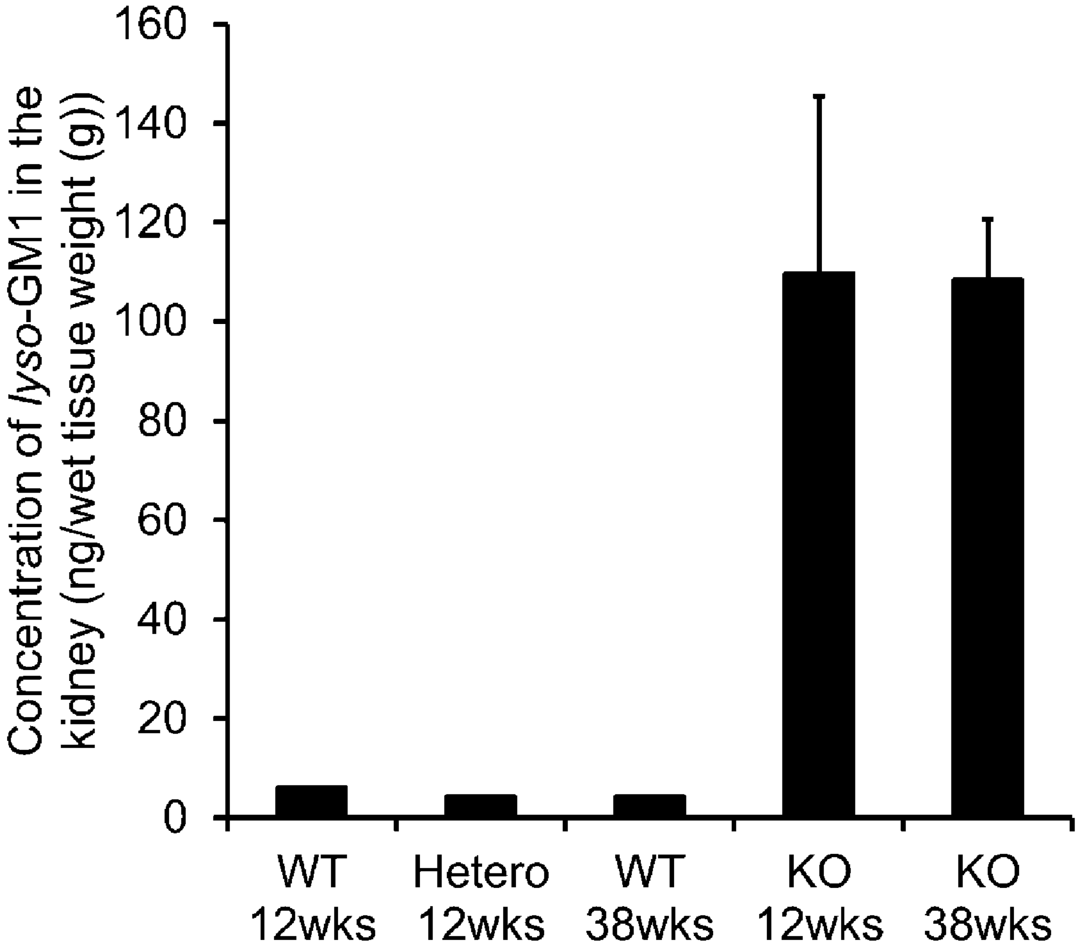
Concentration of lyso-GM1 in the kidney (ng/wet tissue weight (g))
160
140
120
100
80
60
40
20
0
WT 12wks
Hetero 12wks
WT 38wks
KO 12wks
KO 38wks

[FIG. 14]

FIG. 14

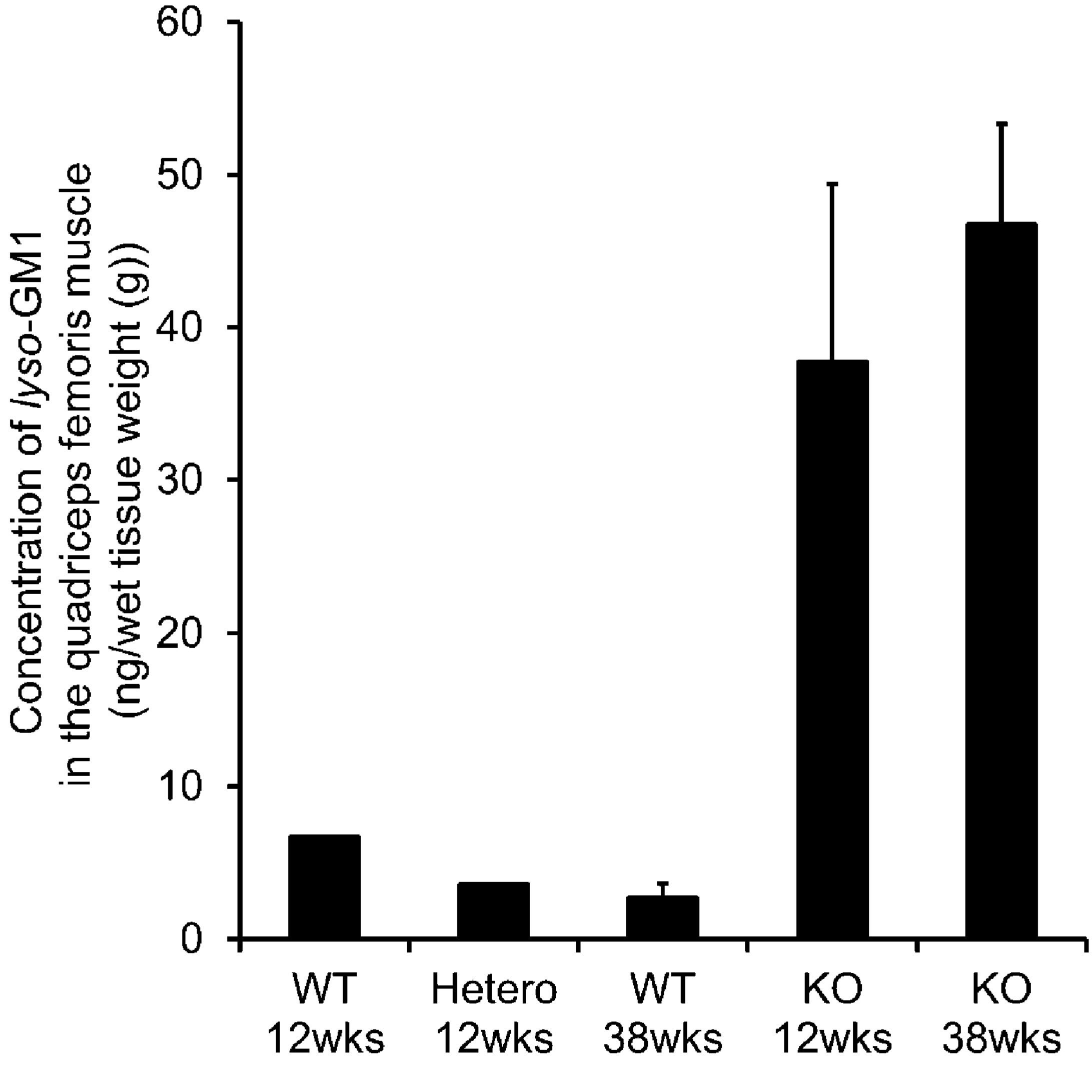
Concentration of lyso-GM1
in the quadriceps femoris muscle
(ng/wet tissue weight (g))
60
50
40
30
20
10
0
WT
12wks
Hetero
12wks
WT
38wks
KO
12wks
KO
38wks

[FIG. 15]

FIG. 15

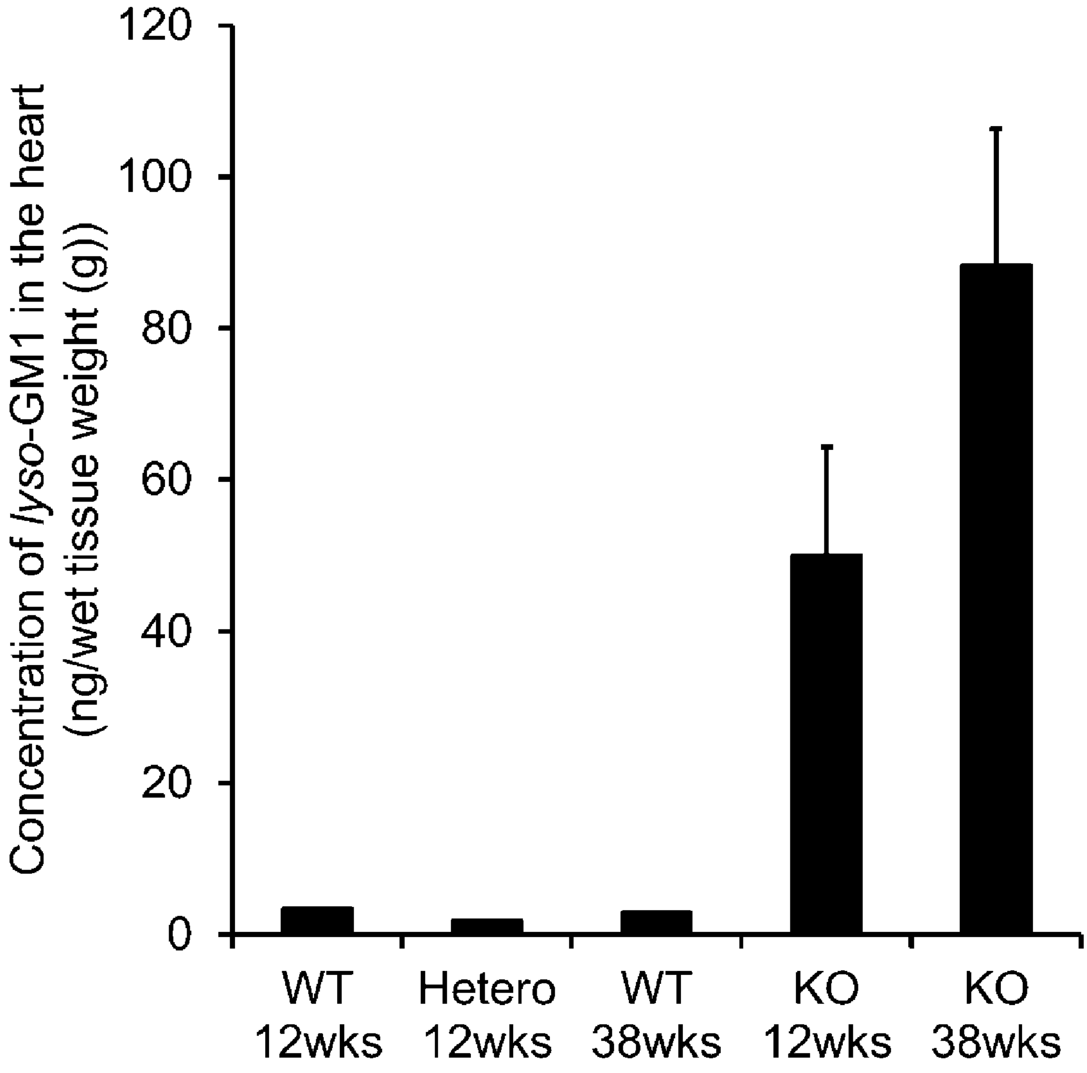
Concentration of lyso-GM1 in the heart
(ng/wet tissue weight (g))
120
100
80
60
40
20
0
WT
12wks
Hetero
12wks
WT
38wks
KO
12wks
KO
38wks

[FIG. 16]

FIG. 16

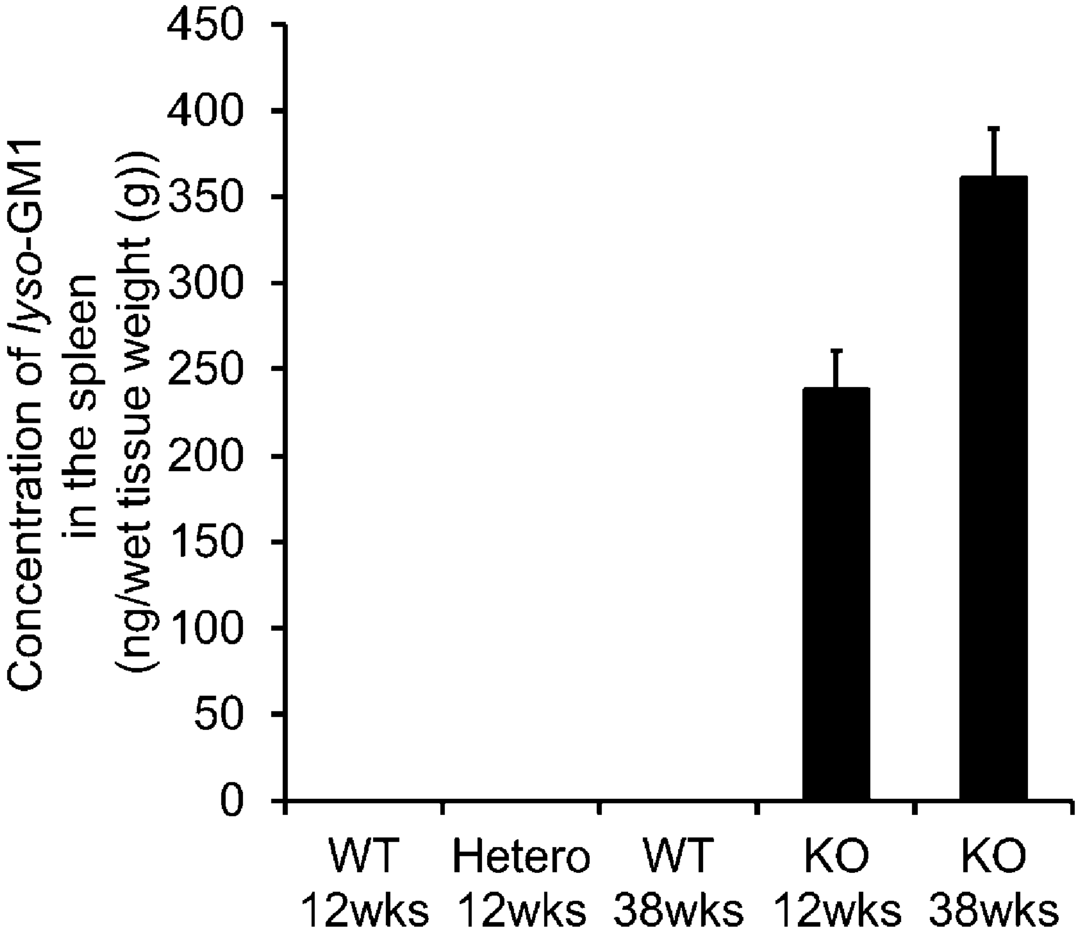
Concentration of lyso-GM1 in the spleen (ng/wet tissue weight (g))
450
400
350
300
250
200
150
100
50
0
WT 12wks
Hetero 12wks
WT 38wks
KO 12wks
KO 38wks

[FIG. 17]

FIG. 17

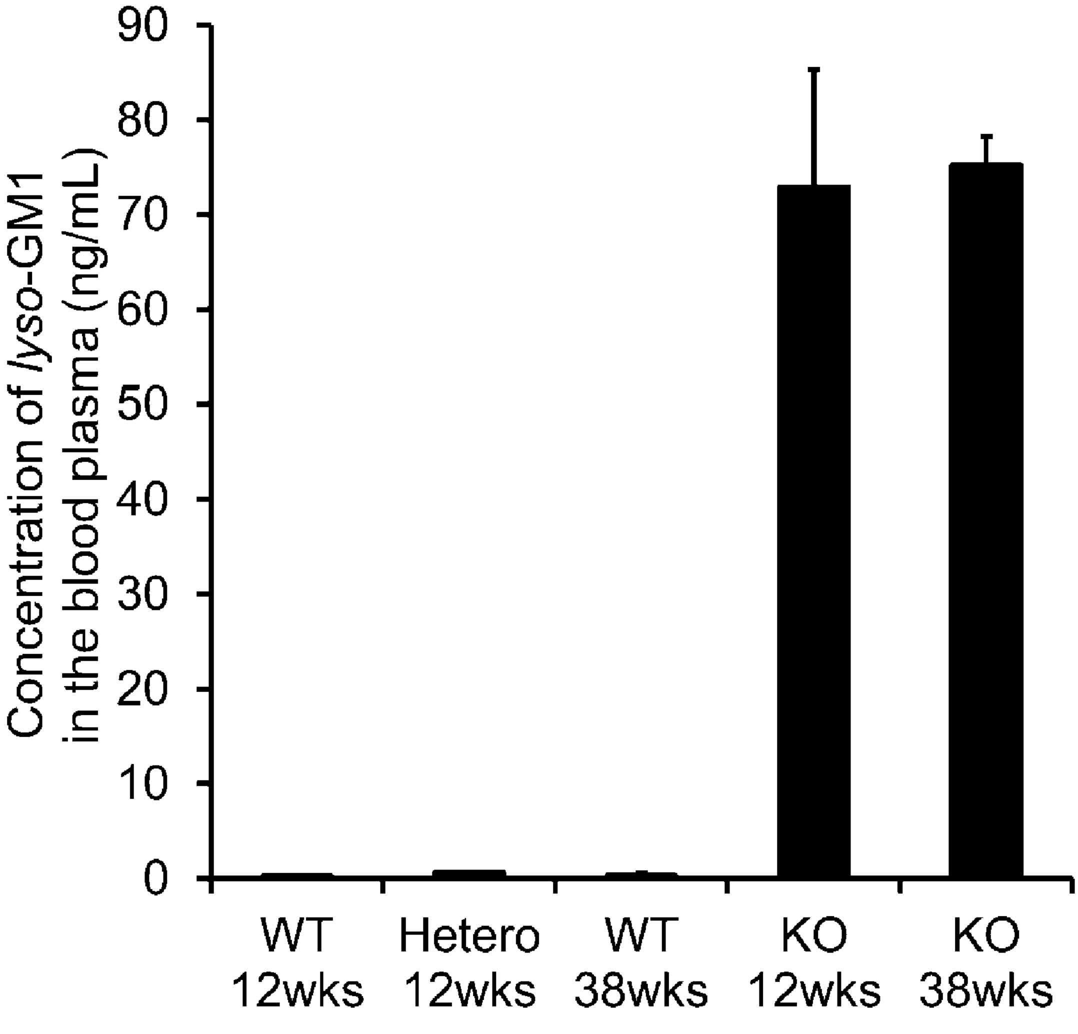
90
80
70
60
50
40
30
20
10
0
Concentration of *lyso*-GM1 in the blood plasma (ng/mL)
WT 12wks
Hetero 12wks
WT 38wks
KO 12wks
KO 38wks

[FIG. 18]

FIG. 18

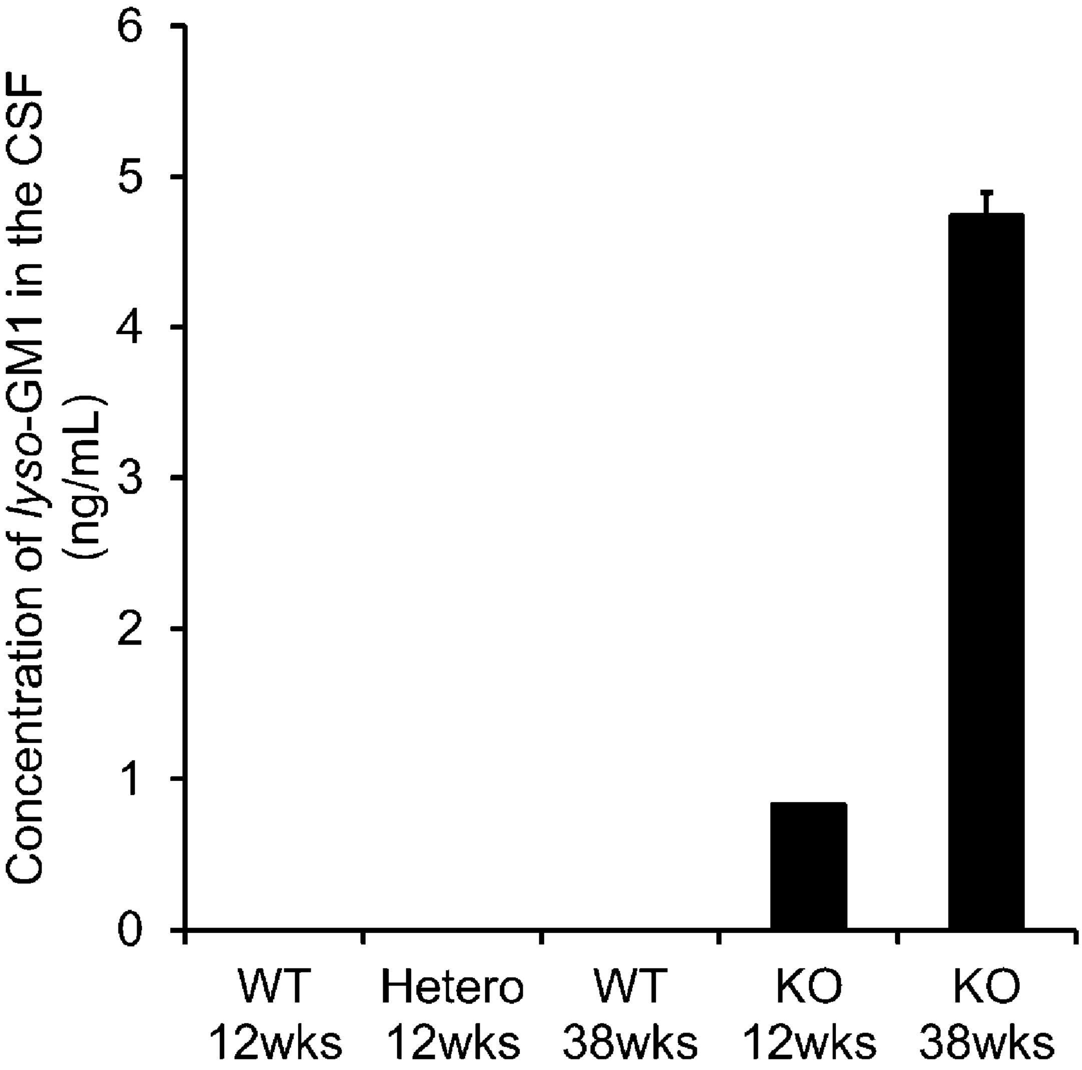
6
5
4
3
2
1
0
Concentration of lyso-GM1 in the CSF (ng/mL)
WT 12wks
Hetero 12wks
WT 38wks
KO 12wks
KO 38wks

[FIG. 19]

FIG. 19

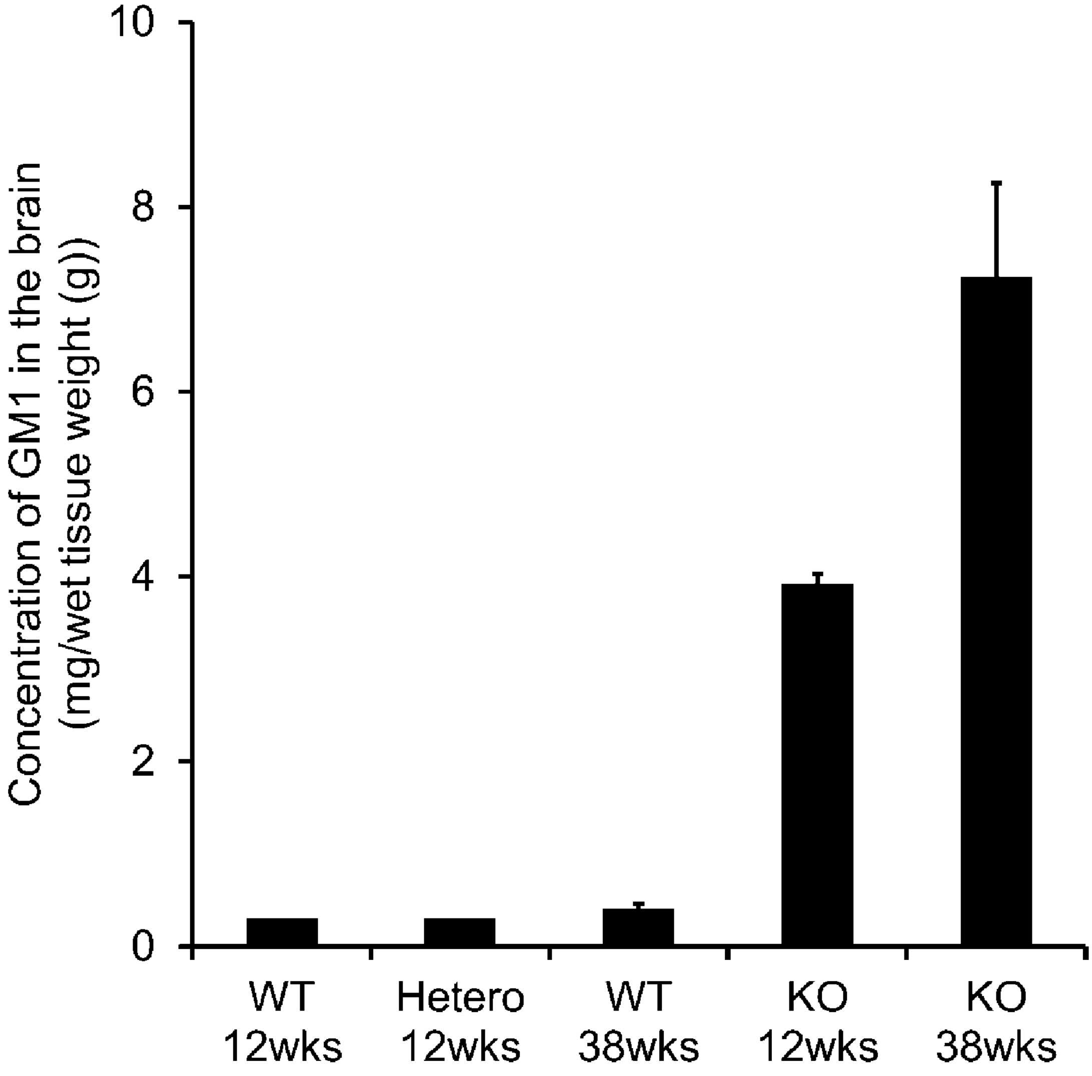
10
8
6
4
2
0
Concentration of GM1 in the brain
(mg/wet tissue weight (g))
WT
12wks
Hetero
12wks
WT
38wks
KO
12wks
KO
38wks

[FIG. 20]

FIG. 20

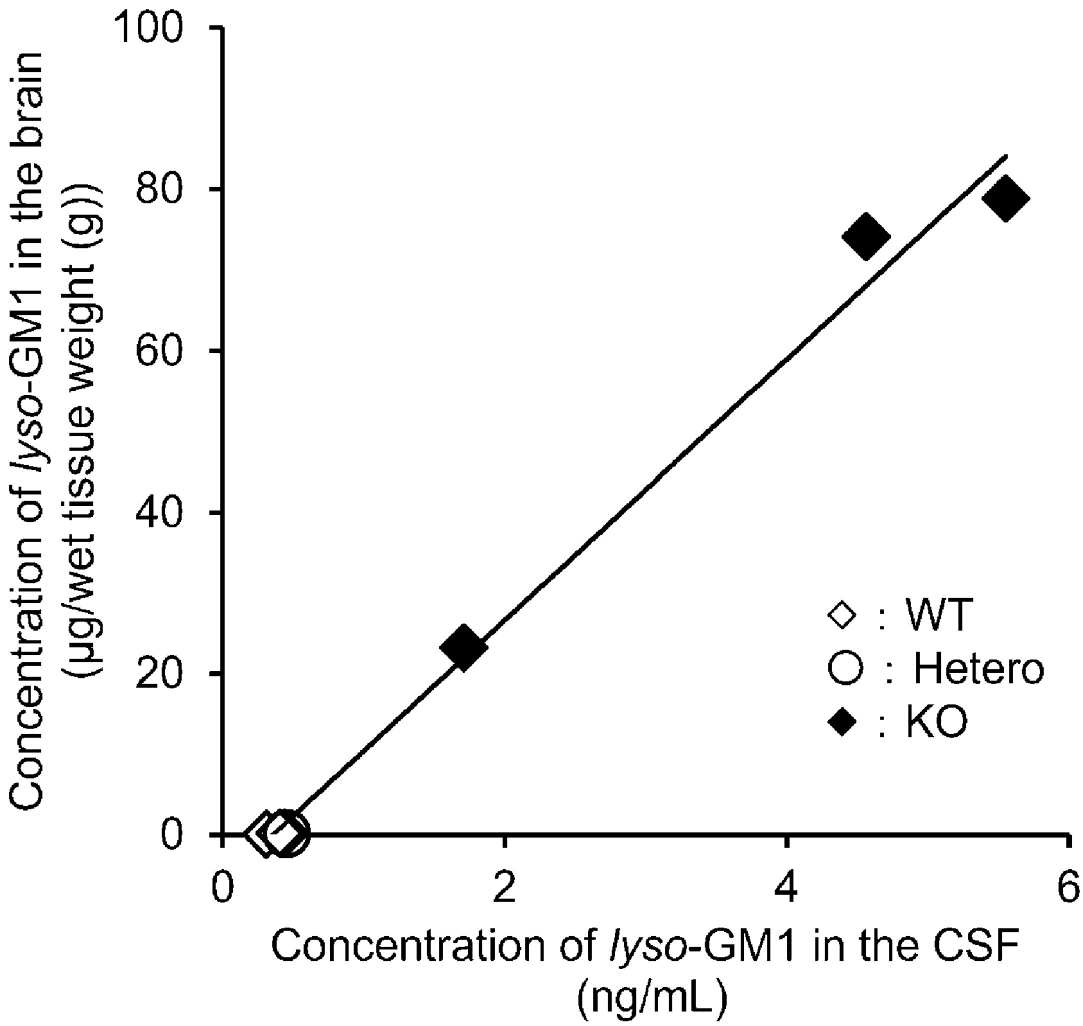
Concentration of *lyso*-GM1 in the brain (µg/wet tissue weight (g))
100
80
60
40
20
0
0
2
4
6
Concentration of *lyso*-GM1 in the CSF (ng/mL)
◇ : WT
○ : Hetero
◆ : KO

[FIG. 21]

FIG. 21

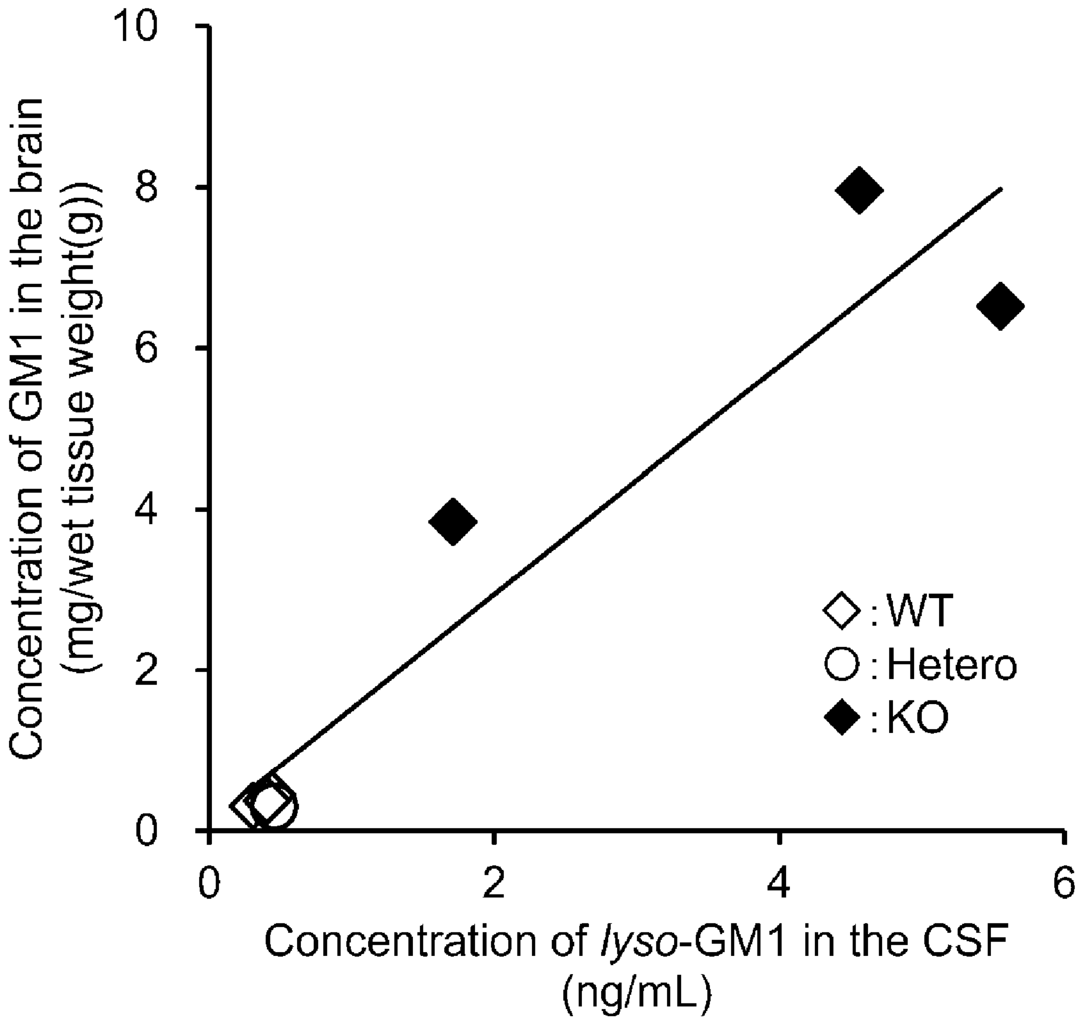
10
8
6
4
2
0
Concentration of GM1 in the brain
(mg/wet tissue weight(g))
0
2
4
6
Concentration of lyso-GM1 in the CSF
(ng/mL)
◇: WT
○: Hetero
◆: KO

[FIG. 22]

FIG. 22

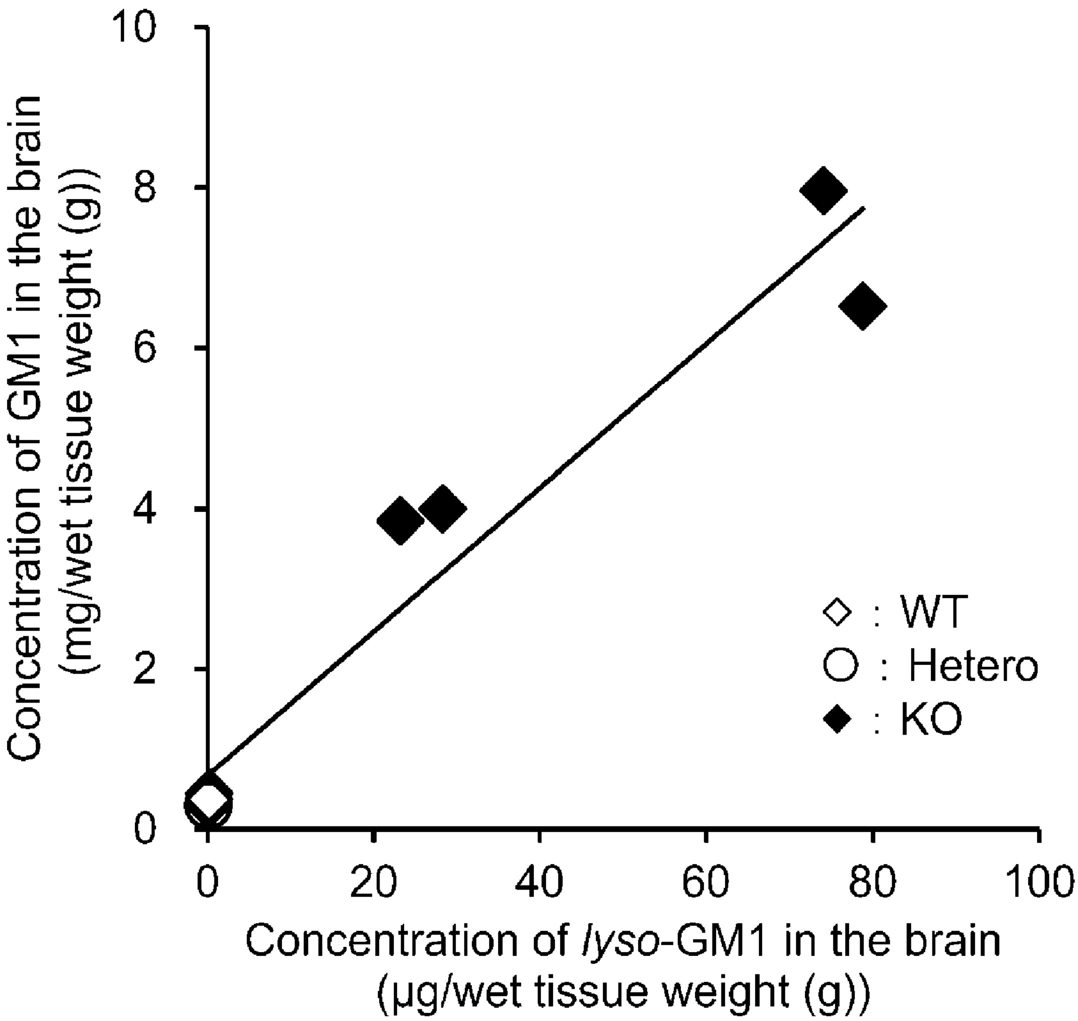
10
8
6
4
2
0
Concentration of GM1 in the brain
(mg/wet tissue weight (g))
0
20
40
60
80
100
Concentration of *lyso*-GM1 in the brain
(µg/wet tissue weight (g))
◇ : WT
○ : Hetero
◆ : KO

[FIG. 23]

FIG. 23

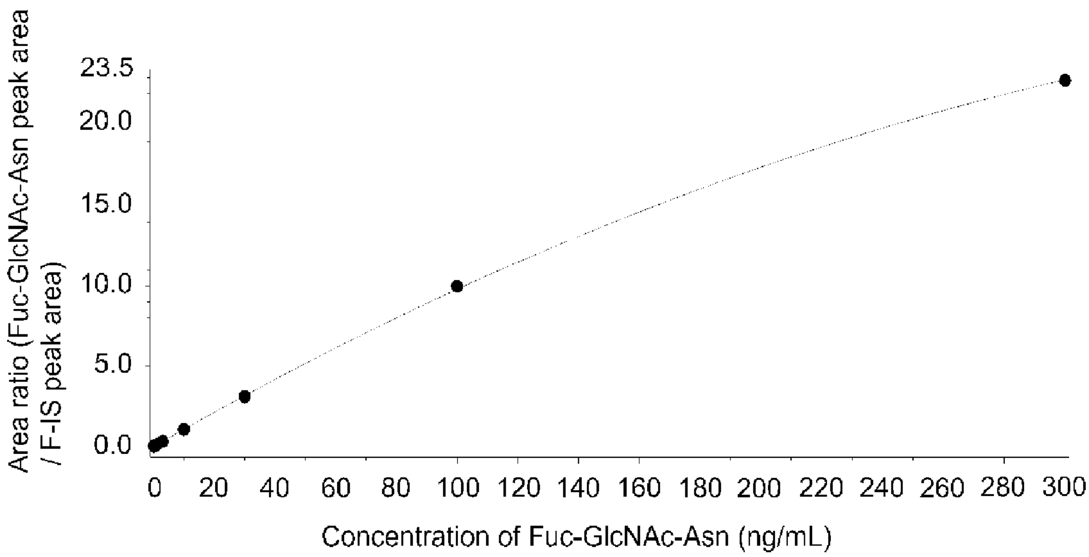
Area ratio (Fuc-GlcNAc-Asn peak area / F-IS peak area)
23.5
20.0
15.0
10.0
5.0
0.0
0 20 40 60 80 100 120 140 160 180 200 220 240 260 280 300
Concentration of Fuc-GlcNAc-Asn (ng/mL)

[FIG. 24]

FIG. 24

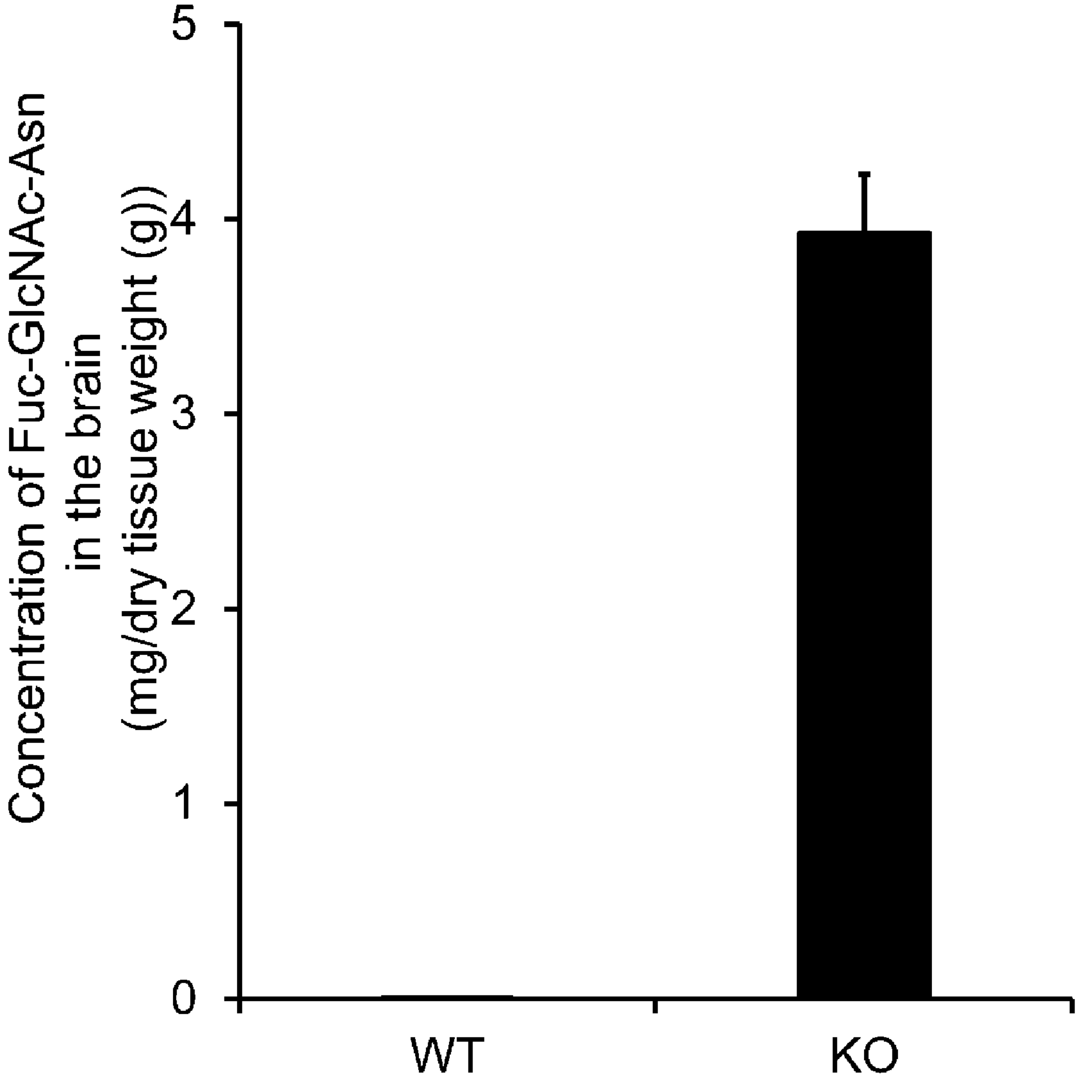
Concentration of Fuc-GlcNAc-Asn
in the brain
(mg/dry tissue weight (g))
5
4
3
2
1
0
WT
KO

[FIG. 25]

FIG. 25

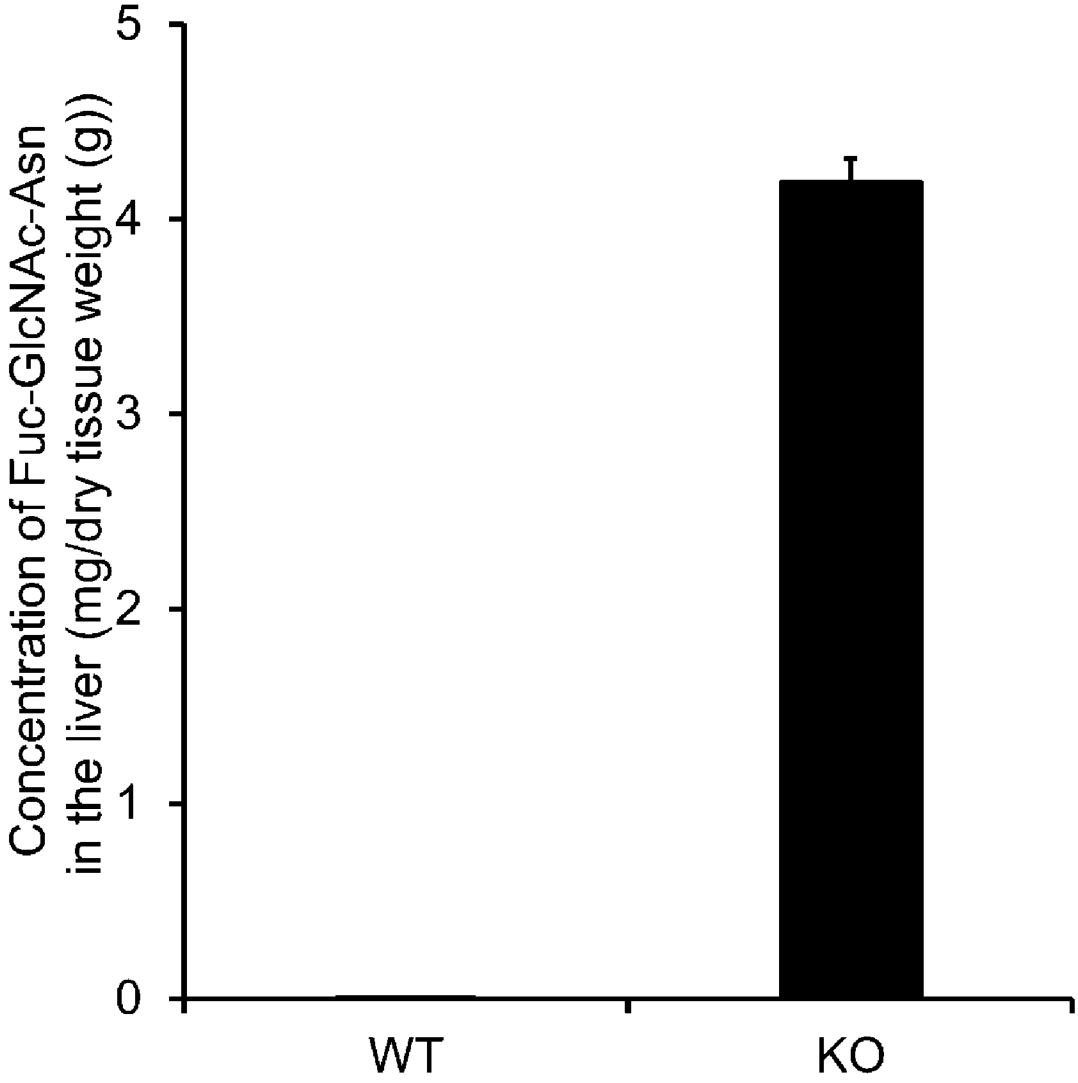
5
4
3
2
1
0
Concentration of Fuc-GlcNAc-Asn
in the liver (mg/dry tissue weight (g))
WT
KO

[FIG. 26]

FIG. 26

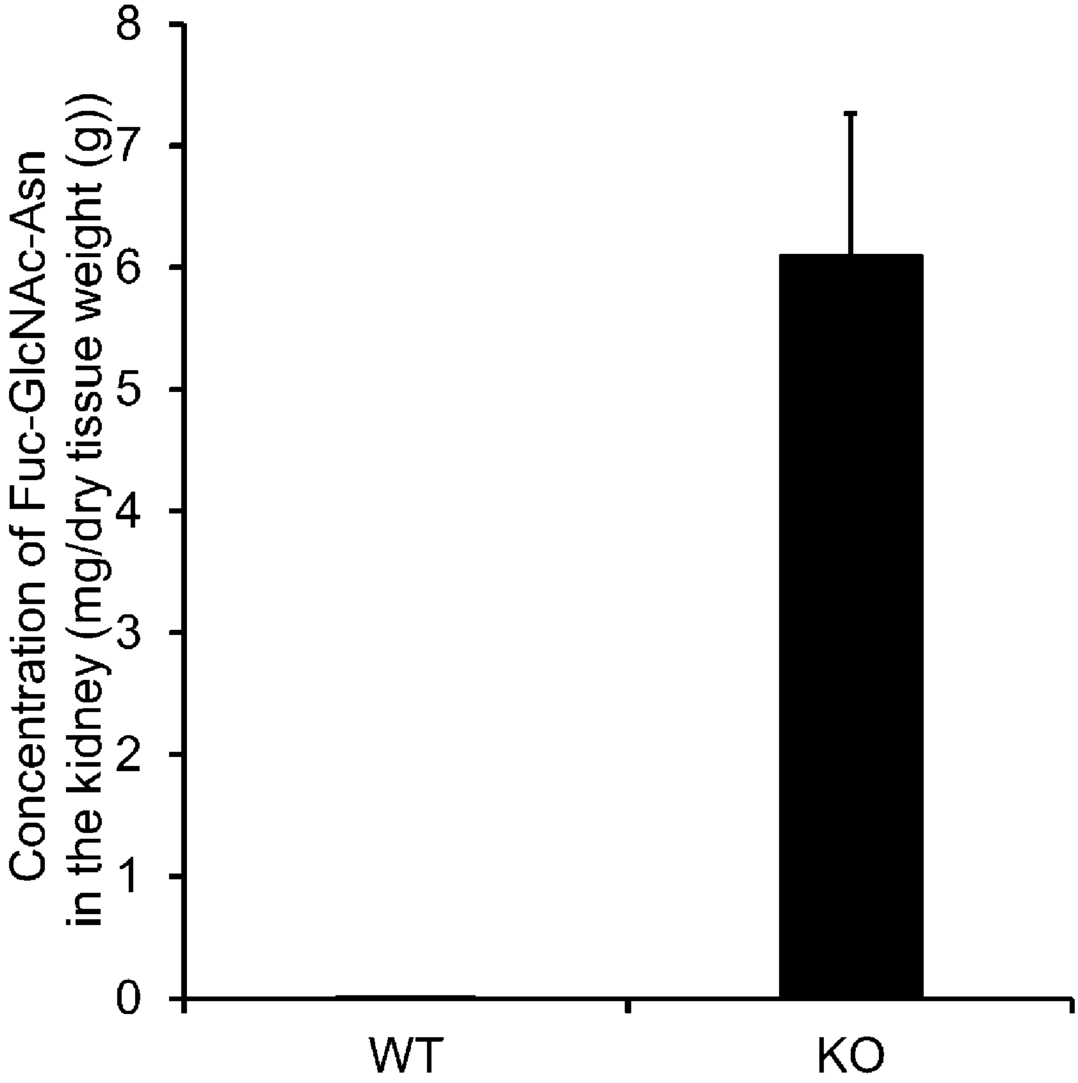
Concentration of Fuc-GlcNAc-Asn
in the kidney (mg/dry tissue weight (g))
8
7
6
5
4
3
2
1
0
WT
KO

[FIG. 27]

FIG. 27

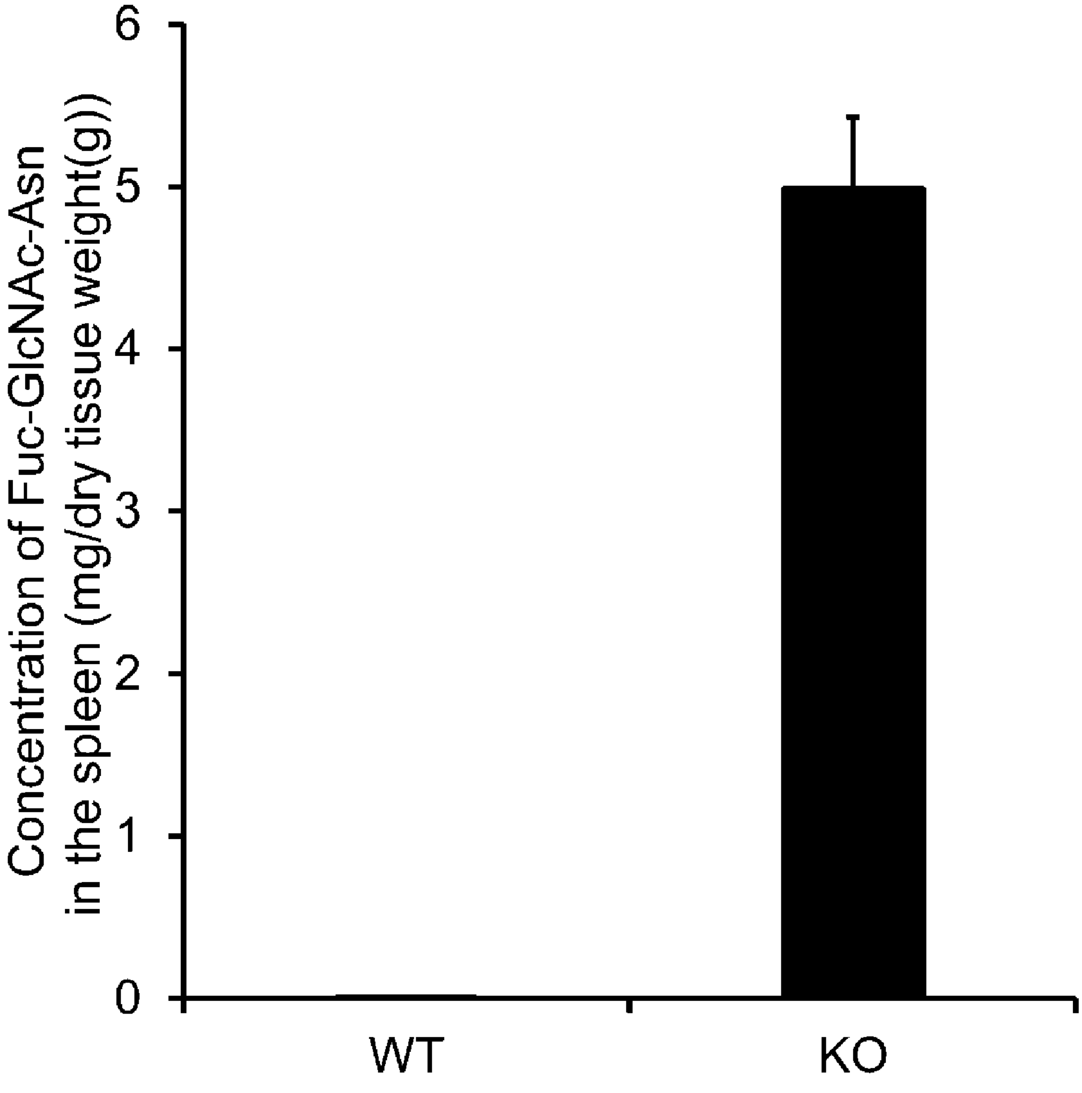
6
5
4
3
2
1
0
Concentration of Fuc-GlcNAc-Asn
in the spleen (mg/dry tissue weight(g))
WT
KO

[FIG. 28]

FIG. 28

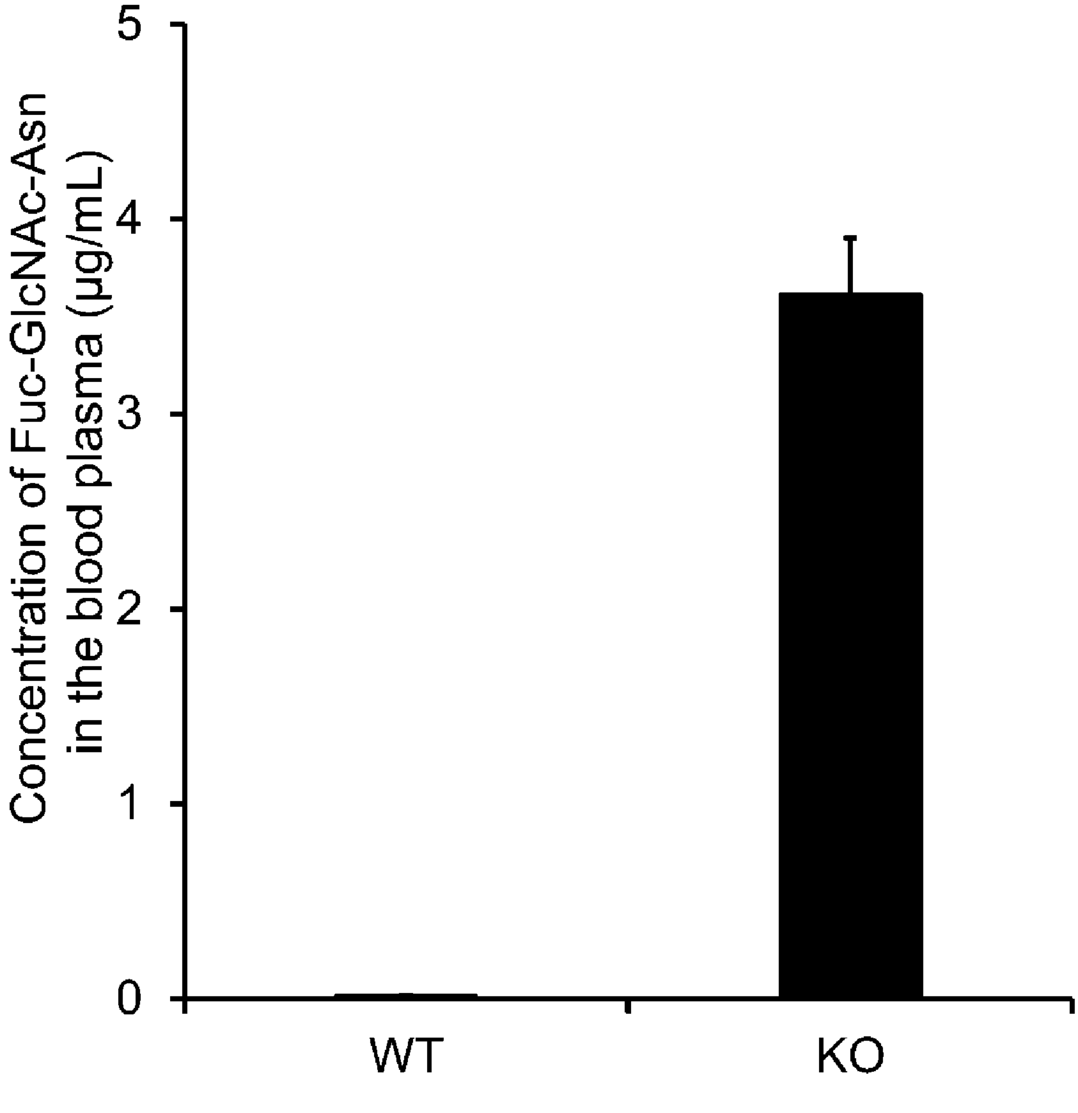
Concentration of Fuc-GlcNAc-Asn in the blood plasma (μg/mL)
0
1
2
3
4
5
WT
KO

[FIG. 29]

FIG. 29

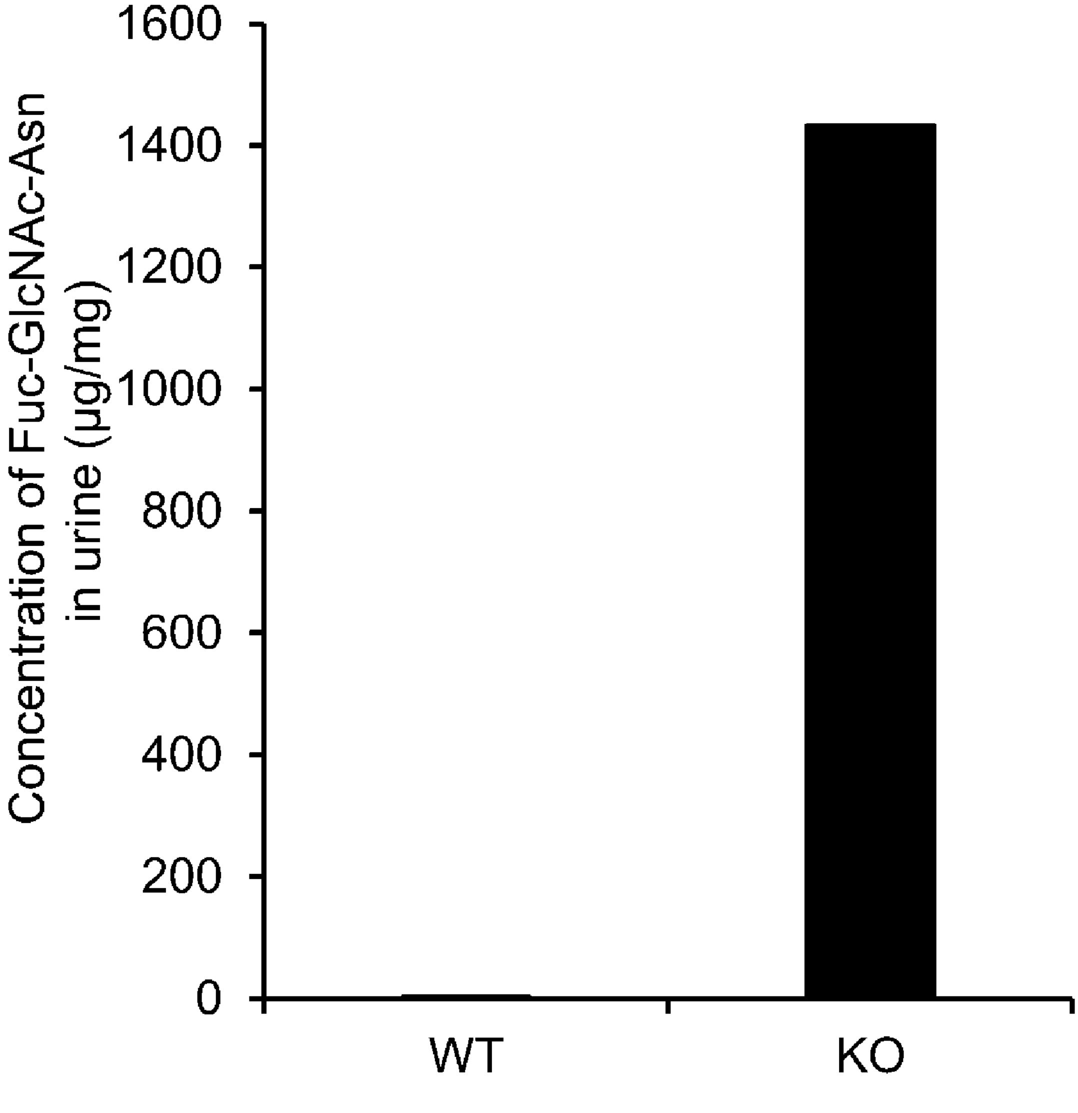
1600
1400
1200
1000
800
600
400
200
0
Concentration of Fuc-GlcNAc-Asn in urine (µg/mg)
WT
KO

[FIG. 30]

FIG. 30

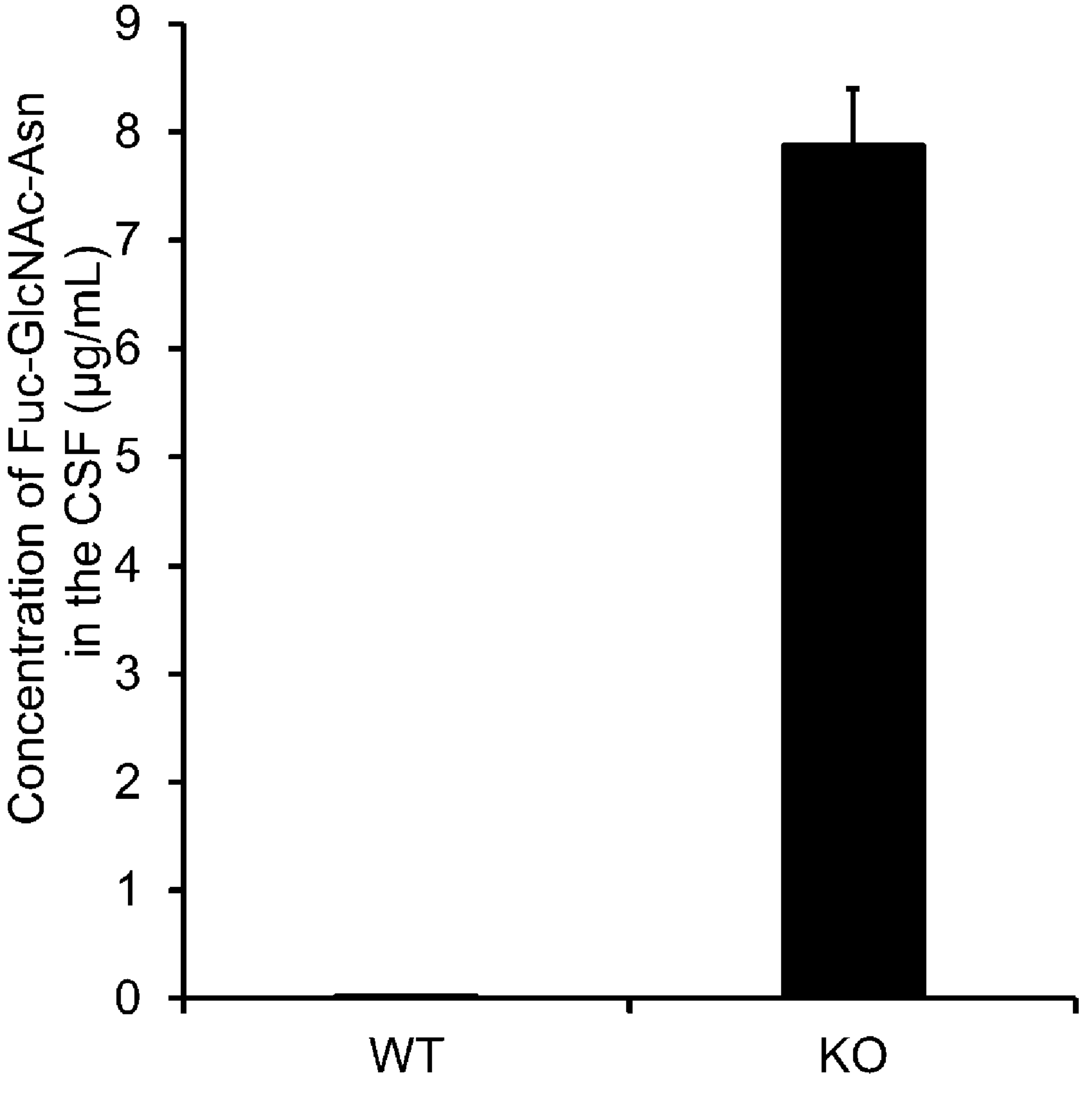
9
8
7
6
5
4
3
2
1
0
Concentration of Fuc-GlcNAc-Asn in the CSF (μg/mL)
WT
KO

[FIG. 31]

FIG. 31

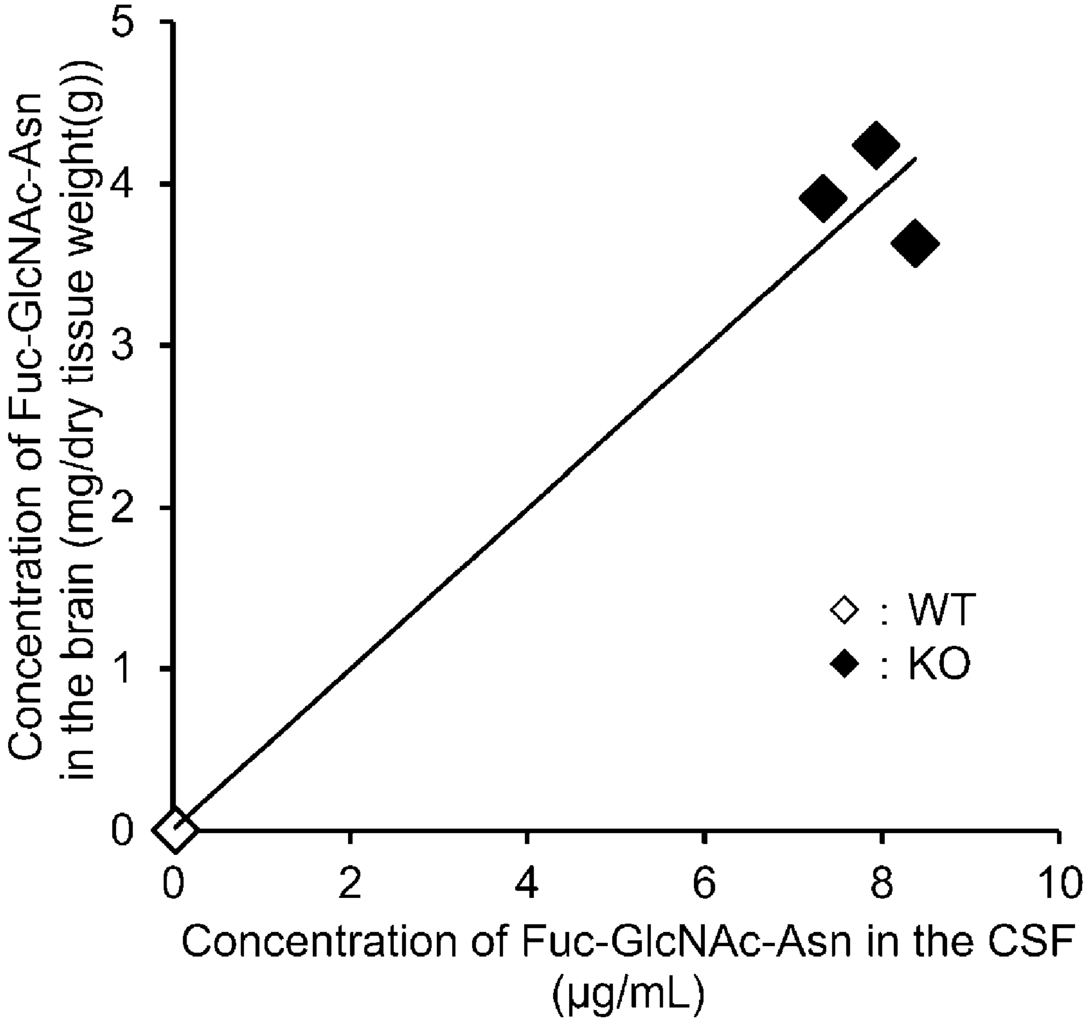
5
4
3
2
1
0
0
2
4
6
8
10
Concentration of Fuc-GlcNAc-Asn in the brain (mg/dry tissue weight(g))
◇ : WT
◆ : KO
Concentration of Fuc-GlcNAc-Asn in the CSF (μg/mL)

[FIG. 32]

FIG. 32

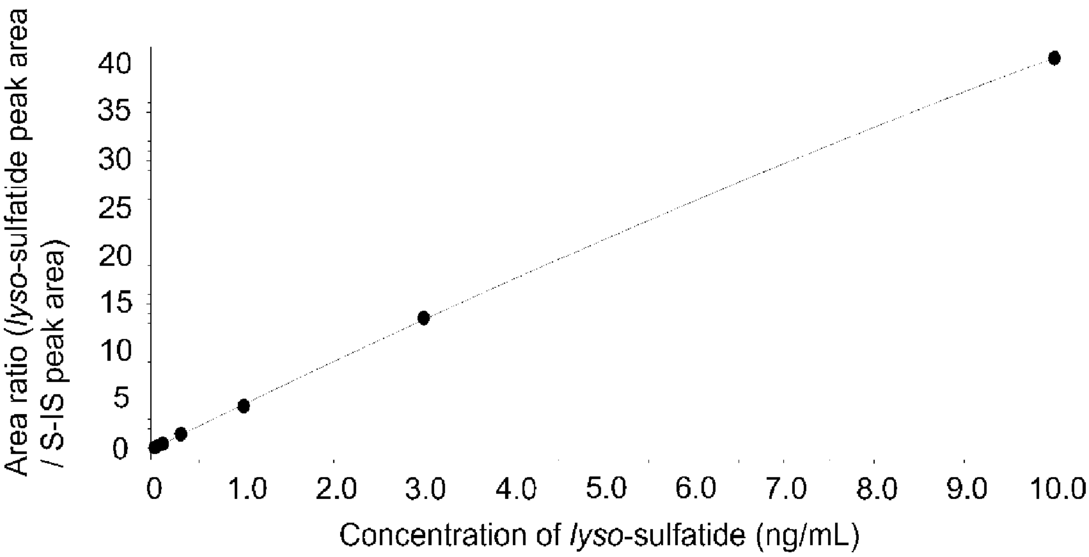
Area ratio (*lyso*-sulfatide peak area / S-IS peak area)
40
35
30
25
20
15
10
5
0
0
1.0
2.0
3.0
4.0
5.0
6.0
7.0
8.0
9.0
10.0
Concentration of *lyso*-sulfatide (ng/mL)

[FIG. 33]

FIG. 33

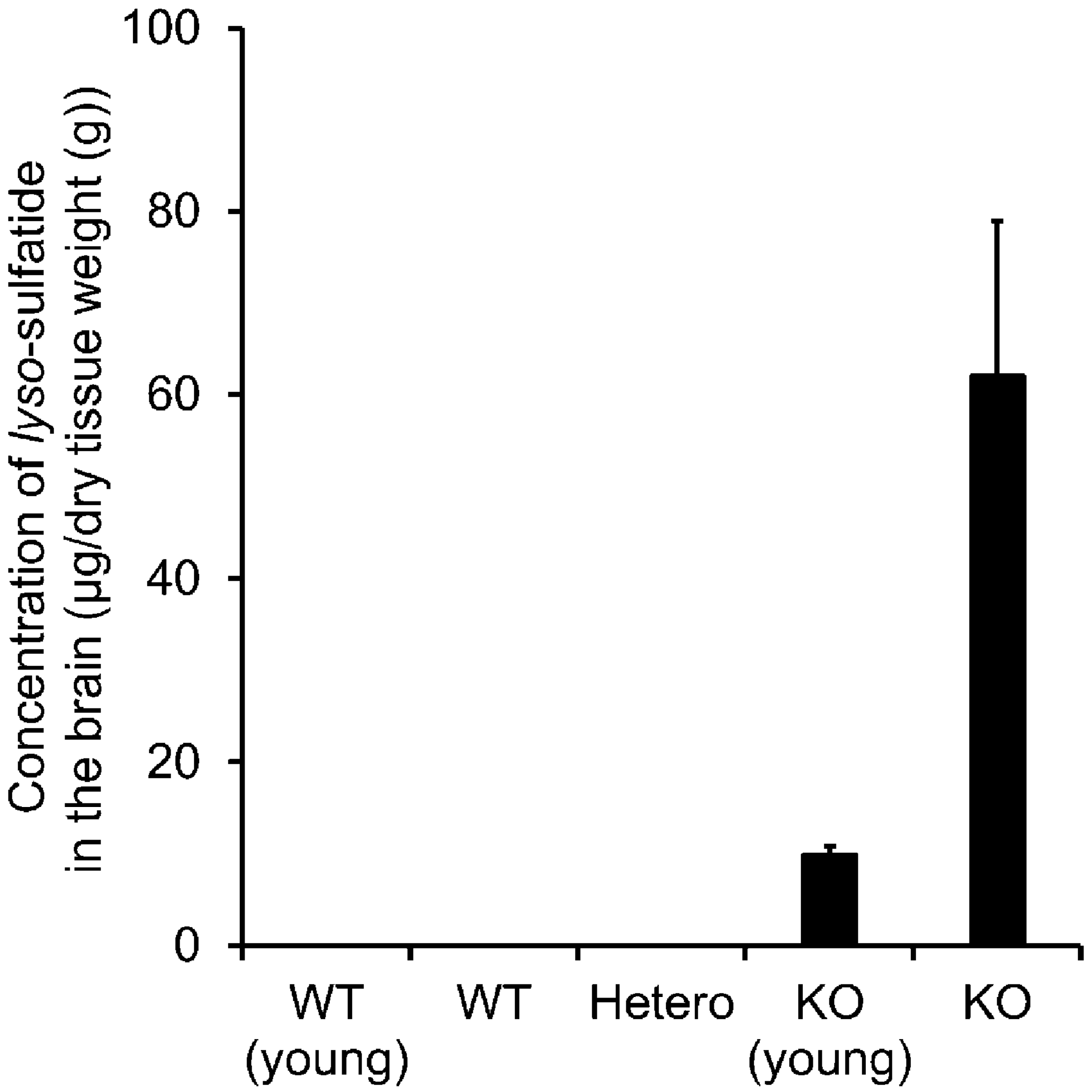
Concentration of lyso-sulfatide
in the brain (µg/dry tissue weight (g))
100
80
60
40
20
0
WT
(young)
WT
Hetero
KO
(young)
KO

[FIG. 34]

FIG. 34

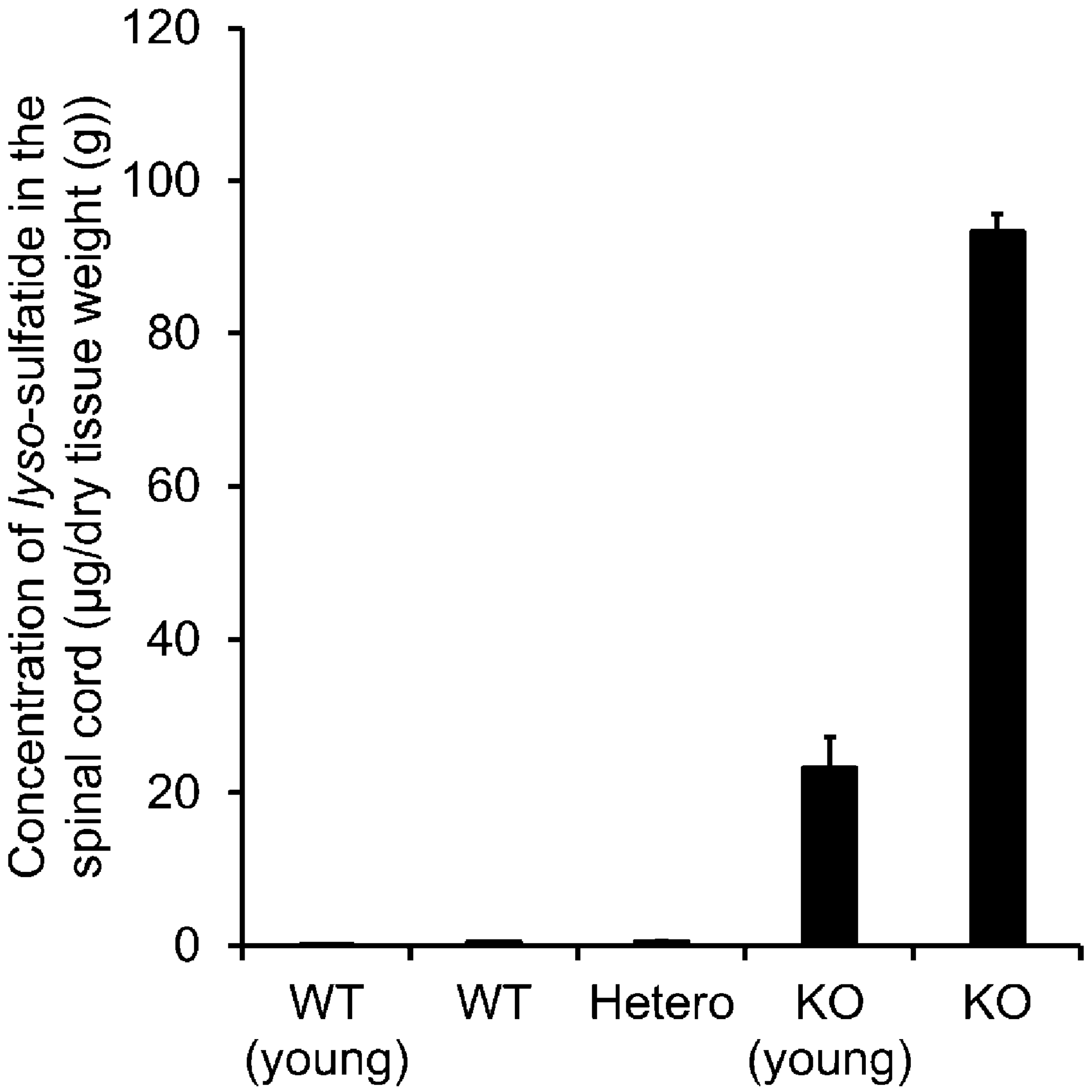
Concentration of lyso-sulfatide in the spinal cord (µg/dry tissue weight (g))
120
100
80
60
40
20
0
WT (young)
WT
Hetero
KO (young)
KO

[FIG. 35]

FIG. 35

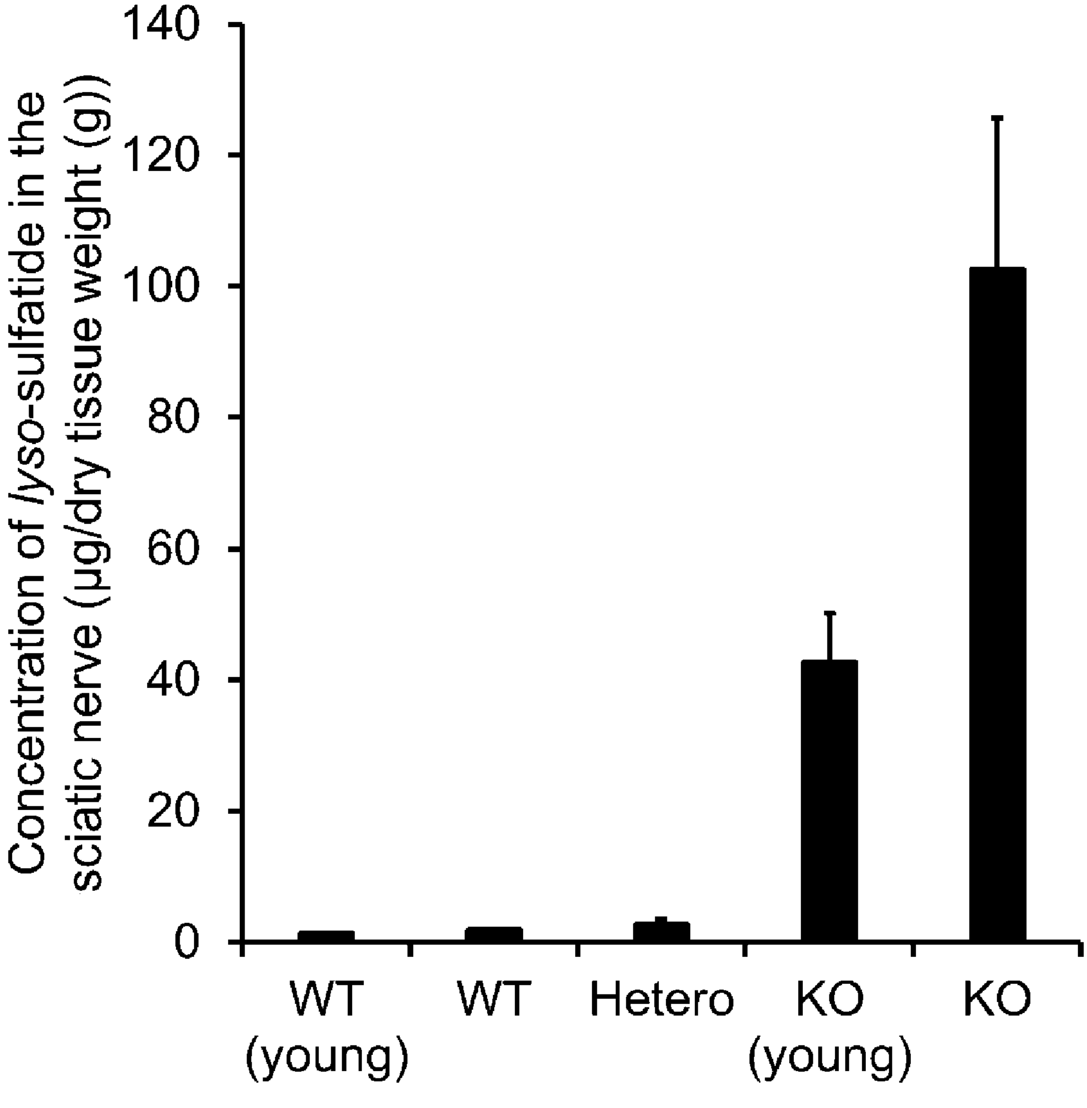
Concentration of lyso-sulfatide in the sciatic nerve (µg/dry tissue weight (g))
140
120
100
80
60
40
20
0
WT (young)
WT
Hetero
KO (young)
KO

[FIG. 36]

FIG. 36

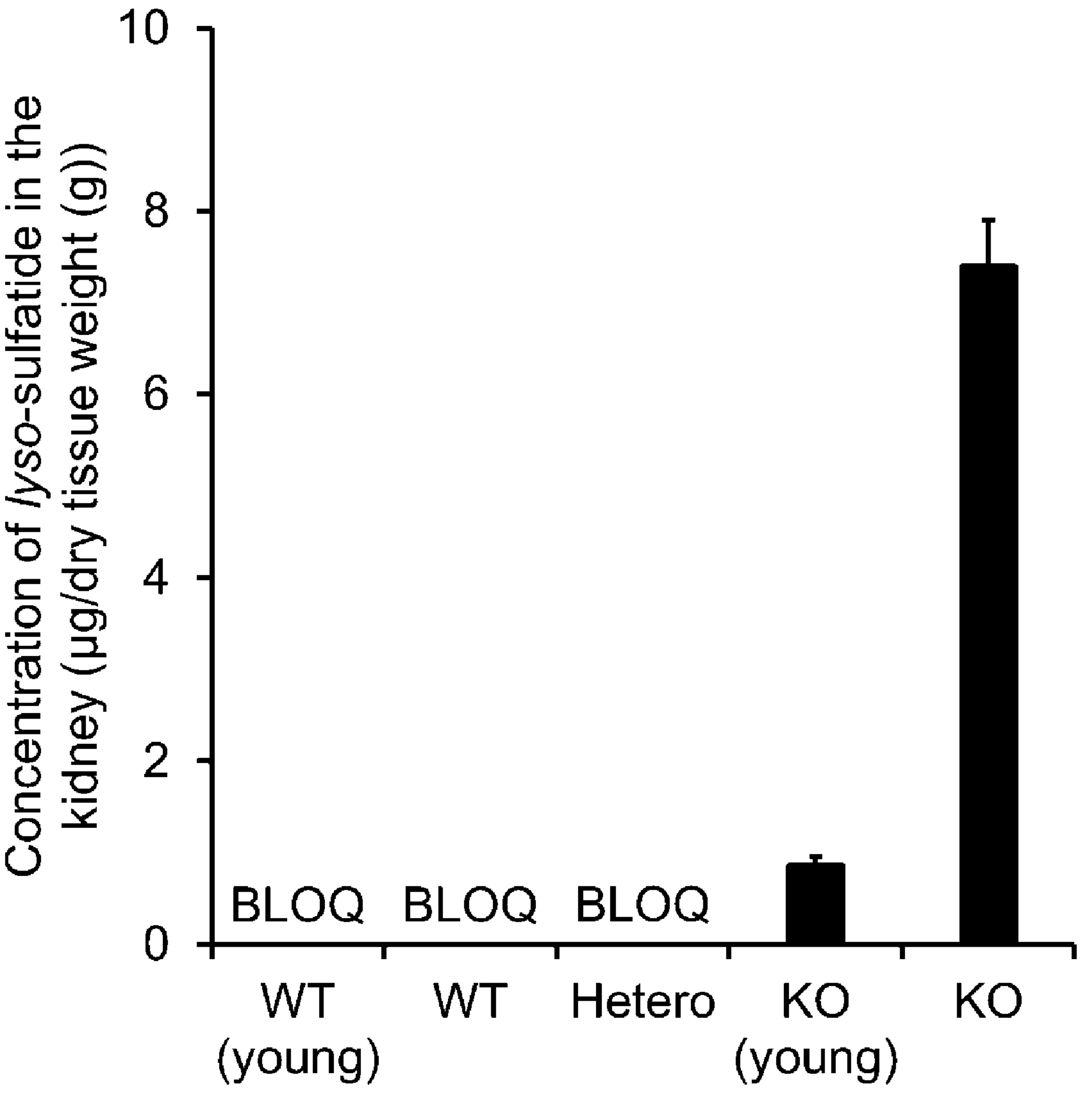
Concentration of lyso-sulfatide in the kidney (μg/dry tissue weight (g))
10
8
6
4
2
0
BLOQ
BLOQ
BLOQ
WT (young)
WT
Hetero
KO (young)
KO

[FIG. 37]

FIG. 37

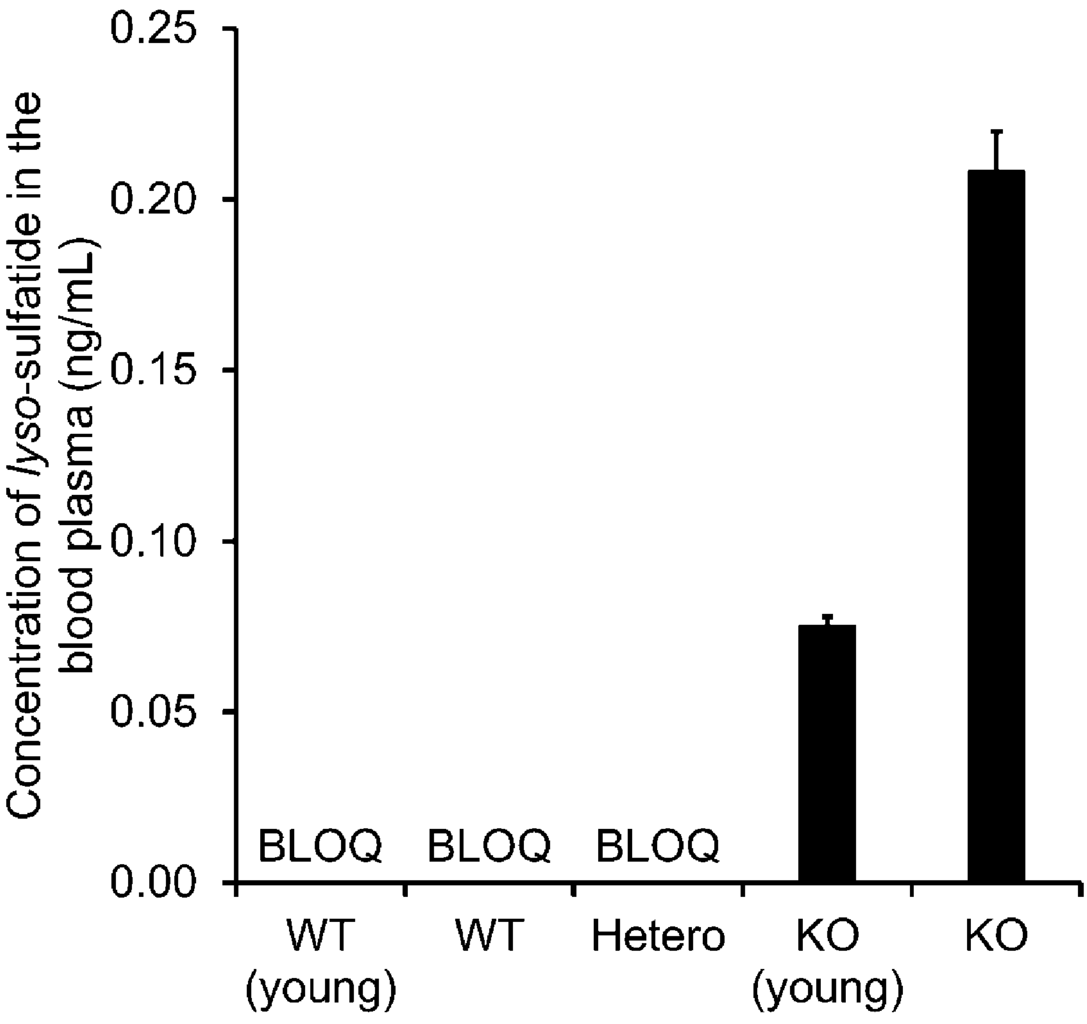
0.25
0.20
0.15
0.10
0.05
0.00
Concentration of lyso-sulfatide in the blood plasma (ng/mL)
BLOQ
BLOQ
BLOQ
WT (young)
WT
Hetero
KO (young)
KO

[FIG. 38]

FIG. 38

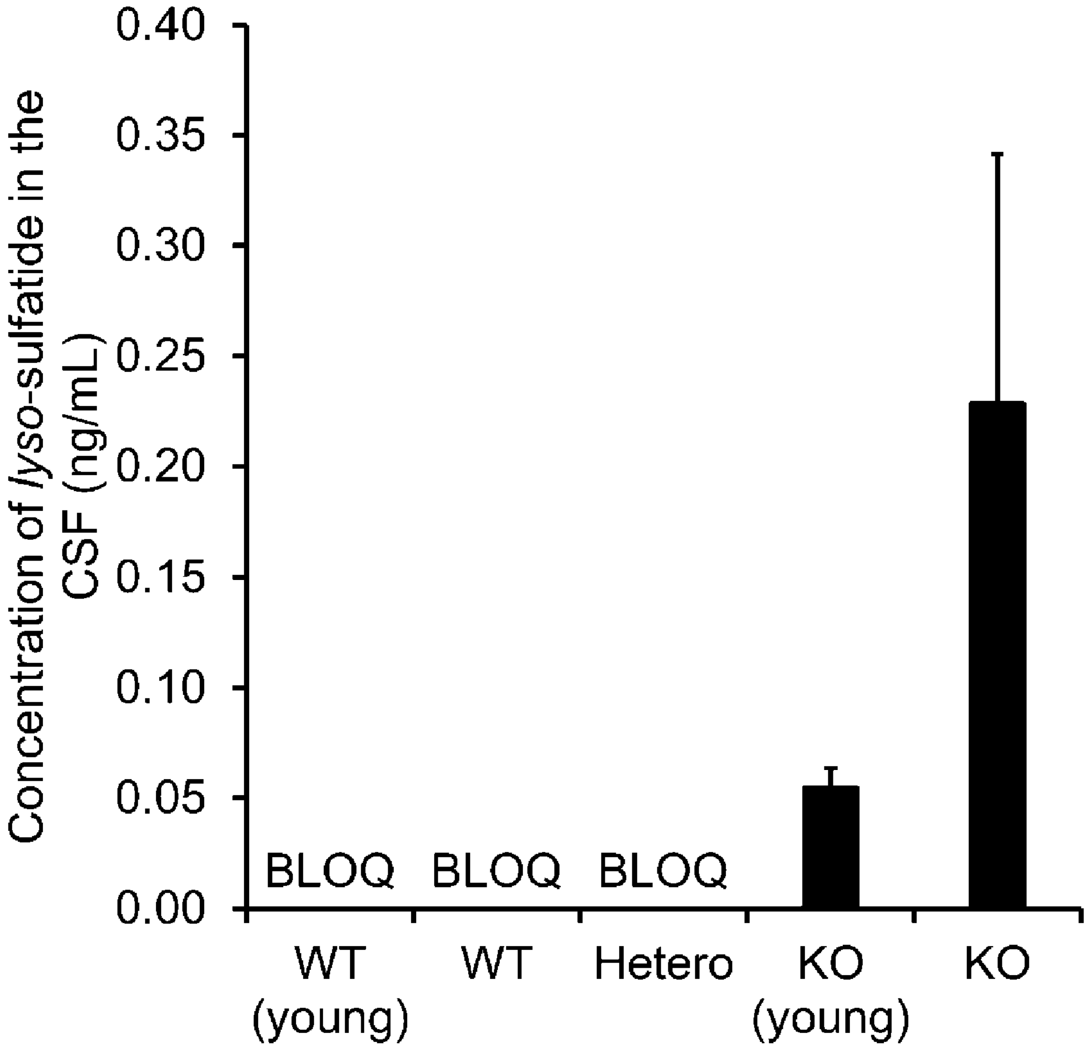
0.40
0.35
0.30
0.25
0.20
0.15
0.10
0.05
0.00
Concentration of *lyso*-sulfatide in the CSF (ng/mL)
BLOQ
BLOQ
BLOQ
WT (young)
WT
Hetero
KO (young)
KO

[FIG. 39]

FIG. 39

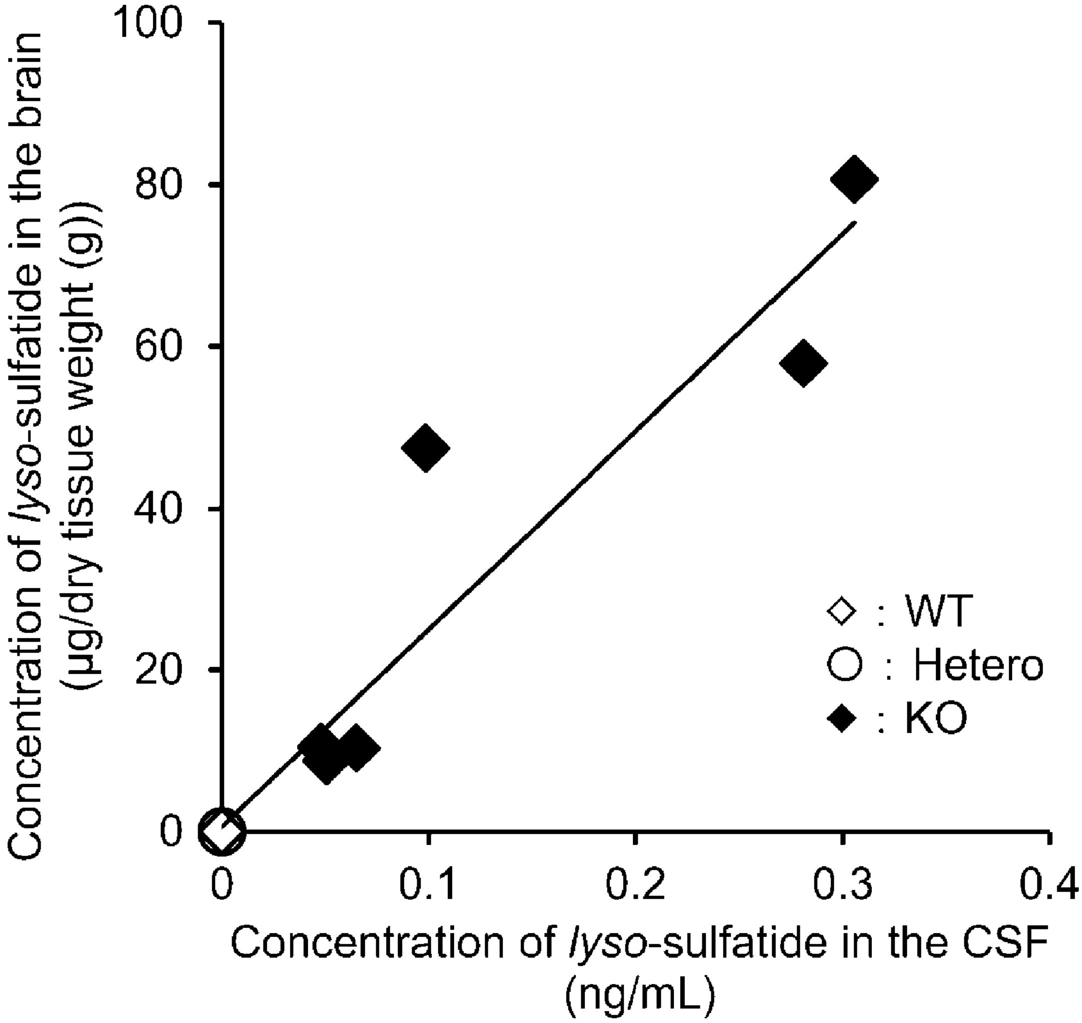
Concentration of lyso-sulfatide in the brain (μg/dry tissue weight (g))
100
80
60
40
20
0
0
0.1
0.2
0.3
0.4
Concentration of lyso-sulfatide in the CSF (ng/mL)
◇ : WT
○ : Hetero
◆ : KO

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004501365 A
- JP 2016506501 A
- US 2002102737 A1


### Non-patent literature cited in the description


- **DERUISSEAU LR. et al.** *Proc Natl Acad Sci U S A.*, 2009, vol. 106 (23), 9419-24
- **CHIEN YH. et al.** *JIMD Rep.*, 2015, vol. 19, 67-73
- **SLUITER W. et al.** *Clin Chem.*, 2012, vol. 58 (7), 1139-47
- **YOUNG SP. et al.** *Genet Med.*, 2009, vol. 11 (7), 536-41
- **AN Y. et al.** *Mol Genet Metab.*, 2005, vol. 85 (4), 247-54
- **YOUNG SP. et al.** *Anal Biochem.*, 2003, vol. 316 (2), 175-80
- **ROZAKLIS T. et al.** *Clin Chem.*, 2002, vol. 48 (1), 131-9
- **JB LOBATO et al.** *Diseases.*, 2016, vol. 4 (4), 40
- **PETTAZZONI et al.** *PLoS ONE.*, 2017, vol. 12 (7), e0181700
- **STRECKER G et al.** *Biochimie.*, 1978, vol. 60 (8), 725-34
- **ABRAHAM D et al.** *Biochem J.*, 1984, vol. 222 (1), 25-33
- **MICHALSKI JC et al.** *Eur J Biochem.*, 1991, vol. 201 (2), 439-58
- **MICHALSKI JC et al.** *Biochim Biophys Acta*, 1999, vol. 1455 (2-3), 69-84
- **WOLF H et al.** *Dis Model Mech.*, 2016, vol. 9 (9), 1015-28
- **TODA K et al.** *Biochem Biophys Res Commun.*, 1989, vol. 159 (2), 605-11
- **BLOMQVIST M et al.** *Lipids Health Dis.*, 2011, vol. 10, 28
- **DALI C et al.** *Ann Clin Transl Neurol.*, 2015, vol. 2 (5), 518-33
- **MIRZAIAN M et al.** *J Lipid Res.*, 2015, vol. 56 (4), 936-43
- **BLOMQVIST M et al.** *J Lipid Res.*, 2017, vol. 58 (7), 1482-1489